# EUROPEAN PATENT APPLICATION

(11) **EP 2 062 895 A1**
(43) Date of publication of application: **27.05.2009**
(21) Application number: 07834964.4
(22) Date of filing: 08.08.2007
(51) Int. Cl.: C07D 487/04, A61K 31/55, A61P 3/14, A61P 9/10, A61P 17/00, A61P 17/02, A61P 17/06, A61P 25/08, A61P 25/14, A61P 25/28

(54) **SUBSTITUTED AZEPINO[4,3-B]INDOLES, PHARMACOLOGICAL COMPOSITION AND A METHOD FOR THE PRODUCTION AND USE THEREOF**

(30) Priority: 24.08.2006 RU 2006130505
(71) Applicant: Alla Chem, LLC., Carson City NV 89701 (US); Ivashchenko, Andrey, Alexandrovich, Moscow 127576 (RU)
(72) Inventor: IVASHCHENKO, Andrey Alexandrovich, 127576 (RU); FROLOV, Yevgeniy Borisovich, Moscow, 123308 (RU); TKACHENKO, Sergey Yevgenievich, Moskovskaya obl., 142432 (RU); KHVAT, Alexander Viktorovich, San Diego, CA 92131 (US); MALYARCHUK, Sergey Viktorovich, San Diego Compas, CA 9472 (US); MITKIN, Oleg Dmitrievich, Moskovskaya obl., 141400 (RU); OKUN, Ilya Matusovich, San Diego, CA 92121 (US); KYSELEV, Alexandr Sergeevich, San Diego, CA 92130 (US); SAVCHUK, Nikolay Filippovich, Moscow, 117420 (RU); IVASHCHENKO, Alexandr Vasilievich, Encinitas, CA 92024 (US)
(74) Representative: Jeck, Anton
(86) International application number: PCT/RU2007/000436
(87) International publication number: WO 2008/024029

(57) **Abstract**

The invention relates to novel chemical compounds, searching for novel physiologically active substanses, leader compounds, "molecular tools", and drug candidates obtained on the basis of screening of combinatorial and focused libraries of the said compounds, and also to pharmaceutical composition, methods for preparation and use thereof.

The invention proposes hydrogenated azepino[4,3-b]indoles of general formula **1** or racemates, optical isomers, geometrical isomers, mixtures of optical or geometrical isomers, pharmaceutically acceptable salts and/or hydrates thereof: 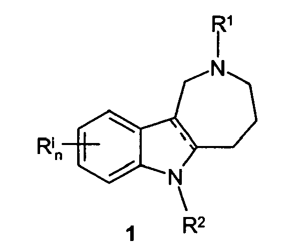 wherein: solid line together with the dotted line (---) represents a single or double bond; R¹ and R² independently of each other are amino group substituents selected from hydrogen; optionally substituted C₁-C₈ alkyl with substituents selected from optionally substituted aryl or 5-6-membered azaheterocyclyl; C₁-C₈ alkoxycarbonyl; optionally substituted phenyl; optionally substituted carbonylamino or thiocarbonylamino; substituted acyl; C₁-C₈ alkylsulfonyl; optionally substituted arylsulfonyl; upon that, the substituents in the said R₁ and R₂ independently selected from C₁-C₈ alkyl, halogen atoms, nitro group, carboxy group, alkoxy, aryl; Rⁱₙ represents one or more "substituents of cyclic structure" of the same or different structure selected from hydrogen, halogen, C₁-C₈ alkyl, C₆-C₁₀ aryl, 5-6-membered azaheterocyclyl.

## Description

### Field of the invention

The present invention relates to the synthesis of novel chemical compounds, searching for novel physiologically active substances, leader-compounds, "molecular tools" and drug candidates obtained on the basis of screening of combinatorial and focused libraries of the said compounds, and also to pharmaceutical composition, methods for preparation and use thereof.

More particularly, the present invention refers to novel annelated azaheterocycles - hydrogenated azepino[4,3-b]indoles, optical and geometrical isomers, mixtures of isomers, pharmaceutically acceptable salts and/or hydrates thereof, to methods for their preparation, to pharmaceutical compositions, containing these compounds as an active ingredients, and also to a way of prophylaxis and treatment of various diseases, among them neurodegenerative diseases, such as, Alzheimer's disease, associated with the excessive Ca⁺² ions entry into neurones, that may initiate a variety of the pathological metabolic processes leading finally to death of neurons [D.W. Choi, Neurone, 1988; 1:623-634].

### Background of the invention

In the origin of pharmacological effect of hydrogenated azepino[4,3-b]indoles lay their ability to decrease effectively the cytosolic concentration of Ca⁺² ions in cases when intracellular Ca⁺² ions concentration becomes excessive as a result of various pathological processes. Another valuable pharmacological property of hydrogenated azepino[4,3-b]indoles is their highly effective antihistaminic activity that makes it possible to use them as active ingredients of pharmaceutical compositions for treatment of broad spectrum of allergic and autoimmune diseases including pollinosis, hives, bronchial asthma, atopic dermatitis, neurodermatitis, angioneurotic hypostasis, Quincke's disease, eczema, ambustial toxemia, and also the allergic reactions caused by medicines, foodstuff, cosmetics, dust, insect stings and so on.

Maintenance of low Ca⁺² ions concentration is extremely important for normal cell functioning because the increased level of Ca⁺² ions in cytosol for a rather long period of time leads to necrocytosis. Such mechanism of cellular death is a characteristic feature of all neurodegenerative diseases, that is why searching for new pharmacological remedies preventing excessive Ca⁺² ions entry into neurones is one of the most important direction in the design of neuroprotectors [Kiewert C., Hartmann J., Stoll J., Thekkumkara T.J., Van der Schyf C.J., Klein J. NGP1-01 is a Brain-permeable Dual Blocker of Neuronal Voltage- and Ligand-operated Calcium Channels. Neurochem Res. 2006 May 3; Epub ahead of print]. Concentration of cytosolic calcium in eucariotic cells is regulated by transmembrane transport and cytoplasmatic calcium handling [Sayer R.J. Intracellular Ca2+ handling. Adv Exp Med Biol. 2002; 513:183-96]. Upon that the following complicated interrelated processes take place: 1) calcium ions transport by two families of Ca⁺² channels, namely: by calcium channels of cellular membrane and calcium channels located in a membrane of endo(sarco)plasmatic reticulum (ER or SR), which form access paths of calcium into cytoplasm; 2) elimination of calcium from cytoplasm by means of active calcium pumps of plasmalemma and/or calcium exchangers; and 3) calcium ions accumulation by intracellular calcium repository and mitochondrias. The latest serve as systems of calcium buffers, capable to accumulate and storage it, thus supporting a calcium homeostasis in cytoplasm. The most part of Ca⁺²-regulated processes in cell occurs at concentration of Ca⁺² iones varying in a range of 10⁻⁷-10⁻⁶ M, whereas its concentration in the extracellular medium is close to 10⁻³ M.

It is obvious, that various proteins supporting calcium homeostasis in cytoplasm play an exceptional role in patogenesis of such neurologic disorders as hypoxia-ischemia, hypoglycemia, convulsive conditions, brain traumas, and also chronic neurodegenerative diseases (including Alzheimer's disease, Huntington's chorea, lathyrism, amyotrophic lateral sclerosis) [J. W. McDonald, M. V. Johnston - Brain Res. Rev., 1990; 15:41-70; Stys P.K. General mechanisms of axonal damage and its prevention. J Neurol Sci. 2005; 233(1-2):3-13]. The possibility of pool regulation of intracellular calcium determines the great pharmacological meaning of selective blocker/activators of various potential-dependent calcium channels (for example, T-, L-, N-, P-, Q-, R-channels) and specific antagonists/modulators of ligand-gated channels (for example, NMDA-, AMPA-, nAChR-, P2X- receptors) [Barry P.H., Lynch J.W. Ligand-gated channels. IEEE Trans Nanobioscience. 2005; 4(1):70-80]. Now a large number of such calcium transport effectors are offered as highly effective medicaments. For example, calcium antagonists are the group of medicinal drugs capable to block reversibly calcium flow through potential-dependent calcium channels. By their chemical structure these drugs are devided into two large subgroups - dihydropyridines (Nifedipine, Amlodipine, Felodipine, etc.) among the properties of which predominates the effect of peripheral vasodilatation, and nondihydropyridines (Verapamil and Diltiazem) in the properties of which prevail negative chrono- and inotropic actions, and also the ability to reduce atrioventricular conductivity [Sica D.A. Pharmacotherapy review: calcium channel blockers. J Clin Hypertens (Greenwich). 2006; 8(1):53-6]. An example of the medicament blocking an excessive entry of calcium ions into neurones through ligand-gated channels (NMDA) is Memantine, widely used now in the treatment of Alzheimer's disease [Rogawski M.A., Wenk G.L. The neuropharmacological basis for the use of memantine in the treatment of Alzheimer's disease. CNS Drug Rev. 2003; 9(3):275-308]. Practically all medicaments mentioned above prevent the excessive entry of calcium ions into cells, however until now calcium homeostasis modulators capable to reduce calcium cytosolic concentration which had become excessive due to any of possible pathological processes were practically unknown.

In this connection searching for effective neuroprotectors capable to prevent neurotoxic action of excessive cytosolic concentration of calcium is unconventional and perspective approach to the development of new medicaments for treatment of a wide range of neurologic and neurodegenerative diseases.

At the same time, hydrogenated azepino[4,3-b]indoles may be considered as close analogues of hydrogenated pyrido[4,3-b]indoles. Antihistaminic medicaments Dimebon and Diazolin could be referred to this class of compounds [Mashkovsky. Medicaments. Pub. 13. Kharkov: Torsing, 1998. v.1. p.280-281], and also Stobadine - a neuroprotector, antioxidant and antiarhythmic agent, being at the second stage of clinical tests [Horakova L., Stolc S. Antioxidant and pharmacodynamic effects of pyridoindole stobadine. Gen Pharmacol. 1998; 30 (5):627-38]. Dimebon and Diazolin are H1-receptor blockers and display pronounced antihistaminic and partial antiserotonin action. Not only antiarhythmic properties were found for Dimebon [Galenko-Iaroshevskii P.A., Chekanova O.A., Skibitskii V.V., Bartashevich V.V., Khankoeva A.I., Poliashova T.I. Antiarhythmic properties of dimebon. Biull Eksp Biol Med. 1995; 119(4):375-7], but also neuroprotective and cognitive-stimulating properties [Bachurin S., Bukatina E., Lermontova N., Tkachenko S., Afanasiev A., Grigoriev V., Grigorieva I., Ivanov Y., Sablin S., Zefirov N. Antihistamine agent Dimebon as a novel neuroprotector and a cognition enhancer. Ann N Y Acad Sci. 2001; 939:425-35]; it turned out to be an effective neuroprotector in animal models of Alzheimer's disease [Lermontova N.N., Lukoyanov N.V., Serkova T.P., Lukoyanova E.A., Bachurin S.O. Dimebon improves learning in animals with experimental Alzheimer's disease.Bull Exp Biol Med. 2000, 129(6), 544-546. Zefirov, N.S.; Afanasiev, A.Z.; Afanasievf, S.V.; Bachurin, S.E.; Tkachenko, S.E,; Grigoriev, V.V.; Jurovskaya, M.A.; Chetverikov, V.P.; Bukatina, E.E,; Grigoriev, I.V. US 6187785, 2001].

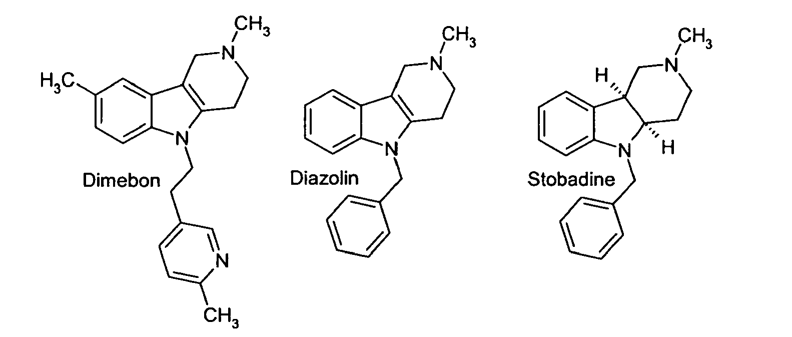

For the purpose of the development of novel highly effective neuroprotective, antihistaminic and antiarhythmic medicaments the authors of the present invention carried out a wide-range investigation in the group of hydrogenated azepino[4,3-b]indoles, the directed modifications of many positions of the main structure were fulfilled and as a result of it a new series of hydrogenated azepino[4,3-b]indoles has been synthesized. These compounds enhance memory effectively, prevent and suppress the development of various diseases, including neurodegenerative diseases, assosiated with the excessive entry of calcium ions into neurones, initiating a variety of pathological metabolic processes and finally inducing death of neurons [D.W. Choi, Neurone, 1988; 1:623-634], for example, Alzheimer's disease, etc. Another pharmacological property of hydrogenated azepino[4,3-b] indoles is their highly effective antihistaminic activity, allowing to use these compounds as active ingredients of pharmaceutical compositions for treatment of a wide range of allergic and autoimmune diseases including pollinosis, hives, bronchial asthma, atopic dermatitis, neurodermatitis, angioneurotic hypostasis, Quincke's disease, eczema, ambustial toxemia, and also the allergic reactions caused by medicines, foodstuff, cosmetics, dust, insect stings and so on.

Literature data concerning hydrogenated azepino[4,3-b]indoles are not so numerous and presented in table 1. 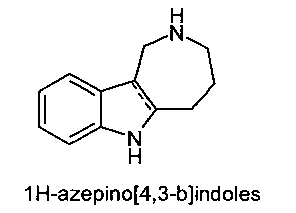 wherein: the solid line together with the dashed line (---) represent single or double bonds, respectively.

**Table 1. The known hydrogenated azepino[4,3-b]indoles**

| Nº | Formula | Reference |
|---|---|---|
| 1 | 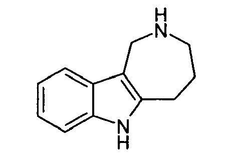 | Hester, J.B., Jr. J. Org. Chem., 1967, 32(12), 3804-3808. |
| 2 | 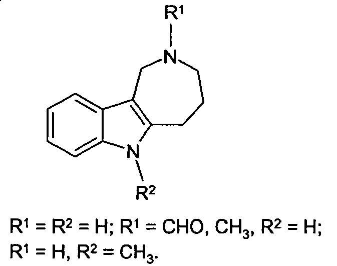 | Hester, J.B., Jr. FR 1566173, 1969**;** US 3563979,1971. Bascop, S.-I.; Laronze, J.-Y.; Sapi, J. Monatsh. Chem., 1999, 130(9), 1159 - 1166. |
| 3 | 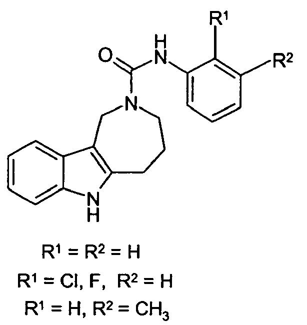 | WO 2004113300 A1, 2004 |

As a result of the conducted investigations the inventors have found a large group of hydrogenated azepino[4,3-b]indoles exhibiting physiological activity.

The present invention relates to the novel hydrogenated azepino[4,3-b]indoles, racemates, optical isomers, geometrical isomers, pharmaceutically acceptable salts and/or hydrates thereof which make up one of the aspects of the present invention, to combinatorial and focused libraries comprising new azaheterocycles, to the methods for their preparation and use.

The next aspect of the invention is pharmaceutical composition having neuroprotective, cognitive stimulating and antihistaminic activity for treatment of the diseases patogenesis of which is associated with the excessive intracellular Ca⁺² ions concentration, hypoxia and/or oxidative stress, apoptosis, and disorder of histaminergetic mediator system - such as arhythmia, ischemia, neuralgic and chronic neurodegenerative diseases, including Alzheimer's disease, Huntington's chorea, cerebral ischemia, epilepsy, schizophrenia, and some critical conditions (including angiospasm, cerebral trauma, paralysis), and disorder in histaminergetic mediator system.

Additional aspect of the invention consists in providing pharmaceutical compositions including therapeutically effective amount of the compound according to the invention and pharmaceutically acceptable inert adjuvant agents - such as carries, fillers, etc.

### Disclosure of the invention

Following below are definitions of terms that are used in describing this invention.
**"Azaheterocycle"** means an aromatic or non-aromatic monocyclic or polycyclic system containing in the ring structure at least one nitrogen atom. An azaheterocyclic structure may contain one or more "substituents of the cyclic system".
**"Aliphatic ralical"** means a radical obtained by removing a hydrogen atom from the nonaromatic C-H bond. An aliphatic radical may further contain substituens, such as aliphatic or aromatic radicals defined in this section. Aliphatic radicals are represented by alkyl, alkenyl, alkynyl, cycloalkyl, cycloalkenyl, heterocyclyl, heterocyclenyl, aralkenyl, aralkyloxyalkyl, aralkyloxycarbonylalkyl, aralkyl, aralkynyl, aralkyloxyalkenyl, heteroaralkenyl, heteroaralkyl, heteroaralkyloxyalkenyl, heteroaralkyloxyalkyl, annelated arylcycloalkyl, annelated heteroarylcycloalkyl, annelated arylcycloalkenyl, annelated heteroarylcycloalkenyl, annelated arylheterocyclyl, annelated heteroarylheterocyclyl, annelated arylheterocyclenyl, annelated heteroarylheterocyclenyl.
**"Alkenyl"** means an aliphatic straight or branched hydrocarbon group containing from 2 to 7 carbon atoms and including double carbon-carbon bond. "Branched" means that one or more lower alkyl groups, such as methyl, ethyl or propyl, may be attached to a straight alkenyl chain. An alkyl group may have one or more substituents such as: halogen, alkenyloxy, cycloalkyl, cyano; hydroxy, alkoxy, carboxy, alkynyloxy, aralkoxy, aryloxy, aryloxycarbonyl, alkylthio, heteroaralkyloxy, heterocyclyl, heterocyclylalkyloxy, alkoxycarbonyl, aralkoxycarbonyl, heteroaralkoxycarbonyl or Rₖ^{a}Rₖ₊₁^{a}N-, Rₖ^{a}Rₖ₊₁^{a}NC(=O)-, Rₖ^{a}Rₖ₊₁^{a}NSO₂-, wherein Rₖ^{a} and Rₖ₊₁^{a} are, independently from one another, "substituents of the amino group", the meaning of which are defined elsewhere in this section, for example, hydrogen, alkyl, aryl, aralkyl, heteroaralkyl, heterocyclyl or heteroaryl, or Rₖ^{a} and Rₖ₊₁^{a} together with the N-atom they are attached to, form through Rₖ^{a} and Rₖ₊₁^{a} a four- to seven-membered heterocyclyl or heterocyclenyl. The preferred alkyl groups are methyl, trifluoromethyl, cyclopropylmethyl, cyclopentylmethyl, ethyl, n-propyl, iso-propyl, n-butyl, tert-butyl, n-pentyl, 3-pentyl, methoxyethyl, carboxymethyl, methoxycarbonylmethyl, benzyloxycarbonylmethyl, and pyridylmethyloxycarbonylmethyl. The preferred alkenyl groups are ethenyl, propenyl, n-butenyl, iso-butenyl, 3-methylbuten-2-yl, n-pentenyl and cyclohexylbutenyl.
**"Alkenyloxy"** means alkenyl-O-group, wherein alkenyl is defined elsewhere in this section. The preferred alkenyloxy groups are allyloxy and 3-butenyloxy.
**"Alkenyloxyalkyl"** means alkenyl-O-alkyl group, wherein alkyl and alkenyl are defined elsewhere in this section.
**"Alkyl"** means an aliphatic hydrocarbon straight or branched group with 1-12 carbon atoms in the chain. Branched means that the alkyl chain has one or more "lower alkyl" substituents. Alkyl group may have one or more substituents of the same or different structure ("alkyl substituent") including halogen, alkenyloxy, cycloalkyl, aryl, heteroaryl, heterocyclyl, aroyl, cyano, hydroxy, alkoxy, carboxy, alkynyloxy, aralkoxy, aryloxy, aryloxycarbonyl, alkylthio, heteroarylthio, aralkylthio, arylsulfonyl, alkylsulfonylheteroaralkyloxy, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, alkoxycarbonyl, aralkoxycarbonyl, heteroaralkyloxycarbonyl or Rₖ^{a}Rₖ₊₁^{a}-, Rₖ^{a}Rₖ₊₁^{a}NC(=O)-, Rₖ^{a}Rₖ₊₁^{a}NC(=S)-, Rₖ^{a}Rₖ₊₁^{a}NSO₂-, wherein Rₖ^{a} and Rₖ₊₁^{a} are, independently from one another, "amino group substituents", the meanings of which are defined elsewhere in this section, for example, hydrogen, alkyl, aryl, aralkyl, heteroaralkyl, heterocyclyl or heteroaryl, or Rₖ^{a} and Rₖ₊₁^{a} together with the N-atom, they are attached to, form through Rₖ^{a} and Rₖ₊₁^{a}, a four- to seven-membered heterocyclyl or heterocyclenyl. The preferred alkyl groups are methyl, trifluoromethyl, cyclopropylmethyl, cyclopentylmethyl, ethyl, n-propyl, iso-propyl, n-butyl, tert-butyl, n-pentyl, 3-pentyl, methoxyethyl, carboxymethyl, methoxycarbonylmethyl, ethoxycarbonylmethyl, benzyloxycarbonylmethyl and pyridylmethyloxycarbonylmethyl. The preferred "alkyl substituents" are cycloalkyl, aryl, heteroaryl, heterocyclyl, hydroxy, alkoxy, alkoxycarbonyl, aralkoxy, aryloxy, alkylthio, heteroarylthio, aralkylthio, alkylsulfonyl, arylsulfonyl, alkoxycarbonyl, aralkoxycarbonyl, heteroaralkyloxycarbonyl or Rₖ^{a}Rₖ₊₁^{a}N-, Rₖ^{a}Rₖ₊₁^{a}NC(=O)-, annelated arylheterocyclenyl, annelated arylheterocyclyl.
**"Alkyloxyalkyl"**means alkyl-O-alkyl group, wherein alkyl groups are independent from one another and defined elsewhere in this section. The preferred alkyloxyalkyl groups are methoxyethyl, ethoxymethyl, n-butoxymethyl, methoxypropyl and iso-propyloxyethyl
**"Alkoxycarbonyl"** means alkyl-O-C(=O)-group, wherein alkyl is defined in this section. The preferred alkoxycarbonyl groups are methoxycarbonyl, ethoxycarbonyl, n-butoxycarbonyl, isopropyloxycarbonyl, benzyloxycarbonyl and phenethyloxycarbonyl.
**"Alkylthio"**means alkyl-S group, wherein alkyl group is defined elsewhere in this section.
**"Alkoxy"** means alkyl-O-group, wherein alkyl is defined elsewhere in this section. The preferred alkoxy groups are methoxy, ethoxy, n-propoxy, iso-propoxy and n-butoxy.
**"Alkoxycarbonylalkyl"** means alkyl-O-C(=O)-alkyl-group, wherein alkyl is defined elsewhere in this section. The preferred alkoxycarbonylalkyl groups are methoxy-carbonylmethyl, ethoxycarbonylmethyl, methoxy-carbonylethyl and ethoxy-carbonylethyl.
**"Amino group"**means Rₖ^{a}Rₓ₊₁^{a}N-group substituted or not by an "amino group substituent", the meanings of Rₖ^{a} and Rₖ₊₁^{a} are defined elsewhere in this section, for example, amino (NH₂), methylamino, diethylamino, pyrrolidino, morpholino, benzylamino or phenethylamino.
**"Aminoacid"** means a natural or synthetic aminoacid, the meaning of latter is defined elsewhere in this section. The preferred aminoacids are aminoacids containing α- or β-aminogroup. Examples of natural aminoacids are α-aminoacids, and also alanine, valine, leucine, isoleucine, proline, phenylalanine, triptophane, methionine, glycine, serine, threonine, and cysteine.
**"Amino-cyano-methylen"** means (NRₖ^{a}Rₖ₊₁^{a})(CN)C= group (radical) substituted or not by "amino group substituents" Rₖ^{a} and Rₖ₊₁^{a}, the meanings of which are defined elsewhere in this section, for example, amino.
**"Annelated cyclic structure"** (condensed cyclic structure) means bi- or poly-cyclic system in which annelated cycle or polycycle with the one it is annelated to, have, at least, two common atoms.
**"Annelated arylheterocycloalkenyl"** means annelated aryl and heterocycloalkenyl, the meanings of which are defined in this section. Annelated arylheterocycloalkenyl could be attached to any other fragment via any atom of its own system. Prefix "aza", "oxa" or "thia" before "heterocycloalkenyl" means that N, O or S atoms are introduced in the appropriate cyclic fragment, respectively. Annelated arylheterocycloalkenyl may have one or more "cyclic system substituens" of the same or different structure. N- and S-atoms belonging to heterocycloalkenyl fragment could be oxidized to N-oxide, S-oxide or S-dioxide. Annelated arylheterocycloalkenyls are represented by indolinyl, 1H-2-oxoquinolinyl, 2H-1-oxoisoquinolinyl, 1,2-dihydroquinolinyl, and so on.
**"Annelated arylheterocycloalkyl"** means an annelated aryl and heterocycloalkyl, the meanings of which are defined elsewhere in this section. Annelated arylheterocycloalkyl may be bound through any possible atom of the cyclic system. The prefixes "aza", "oxa" or "thia" preceding the word "heterocycloalkyl" indicate the presence of a nitrogen atom, an oxygen atom, or a sulfur atom, respectively, in the cyclic system. Annelated arylheterocycloalkyl may have one or more "cyclic system substituens" of the same or different structure. Nitrogen and sulfur atoms in the heterocycloalkyl part may be oxidized to an N-oxide, an S-oxide and an S-dioxide. Annelated arylheterocycloalkyls are represented by indolyl, 1,2,3,4-tetrahydroisoquinolinyl, 1,3-benzodioxolyl, and so on.
**"Annelated arylcycloalkenyl"**means an annelated aryl and cycloalkenyl, the meanings of which are defined elsewhere in this section. Annelated arylcycloalkenyl may be bound through any possible atom of the cyclic system. Annelated arylcycloalkenyl may have one or more "cyclic system substituents" of the same or different structure. Annelated arylcycloalkenyls are represented by 1,2-dihydro-naphthalenyl, indenyl, and so on.
**"Annelated arylcycloalkyl"** means an annelated aryl and cycloalkyl, the meanings of which are defined elsewhere in this section. Annelated arylcycloalkyl may be bound through any possible atom of the cyclic system. Annelated arylcycloalkyl may have one or more "cyclic system substituens" of the same or different structure. Annelated arylcycloalkyls are represented by indaninyl, 1,2,3,4-tetrahydronaphthyl, 5,6,7,8-tetrahydronapht-1-yl, and so on.
**"Annelated heteroarylcycloalkenyl"** means an annelated heteroaryl and cycloalkenyl, the meanings of which are defined in this section. Annelated heteroarylcycloalkenyl may be bound through any possible atom of the cyclic system. The prefixes "aza", "oxa" or "thia" preceding the word "heteroaryl" indicate the presence of a nitrogen atom, an oxygen atom, or a sulfur atom, respectively, in the cyclic system Annelated heteroarylcycloalkenyl may have one or more "cyclic system substituents" of the same or different structure. The nitrogen atom in the heteroaryl part may be oxidized to N-oxide. Annelated heteroarylcycloalkenyls are represented by 5,6-dihydroquinolinyl, 5,6-dihydroisoquinolinyl, 4,5-dihydro-1H-benzimidazolyl, and so on. **"Annelated heteroarylcycloalkyl"** means an annelated heteroaryl and cycloalkyl, the meanings of which are defined elsewhere in this section. Annelated heteroarylcycloalkyl may be bound through any possible atom of the cyclic system. The prefixes "aza", "oxa" or "thia" preceding the word "heteroaryl" indicate the presence of a nitrogen atom, an oxygen atom, or a sulfur atom, respectively, in the cyclic system. Annelated heteroarylcycloalkyl may have one or more "cyclic system substituents" of the same or different structure. The nitrogen atom in the heteroaryl part may be oxidized to N-oxide. Annelated heteroarylcycloalkyles are represented by 5,6,7,8-tetrahydroquinolinyl, 5,6,7,8-tetrahydroisoquinolinyl, 4,5,6,7-tetrahydro-1H-benzoimidazolyl, and so on.
**"Annelated heteroarylheterocyclenyl"** means an annelated heteroaryl and heterocyclenyl, the meanings of which are defined elsewhere in this section. Annelated heteroarylheterocyclenyl may be bound through any possible atom of the cyclic system. The prefixes "aza", "oxa" or "thia" preceding the word "heteroaryl" indicate the presence of a nitrogen atom, an oxygen atom, or a sulfur atom, respectively, in the cyclic system. Annelated heteroarylheterocyclenyl may have one or more "cyclic system substituents" of the same or different structure. The nitrogen atom in the heteroaryl part may be oxidized to N-oxide. The nitrogen atom and the sulfur atom in the heterocyclenyl part may be oxidized to N-oxide, S-oxide and S-dioxide. Annelated heteroarylheterocyclenyls are represented by 1,2-dihydro[2,7]naphthiridinyl, 7,8-dihydro[1,7]naphthiridinyl, 6,7-dihydro-3H-imidazo[4,5-c]pyridyl, and so on.
**"Annelated heteroarylheterocyclyl"** means an annelated heteroaryl and heterocyclyl, the meanings of which are defined elsewhere in this section. Annelated heteroarylheterocyclyl may be bound through any possible atom of the cyclic system. The prefixes "aza", "oxa" or "thia" preceding the word "heteroaryl" indicate the presence of a nitrogen atom, an oxygen atom, or a sulfur atom, respectively, in the cyclic system.. Annelated heteroarylheterocyclyl may have one or more "cyclic system substituents" of the same or different structure. The nitrogen atom in the heteroaryl part may be oxidized to N-oxide. The nitrogen atom and the sulfur atom in the heterocyclyl part may be oxidized to N-oxide, S-oxide and S-dioxide. Annelated heteroarylheterocyclyl are represented by 2,3-dihydro-1H-pyrrolo[3,4-b]quinolin-2-yl, 2,3-dihydro-1H-pyrrolo[3,4-b]indol-2-yl, 1,2,3,4-tetrahydro[1,5]naphthiridinyl, and so on.
**"Aralkenyl"** means an aryl-alkenyl group, wherein aryl and alkenyl are defined elsewhere in this section. For example, 2-phenethenyl is aralkenyl group.
**"Aralkyl"** means an alkyl group substituted by one or more aryl groups, wherein aryl and alkyl are defined elsewhere in this section. For example, benzyl-, 2,2-diphenylethyl- or phenethyl- are aralkyl groups.
**"Aralkylamino"** means an aryl-alkyl-NH-group, wherein aryl and alkyl are defined elsewhere in this section.
**"Aralkylsulfinyl"** means an aralkyl-SO-group, wherein aralkyl is defined elsewhere in this section.
**"Aralkylsulfonyl"** means aralkyl-SO₂-group, wherein aralkyl is defined elsewhere in this section.
**"Aralkylthio"** means an aralkyl-S-group, wherein aralkyl is defined elsewhere in this section.
**"Aralkoxy"** means an aralkyl-O-group, wherein aralkyl is defined elsewhere in this section. For example, benzyloxy or 1- or 2-naphthylenmethoxy are aralkyloxy groups.
**"Aralkoxyalkyl"** means an aralkyl-O-alkyl-group, wherein aralkyl and alkyl are defined elsewhere in this section. For example, benzyloxyethyl is aralkyl-O-alkyl group.
**"Aralkoxycarbonyl"** means an aralkyl-O-C(=O)-group, wherein aralkyl is defined elsewhere in this section. Benzyloxycarbonyl is an example of aralkoxycarbonyl group.
**"Aralkoxycarbonylalkyl"** means an aralkyl-O-C(=O)-alkyl-group, wherein aralkyl and alkyl are defined elsewhere in this section. Benzyloxycarbonylmethyl or benzyloxycarbonylethyl are examples of aralkoxycarbonylalkyl groups.
**"Aryl"** means aromatic monocyclic or polycyclic system containing from 6 to 14 carbon atoms, preferably from 6 to 10 carbon atoms. Aryl may contain one or more "cyclic system substituents" of the same or different structure. Aryl groups are represented by phenyl or naphthyl, substituted phenyl or substituted naphthyl. Aryl may be annelated to nonaromatic cyclic system or heterocyclic structure.
**"Arylcarbamoyl"** means an aryl-NHC(=O)-group, wherein aryl is defined elsewhere in this section.
**"Aryloxy"** means an aryl-O-group, wherein aryl is defined elsewhere in this section. Aryloxy groups are represented by phenoxy- and 2-naphthyloxy.
**"Aryloxycarbonyl"** means an aryl-O-C(=O)-group, wherein aryl is defined elsewhere in this section. Aryloxycarbonyl groups are represented by phenoxycarbonyl and 2-naphthoxycarbonyl groups.
**"Arylsulfinyl"** means an aryl-SO-group, wherein aryl is defined elsewhere in this section.
**"Arylsulfonyl"** means an aryl-SO₂-group, wherein aryl is defined elsewhere in this section.
**"Arylthio"** means an aryl-S-group, wherein aryl is defined elsewhere in this section. Arylthio groups are represented by phenylthio and 2-naphthylthio groups.
**"Aroylamino"** means an aroyl-NH-group, wherein aroyl is defined elsewhere in this section.
**"Aroyl"** means an aryl-C(=O)-group, wherein aryl is defined elsewhere in this section. Aroyl groups are represented by benzoyl, 1- and 2-naphthoyl groups.
"Aromatic radical" means a radical obtained by removing hydrogen atom from the aromatic C-H bond. "Aromatic" radical implies aryl and heteroaryl cyclic structures, defined in this section. Aryl and heteroaryl cyclic structures may further contain substituents, such as aliphatic and aromatic radicals, defined in this section. Aromatic radicals are represented by aryl, annelated cycloalkenylaryl, annelated cycloalkylaryl, annelated heterocyclylaryl, annelated heterocyclenylaryl, heteroaryl, annelated cycloalkylheteroaryl, annelated cycloalkenylheteroaryl, annelated heterocyclenylheteroaryl and annelated heterocyclylheteroaryl.
**"Aromatic cyclic structure"** means a planar cyclic system, in which all cyclic system atoms are involved in the formation of common conjugation system containing, according to Hückel rule, (4n + 2) π-electrons (n is a whole non-negative number). Examples of aromatic cyclic structures are benzene, naphthalene, anthracene, and so on. In the case of "heteroaromatic cyclic structures", the conjugation system involves π-electrons and p-electrons of heteroatoms, their total number being (4n + 2) as well. Examples of such cyclic structures are pyridine, thiophene, pyrrole, furan, thiazol, and so on. The aromatic cyclic structure may have one or more "cyclic system substituents" or may be annelated to non-aromatic cyclic structure, heteroaromatic or heterocyclic system.
**"Acyl"** means H-C(=O)-, alkyl-C(=O)-, cycloalkyl-C(=O), heterocyclyl-C(=O)-, heterocyclylalkyl-C(=O)-, aryl-C(=O)-, arylalkyl-C(=O)-, heteroaryl-C(=O)-, heteroarylalkyl-C(=O)-groups, wherein alkyl-, cycloalkyl-, heterocyclyl-, heterocyclylalkyl-, aryl-, arylalkyl-, heteroaryl-, heteroarylalkyl are defined elsewhere in this section.
**"Acylamino"**means an acyl-NH-group wherein acyl is defined elsewhere in this section.
**"Bifunctional reagent"** means a chemical compound having two reaction centers participating simultaneously or consecutively in reactions. Examples of bifunctional reagents are reagents containing carboxy group and formyl or keto group, such as 2-formylbenzoic acid, 2-(2-oxoethylcarbamoyl)benzoic acid, 2-(3-formylthiophen-2-yl)benzoic acid and 2-(2-formylphenyl)thiophen-3-carboxylic acid.
**"1,2-Vinyl radical"** means -CH=CH-group with one or more "alkyl substituents" of the same or different structure, the meanings of which are defined elsewhere in this section.
**"Halogen"** means fluorine, chlorine, bromine and iodine. Preference is given to fluorine, chlorine and bromine.
**"Heteroannelated cyclic structure"** means that the cyclic structure that is attached (annelated or condenced) to another cyclic or polycyclic structure contains at least one heteroatom.
**"Heteroaralkenyl"** means a heteroaryl-alkenyl-group, wherein heteroaryl and alkenyl are defined elsewhere in this section. Preferably, heteroarylalkenyl contains the lower alkenyl group. Heteroarylalkenyls are represented by 4-pyridylvinyl, thienylethenyl, imidazolylethenyl, pyrazinylethenyl, and so on.
**"Heteroaralkyl"** means a heteroaryl-alkyl-group, wherein heteroaryl and alkyl are defined elsewhere in this section. Heteroaralkyls are represented by pyridylmethyl, thienylmethyl, furylmethyl, imidazolylmethyl, pyrazinylmethyl, and so on.
**"Heteroaralkyloxy group"** means a heteroarylalkyl-O-group, wherein heteroarylalkyl is defined elsewhere in this section. Heteroaralkyloxy groups are represented by 4-pyridylmethyloxy, 2-thienylmethyloxy, and so on.
**"Heteroaryl"** means an aromatic monocyclic or polycyclic system with 5 to 14 carbon atoms, preferably 5 - 10 carbon atoms, wherein one or more carbon atoms are substituted by one or more heteroatoms, such as N, S or O. The prefixes "aza", "oxa" or"thia" preceding the word "heteroaryl" indicate the presence of a nitrogen atom, an oxygen atom, or a sulfur atom, respectively, in the cyclic system. Nitrogen atom in the heteroaryl part may be oxidized to an N-oxide. Heteroaryl may have one or more "cyclic system sustituents" of the same or different structure. Heteroaryl radicals are represented by pyrrolyl, furanyl, thienyl, pyridyl, pyrazinyl, pyrimidinyl, isoxazolyl, isothiazolyl, tetrazolyl, oxazolyl, thiazolyl, pyrazolyl, furazanyl, triazolyl, 1,2,4-thiadiazolyl, pyridazinyl, quinoxalinyl, phthalazinyl, imidazo[1,2-a]pyridinyl, imidazo[2,1-b]thiazolyl, benzofurazanyl, indolyl, azaindolyl, benzimidazolyl, benzothiazenyl, quinolinyl, imidazolyl, thienopyridyl, quinazolinyl, thienopyrimidinyl, pyrrolopyridinyl, imidazopyridinyl, isoquinolinyl, benzoazaindolyl, 1,2,4-triazinyl, thienopyrrolyl, furopyrrolyl, and so on.
**"Heteroarylsulfonylcarbamoyl"** means a heteroaryl-SO₂-NH-C(=O)-group, wherein heteroaryl is defined elsewhere in this section.
**"Heteroaroyl"-** means a a heteroaryl-C(=O)-group, wherein heteroaryl is defined elsewhere in this section. Heteroaroyl groups are represented by nicotinoyl, thienoyl, pyrazoloyl, and so on.
**"Heterocyclenyl"** means a non-aromatic monocyclic or polycyclic system containing 3 to 13 carbon atoms, preferably from 5 to 13 carbon atoms in which one or more carbon atoms are replaced with a heteroatoms such as nitrogen, oxygen or sulfur, and which contains at least one double carbon-carbon or double carbon-nitrogen bond. The prefixes "aza", "oxa" or "thia" preceding the word "heterocyclenyl" indicate the presence of a nitrogen atom, an oxygen atom, or a sulfur atom, respectively, in the cyclic system. Heterocyclenyl may have one or more "cyclic system substituens" of the same or different structure. Nitrogen atom and sulfur atom in the heterocyclenyl part may be oxidized to an N-oxide, an S-oxide and an S-dioxide. Heterocyclenyl groups are represented by 1,2,3,4-tetrahydropyridinyl, 1,2-dihydropyridinyl, 1,4-dihydropyridinyl, 2-pyrrolinyl, 3-pyrrolinyl, 2-imidazolyl, 2-pyrazolinyl, dihydrofuranyl, dihydrothiophenyl, and so on.
**"Heterocyclyl"** means aromatic or nonaromatic mono- or polycyclic system with 3 - 10 C-atoms, preferably 5 - 6 C- atoms in which one or more carbon atoms are substituted by heteroatom such as N, O or S. Prefix "aza", "oxa" or "thia" before "heterocyclyl" means that N, O or S atoms are introduced in the appropriate cyclic fragment, respectively. Heterocyclyl may have one or more "cyclic system substituents" of the same or different structure. N- And S-atoms belonging to heterocyclic fragment could be oxidized to N-oxide, S-oxide and S-dioxide. Heterocyclyl groups are represented by piperidinyl, pyrrolidinyl, piperazinyl, morpholinyl, thiomorpholinyl, thiazolidinyl, 1,4-dioxan-2-yl, tetrahydrofuranyl, tetrahydrothiophenyl, and so on.
**"Heterocyclyloxy"** means a heterocyclyl-O-group, wherein heterocyclyl is defined elsewhere in this section.
**"Hydrate"** means stoichiometric or nonstoichiometric compositions of the compounds or their salts with water.
**"Hydroxyalkyl"** means a HO-alkyl-group, wherein alkyl is defined elsewhere in this section.
**"Substituent"** means a chemical radical attached to the scaffold (fragment), for example, "alkyl group substituent", "amino group substituent", "carbamoyl substituent", "cyclic system substituent", the meanings of which are defined elsewhere in this section.
**"Alkyl substituent"** means a chemical radical that is attached to alkyl or alkenyl group, the meanings of which are defined elsewhere in this section. It may be selected from hydrogen, alkyl, halogen, alkenyloxy, cycloalkyl, aryl, heteroaryl, heterocyclyl, aroyl, cyano, hydroxy, alkoxy, carboxy, alkynyloxy, aralkoxy, aryloxy, aryloxycarbonyl, alkylthio, heteroarylthio, aralkylthio, arylsulfonyl, alkylsulfonylheteroaralkyloxy, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, alkoxycarbonyl, aralkoxycarbonyl, heteroaralkyloxycarbonyl or Rₖ^{a}Rₖ₊₁^{a}N-, Rₖ^{a}Rₖ₊₁^{a}NC(=O)-, Rₖ^{a}Rₖ₊₁^{a}NSO₂-, wherein Rₖ^{a} and Rₖ₊₁^{a} are, independently from one another "amino group substituent", the meanings of which are defined in this section, for example, hydrogen, alkyl, aryl, aralkyl, heteroaralkyl, heterocyclyl or heteroaryl, or Rₖ^{a} and Rₖ₊₁^{a} together with the nitrogen atom, which they are bound to, form through Rₖ^{a} and Rₖ₊₁^{a} a four- to seven-membered heterocyclyl or heterocyclenyl. The preferred alkyl groups are represented by methyl, trifluoromethyl, cyclopropylmethyl, cyclopentylmethyl, ethyl, n-propyl, iso-propyl, n-butyl, tert-butyl, n-pentyl, 3-pentyl, methoxyethyl, carboxymethyl, methoxycarbonylmethyl, ethoxycarbonylmethyl, benzyloxycarbonylmethyl, methoxycarbonylmethyl, and pyridylmethyloxycarbonylmethyl. The preferred "alkyl substituents" are represented by cycloalkyl, aryl, heteroaryl, heterocyclyl, hydroxy, alkoxy, alkoxycarbonyl, aralkoxy, aryloxy, alkylthio, heteroarylthio, aralkylthio, alkylsulfonyl, arylsulfonyl, alkoxycarbonyl, aralkoxycarbonyl, heteroaralkyloxycarbonyl or Rₖ^{a}Rₖ₊₁^{a}N-, Rₖ^{a}Rₖ₊₁^{a}NC(=O)-, annelated arylheterocyclenyl, annelated arylheterocyclyl. The meanings of "alkyl group substituents" are defined elsewhere in this section.
**"Amino group substituent"** means a substituent attached to an amino group. Amino group substituent represents hydrogen, alkyl, cycloalkyl, aryl, heteroaryl, heterocyclyl, acyl, aroyl, alkylsulfonyl, arylsulfonyl, heteroarylsulfonyl, alkylaminocarbonyl, arylaminocarbonyl, heteroarylaminocarbonyl, heterocyclylaminocarbonyl, alkylaminothiocarbonyl, arylaminothiocarbonyl, heteroarylaminothiocarbonyl, heterocyclylaminothiocarbonyl, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, alkoxycarbonylalkyl, aralkoxycarbonylalkyl, heteroaralkyloxycarbonylalkyl. The meanings of "amino group substituents" are defined elsewhere in this section.
**"Carbamoyl substituent"** means a substituent attached to carbamoyl group, the meaning of which is defined elsewhere in this section. Carbamoyl substituent may be selected from hydrogen, alkyl, cycloalkyl, aryl, heteroaryl, heterocyclyl, alkoxycarbonylalkyl, aralkoxycarbonylalkyl, heteroaralkyloxycarbonylalkyl or Rₖ^{a}Rₖ₊₁^{a}N-, Rₖ^{a}Rₖ₊₁^{a}C(=O)-alkyl, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl. The preferred "carbamoyl substituents" are represented by alkyl, cycloalkyl, aryl, heteroaryl, heterocyclyl, alkoxycarbonylalkyl, aralkoxycarbonylalkyl, heteroaralkyloxycarbonylalkyl or Rₖ^{a}Rₖ₊₁^{a}N-, Rₖ^{a}Rₖ₊₁^{a}NC(=O)-alkyl, annelated arylheterocyclenyl, annelated arylheterocyclyl. The meanings of "carbamoyl substituents" are defined elsewhere in this section.
**"Nucleophilic substituent"** is a chemical radical that is attached to the scaffold as a result of a reaction with a nucleophilic reagent, for example, one selected from the group of primary or secondary amines, alcohols, phenols, mercaptans and thiophenols.
**"Cyclic system substituent"** means a substituent attached to an aromatic or nonaromatic cyclic system selected from hydrogen, alkyl, alkenyl, alkynyl, aryl, heteroaryl, aralkyl, heteroaralkyl, hydroxy, hydroxyalkyl, amino, aminoalkyl, alkoxy, aryloxy, acyl, aroyl, halogen, nitro, cyano, carboxy, alkoxycarbonyl, aryloxycarbonyl, aralkoxycarbonyl, alkyloxyalkyl, aryloxyalkyl, heterocyclyloxyalkyl, arylalkyloxyalkyl, heterocyclylalkyloxyalkyl, alkylsulfonyl, arylsulfonyl, heterocyclylsulfonyl, alkylsulfinyl, arylsulfinyl, heterocyclylsulfinyl, alkylthio, arylthio, heterocyclylthio, alkylsulfonylalkyl, arylsulfonylalkyl, heterocyclylsulfonylalkyl, alkylsulfinylalkyl, arylsulfinylalkyl, heterocyclylsulfinylalkyl, alkylthioalkyl, arylthioalkyl, heterocyclylthioalkyl, arylalkylsulfonylalkyl, heterocyclylalkylsulfonylalkyl, arylalkylthioalkyl, heterocyclylalkylthioalkyl, cycloalkyl, cycloalkenyl, heterocyclyl, heterocyclenyl, amidino, Rₖ^{a}Rₖ₊₁^{a}N-, Rₖ^{a}N=, Rₖ^{a}Rₖ₊₁^{a}-alkyl, Rₖ^{a}Rₖ₊₁^{a}NC(=O)- or Rₖ^{a}Rₖ₊₁^{a}NSO₂-, wherein Rₖ^{a} and Rₖ₊₁^{a} are, independently from one another, an "amino group substituent", the meanings of which are defined elsewhere in this section, for example, hydrogen, optionally substituted alkyl, optionally substituted aryl, optionally substituted aralkyl, optionally substituted heteroaralkyl or Rₖ^{a}Rₖ₊₁^{a}N-substituent wherein Rₖ^{a} may be acyl or aroyl, the meaning of Rₖ₊₁^{a} is defined above, or "cyclic system substituents" are Rₖ^{a}Rₖ₊₁^{a}C(=O)- or Rₖ^{a}Rₖ₊₁^{a}SO₂- ,wherein Rₖ^{a} and Rₖ₊₁^{a} together with the nitrogen atom they are bound to, form through Rₖ^{a} and Rₖ₊₁^{a} a four to seven-membered heterocyclyl or heterocyclenyl.
**"Electrophilic substituent"** means a chemical radical attached to the scaffold as a result of a reaction with an electrophilic reagent, for example, one selected from a group of organic acids or their derivatives (anhydrides, imidazolides, acid chlorides), organic sulfonic acid esters or chlorides, organic haloformates, organic isocyanates and organic isothiocyanates.
**"Substituted amino group"** means Rₖ^{a}Rₖ₊₁^{a}-group wherein Rₖ^{a} and Rₖ₊₁^{a} are the substituents of an amino group, the meanings of which are defined elsewhere in this section.
**"Substituted carboxy group"** means C(O)OR-group. Substituted carboxyl has substituent R selected from alkenyl, alkyl, aryl, heteroaryl, heterocyclyl, the meanings of which are defined elsewhere in this section.
**"Substituted mercapto group"** means SR, S(O)R or S(O₂)R group wherein substituent R represents alkenyl, alkyl, aryl, heteroaryl, heterocyclyl, the meanings of which are defined elsewhere in this section.
**"Protective group" (PG)** means a chemical radical that is attached to the scaffold or synthetic intermediate for temporary protection of amino group in multifunctional compounds, including, but not limited to: amide substituent, such as formyl, optionally substituted acetyl (for example, trichloroacetyl, trifluoroacetyl, 3-phenylpropionyl and so on), optionally substituted benzoyl and so on; a carbamate substituent, such as: optionally substituted C₁-C₇-alkoxycarbonyl, for example, methyloxycarbonyl, ethyloxycarbonyl, tert-butyloxycarbonyl, 9-fluorenylmethyloxycarbonyl (Fmoc) and others; optionally substituted C₁-C₇-alkyl substituent, for example, tert-butyl, benzyl, 2,4-dimethoxybenzyl, 9-phenylfluorenyl and others; sulfonyl substituent, for example, benzenesulfonyl, p-toluenesulfonyl, etc.
**"Protected primary or secondary amine"** means a group of the general formula Rₖ^{a}Rₖ₊₁^{a}-, wherein Rₖ^{a} is a protective group PG, Rₖ₊₁^{a} is hydrogen, an "amino group substituent", the meaning of which is defined elsewhere in this section, for example, selected from alkyl, alkenyl, aryl, aralkyl, annelated arylcycloalkenyl, annelated arylcycloalkyl, annelated arylheterocyclenyl, annelated arylheterocyclyl, cycloalkyl, cyckloalkenyl, heteroaralkyl, heteroaryl, annelated heteroarylcycloalkenyl, annelated heteroarylcycloalkyl, annelated heteroarylheterocyclenyl, annelated heteroarylheterocyclyl, heterocyclenyl or heterocyclyl.
**"Imino group"** means Rₖ^{a}N= group substituted or not by an "amino group substituent" Rₖ^{a}, the meaning of which is defined elsewhere in this section, for example, imino (HN=), methylimino (CH₃N=), ethylimino (C₂H₅N=), benzylimino (PhCH₂N=) or phenethylimino (PhCH₂CH₂N=).
**"Inert substituent"** ("non-interfering substituent") means a low- or non-reactive radical, including, but not limited to: C₁-C₇ alkyl, C₂-C₇ alkenyl, C₂-C₇ alkynyl, C₁-C₇ alkoxy, C₇-C₁₂ aralkyl, substituted by inert substituents aralkyl, C₇-C₁₂ heterocyclylalkyl, substituted by inert substituents heterocyclylalkyl, C₇-C₁₂ alkaryl, C₃-C₁₀ cycloalkyl, C₃-C₁₀ cycloalkenyl, phenyl, substituted phenyl, toluyl, xylenyl, biphenyl, C₂-C₁₂ alkoxyalkyl, C₂-C₁₀ alkylsulfinyl, C₂-C₁₀ alkylsulfonyl, (CH₂)ₘ-O-(C₁-C₇ alkyl), -(CH₂)ₘ-N(C₁-C₇ alkyl)ₙ, aryl; aryl substituted by halogen or inert substituent, alkoxy substituted by inert substituent, fluoroalkyl, aryloxyalkyl, heterocyclyl, heterocyclyl substituted by inert substituents and nitroalkyl; where m and n are ranged from 1 to 7. The preferred "non-interfering substituents" are represented by C₁-C₇ alkyl, C₂-C₇ alkenyl, C₂-C₇ alkynyl, C₁-C₇ alkoxy, C₇-C₁₂ aralkyl, C₇-C₁₂ alkaryl, C₃-C₁₀ cycloalkyl, C₃-C₁₀ cycloalkenyl, C₁-C₇ alkyl substituted by inert substituents, phenyl; phenyl substituted by inert substituents, (CH₂)ₘ O-(C₁-C₇ alkyl), -(CH₂)ₘ-N(C₁-C₇ alkyl)ₙ, aryl; aryl substituted by inert substituents, heterocyclyl and heterocyclyl substituted by inert substituents.
**"Carbamoyl"** means C(=O)NRₖ^{a}Rₖ₊₁^{a}- group. Carbamoyl may have one or more "carbamoyl substituents" Rₖ^{a} and Rₖ₊₁^{a}, selected from hydrogen, alkyl, alkenyl, aryl, heteroaryl, heterocyclyl, the meanings of which are defined in this section.
**"Carbamoylazaheterocycle"** means an azaheterocycle with at least one carbamoyl group as a "cyclic system substituent". The meanings of "azaheterocycle", "cyclic system substituent", and "carbamoyl group" are defined elsewhere in this section.
**"Carboxy"** means HOC(=O)- (carboxy) group.
**"Carboxyalkyl"** means HOC(=O)-alkyl group,wherein alkyl is defined elsewhere in this section.
**"Carbocyclic structure"** means mono- or poly-cyclic system consisting of carbon atoms only. Carbocyclic rings can be either aromatic or alicyclic. Alicyclic polycycles may have one or more common atoms. In the case of one common atom, spiro-carbocyclic compounds (for example, spiro[2,2]pentane) are formed; in the case of two common atoms, various condensed systems (for example, decaline) are produced; three common atoms result in bridged systems (for example, bicycle[3,3,1]nonane); the greater number of common atoms leads to various polyhedral systems (for example, adamantane). Alicyclic structures may be "saturated", such as, for example, cyclohexane, or "partially saturated" such a, for example, tetraline.
**"Combinatorial library"** means a collection of compounds produced by parallel synthesis and intended for searching a hit or leader compound, and for optimization of physiological activity of the hit or leader as well, each compound of the library corresponds to the common scaffold, in this way the library is a collection of related homologues or analogues.
**"Methylene radical"** means -CH₂-group with one or two "alkyl substituents" of the same or different structure, the meanings of which are defined in this section.
**"Nonaromatic cyclic structure"** (saturated cyclic structure or partly saturated cyclic structure) means non-aromatic cyclic or polycyclic system formally derived as a result of complete or partial hydrogenization of unsaturated -C=C- or -C=N- bonds. A non-aromatic cyclic structure may have one or more "cyclic system substituents" and may be annelated to aromatic, heteroaromatic or heterocyclic systems. Examples of nonaromatic cyclic structures are cyclohexane or piperidine; examples of partly saturated cyclic structures are cyclohexene and piperideine.
**"Non-natural aminoacid"** means an aminoacid of not nucleinic origin. D-isomers of natural α-aminoacids, such as amino-butyric acid, 2-amino-butyric acid, γ-amino-butyric acid, N-α-alkyl aminoacids, 2,2-dialkyl-α-aminoacids, 1-amino-cycloalkylcarboxylic acids, β-alanine, 2-alkyl-β-alanines, 2-cycloalkyl-β-alanines, 2-aryl-β-alanines, 2-heteroaryl-β-alanines, 2-heterocyclyl-β-alanines and (1-aminocycloalkyl)-acetic acids are the representatives of not natural aminoacids in which the meanings of alkyl, cycloalkyl, aryl, heteroaryl and heterocyclyl are defined in this section.
**"Optionally aromatic cycle"** means a cycle which could be both aromatic and non-aromatic, the meanings of which are defined elsewhere in this section.
**"Optionally substituted radical"** means a radical without or with one or more substituents.
**"Optionally annelated (condensed) cyclic structure"** means a condensed or non-condensed cyclic structure, the meanings of which are defined elsewhere in this section.
**"Lower alkyl"** means a straight or branched alkyl radical containing from 1 to 4 carbon atoms.
**"Parallel synthesis"** means a method for carrying out a chemical synthesis of combinatorial library of individual compounds.
**"1,3-Propylene radical"** means -CH₂-CH₂-CH₂-group with one or more "alkyl substituents" of the same or different structure, the meanings of which are defined elsewhere in this section.
**"Leader compound"** (leader) means a compound of outstanding (maximum) physiological activity associated with a concrete biotarget related to a definite (or several) pathology or disease.
**"Hit compound"** (hit) means a compound demonstrated the desired physiological activity during the primary screening process.
**"Sulfamoyl group"** means Rₖ^{a}Rₖ₊₁^{a}NSO₂-group substituted or not by "amino group substituens" Rₖ^{a} and Rₖ₊₁^{a}, the meanings of which are defined elsewhere in this section.
"Sulfonyl" means R-SO₂-group, wherein R may be selected from alkyl, cycloalkyl, aryl, heteroaryl, heterocyclyl, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, the meanings of which are defined in this section.
**"Template"** means the common structural formula of a group of compounds or compounds forming the combinatorial library.
**"Thiocarbamoyl"** means Rₖ^{a}Rₖ₊₁^{a}NC(=S)-group. Thiocarbamoyl may have one or more "amino group substituents" Rₖ^{a} and Rₖ₊₁^{a}, the meanings of which are defined in this section, for example, alkyl, alkenyl, aryl, heteroaryl and heterocyclyl the meanings of which are defined in this section.
**"Cycloalkyl"** means non-aromatic monocyclic or polycyclic system containing from 3 to 10 carbon atoms. Cycloalkyl may have one or more "cyclic system substituents" of the same or different structure. The cycloalkyl groups are represented by cyclopropyl, cyclobutyl, cyclopentyl, cyclohexyl, decalinyl, norbornyl, adamant-1-yl, and so on. Cycloalkyl may be annelated to aromatic cycle or heterocycle. The preferred "cyclic system substituents" are represented by alkyl, aralkoxy, hydroxy or Rₖ^{a}Rₖ₊₁^{a}-, the meanings of which are defined elsewhere in this section.
**"Cycloalkylcarbonyl"** means cycloalkyl-C(=O)-group, wherein cycloalkyl is defined in this section. Cyclopropylcarbonyl and cyclohexylcarbonyl are the representatives of cycloalkylcarbonyl groups.
**"Cycloalkoxy"** means cycloalkyl-O-group, wherein cycloalkyl is defined elsewhere in this section.
**"Pharmaceutical composition"** means a composition containing a compound of formula I and at least one of the components selected from a group consisting of pharmaceutically acceptable and pharmacologicaly compatible fillers, solvents, diluents, auxiliary, distributing and sensing agents, delivery agents, such as preservatives, stabilizers, disintegrators, moisteners, emulsifiers, suspending agents, thickeners, sweeteners, flavouring agents, aromatizing agents, antibacterial agents, fungicides, lubricants, and prolonged delivery controllers, the choice and suitable proportions of which depend on the nature and the way of administration and dosage. Examples of suitable suspending agents are ethoxylated isostearyl alcohol, polyoxyethene, sorbitol and sorbitol ether, microcrystalline cellulose, aluminum metahydroxide, bentonite, agar-agar and tragacant and the mixtures of thereof as well. Protection against the effect of microorganisms can be provided by various antibacterial and antifungal agents, such as, for example, parabens, chlorobutanole, sorbic acid, and similar compounds. A composition may also contain isotonic agents, such as, for example, sugar, sodium chloride, and similar compounds. A prolonged effect of the composition may be achieved by agents slowing down absorption of the active ingredient, for example, aluminum monostearate and gelatine. Examples of suitable carriers, solvents, diluents and delivery agents include water, ethanol, polyalcohols and their mixtures, natural oils (such as olive oil) and for injection-grade organic esters (such as ethyl oleate). Examples of fillers are lactose, milk-sugar, sodium citrate, calcium carbonate, calcium phosphate and the like. Examples of disintegrators and distributors are starch, alginic acid and its salts, and silicates. Examples of suitable lubricants are magnesium stearate, sodium lauryl sulfate, talc and high molecular weight polyethylene glycol. A pharmaceutical composition for peroral, sublingval, transdermal, intramuscular, intravenous, subcutaneous, local or rectal administration of the active ingredient, alone or in combination with another active compound may be administered to humans and animals in a standard administration form, or in a mixture with traditional pharmaceutical carriers. Suitable standard administration forms include peroral forms such as tablets, gelatin capsules, pills, powders, granules, chewing-gums and peroral solutions or suspensions, for example, therapeutic kit; sublingval and transbuccal administration forms; aerosols; implants; local, transdermal, subcutaneous, intramuscular, intravenous, intranasal or intraocular forms and rectal administration forms.
**"Pharmaceutically acceptable salt"** means relatively nontoxic both organic and inorganic salts of acids and bases disclosed in this invention. The salts may be prepared ***in situ*** during synthesis, isolation or purification of compounds, or to be prepared purposely. In particular, bases salts may be prepared purposely starting from a purified free base of a disclosed compound and a suitable organic or mineral acid. Examples of salts prepared in this manner include hydrochlorides, hydrobromides, sulfates, bisulfates, phosphates, nitrates, acetates, oxalates, valeriates, oleates, palmitates, stearates, laurates, borates, benzoates, lactates, p-toluenesulfonates, citrates, maleates, fumarates, succinates, tartrates, methane sulphonates, malonates, salicylates, propionates, ethane sulphonates, benzene sulfonates, sulfamates and the like (Detailed description of the properties of such salts is given in: Berge S.M., et al., "Pharmaceutical Salts" J.Pharm.Sci., 1977, 66: 1-19). Salts of the disclosed acids may be also prepared by the reaction of purified acids specifically with a suitable bases; moreover, metal salts and amine salts may be synthesized too. Metal salts are salts of sodium, potassium, calcium, barium, magnesium, lithium and aluminum, sodium and potassium salts being preferred. Suitable inorganic bases from which metal salts can be prepared are sodium hydroxide, carbonate, bicarbonate and hydride; potassium hydroxide, carbonate and bicarbonate, lithium hydroxide, calcium hydroxide, magnesium hydroxide, zinc hydroxide. Organic bases suitable for preparation of the disclosed acid salts are amines and amino acids of the sufficient basicity to produce a stable salt and suitable for use for medical purposes (in particular, they are to have low toxicity). Such amines include ammonia, methylamine, dimethylamine, trimethylamine, ethylamine, diethylamine, triethylamine, benzylamine, dibenzylamine, dicyclohexylamine, piperazine, ethylpiperidine, tris(hydroxymethyl)aminomethane and the like. Besides, salts can be prepared using some tetraalkylammonium hydroxides, such as holine, tetramethylammonium, tetraethylammonium, and the like. Aminoacids may be selected from the main aminoacids - lysine, ornithine and agrinine.
**"Fragment"** (scaffold) means the structural carcass characteristic of a group of compounds in the "combinatorial library".
**"Focuced library"** means a combinatorial library or a collection of several combinatorial libraries or a collection of libraries and compounds arranged in a special way for the purpose of increased probability of finding hit-compounds and leader-compounds or with the purpose of intensification of optimization theirof. As a rule, the design of focused libraries is associated with directed search for effectors (inhibitors, activators, agonists, antagonists, and so on) of definite biotargets (enzymes, receptors, ion channels, and so on).
**"1,2-Ethylene radical"** means -CH₂-CH₂-group containing one or more "alkyl substituents" of the same or different structure, the meanings of which are defined elsewhere in this section.

The purpose of the present invention is the unknown, substituted hydrogenated azepino[4,3-b]indoles possessing biological activity.

The object is achieved by hydrogenated azepino[4,3-b]indoles of general formula **1** or racemates, or optical isomers, or geometrical isomers, or mixtures of optical or geometrical isomers, pharmaceutically acceptable salts and/or hydrates thereof: 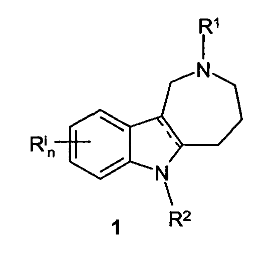 wherein:
the solid line together with the dashed line (---) represents single or double bond;
R¹ and R² independently of each other represent amino group substituents selected from hydrogen; optionally substituted C₁-C₈ alkyl with substituents selected from optionally substituted aryl or 5-6-membered azaheterocyclyl; C₁-C₈ alkoxycarbonyl; optionally substituted phenyl; optionally substituted carbonylamino or thiocarbonylamino; substituted acyl; C₁-C₈ alkylsulfonyl; optionally substituted arylsulfonyl; in addition, the substituents in the said R₁ and
R₂ independently selected from C₁-C₈ alkyl, halogen atoms, nitro group, carboxy group, alkoxy, aryl;
Rⁱₙ represents one or more "substituents of cyclic structure" of the same or different structure selected from hydrogen, halogen, C₁-C₈ alkyl, C₆-C₁₀ aryl, 5-6-membered azaheterocyclyl, with the exception of:
1,2,3,4,5,6-hexahydroazepino[4,3-b]indole **A(1)** and also 7-methyl, isopropyl, chloro; 8-ethyl, fluoro; 9-propyl, chloro, bromo; 10-ethyl, fluoro- substituted 1,2,3,4,5,6-hexahydroazepino[4,3-b]indoles; 2-methyl-**A(2),** 2-formyl-**A(3),** 2-acetyl-**A(4)** and 6-methyl-1,2,3,4,5,6-hexahydroazepino[4,3-b]indoles **A(5),** and also 3,4,5,6-tetrahydro-N-phenylazepino[4,3-b]indole-2(1H)-carboxamide **A(6);** 3,4,5,6-tetrahydro-N-(2-chlorophenyl)-azepino[4,3-b]indole-2(1H)-carboxamide **A(7);** 3,4,5,6-tetrahydro-N-(2-fluorophenyl)-azepino[4,3-b]indole-2(1H)-carboxamide **A(8);** 3,4,5,6-tetrahydro-N-(3-methylphenyl)-azepino[4,3-b]indole-2(1H)-carboxamide **A(9),**

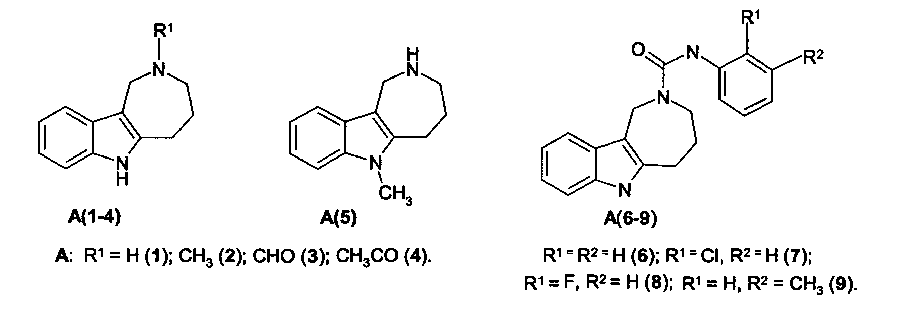

More preferred hydrogenated azepino[4,3-b]indoles are 1,2,3,4,5,6-hexahydroazepino[4,3-b]indoles of general formula **1.1,** *trans*-1,2,3,4,5,5a,6,10b-octahydroazepino[4,3-b]indoles of general formula **1.2** and *cis*-1,2,3,4,5,5a,6,10b-octahydroazepino[4,3-b]indoles of the general formula **1.3.** 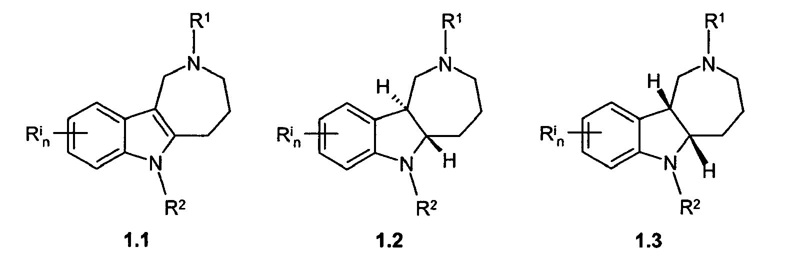 wherein:
R¹, R² and Rⁱₙ have the above meanings.

More preferred hydrogenated azepino[4,3-b]indoles are 1,2,3,4,5,6-hexahydroazepino[4,3-b]indoles of general formulas **1.1.1, 1.1.2, 1.1.3,** *trans-*1,2,3,4,5,5a,6,10b-octahydroazepino[4,3-b]indoles of general formulas **1.2.1, 1.2.2, 1.2.3** or *cis-*1,2,3,4,5,5a,6,10b-octahydroazepino[4,3-b]indoles of general formulas **1.3.1, 1.3.2, 1.3.3.** 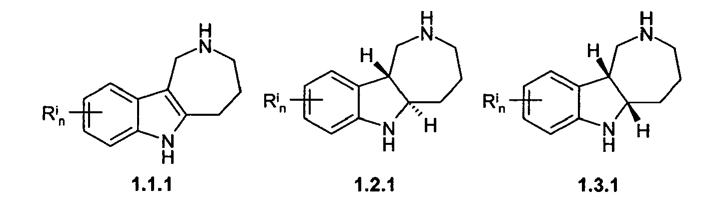 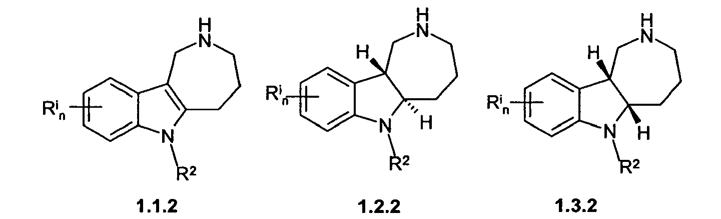 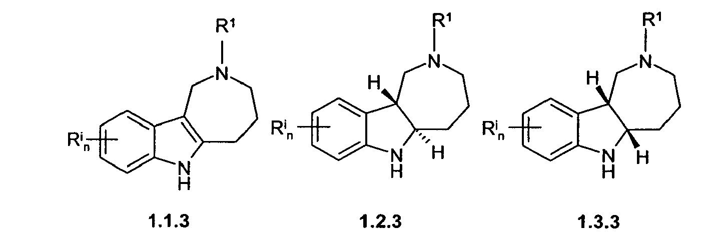 wherein:
R¹, R² and Rⁱₙ have the above meanings.

More preferred hydrogenated azepino[4,3-b]indoles are 1,2,3,4,5,6-hexahydroazepino[4,3-b]indoles of general formulas **1.1.1.1, 1.1.2.1,** *trans*-1,2,3,4,5,5a,6,10b-octahydroazepino[4,3-b]indoles of general formulas **1.2.1.1, 1.2.2.1** or *cis*-1,2,3,4,5,5a,6,10b-octahydroazepino[4,3-b]indoles of general formulas **1.3.1.1, 1.3.2.1.** 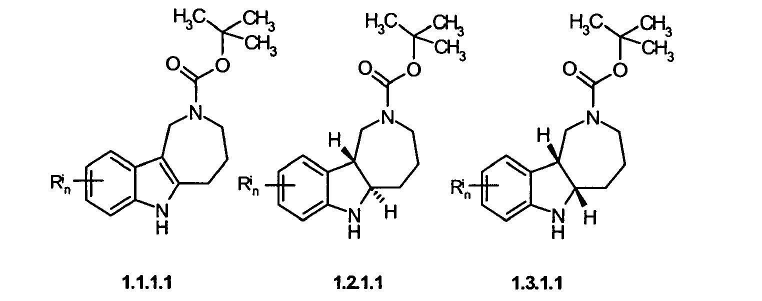 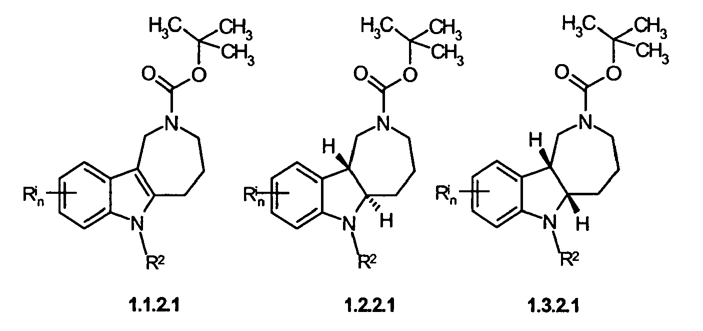 wherein:
R² and Rⁱₙ have the above meanings.

More preferred hydrogenated azepino[4,3-b]indoles are azepino[4,3-b]indoles of general formulas **1.1.4, 1.2.4, 1.3.4, 1.1.5, 1.2.5, 1.3.5, 1.1.6, 1.2.6, 1.3.6.** 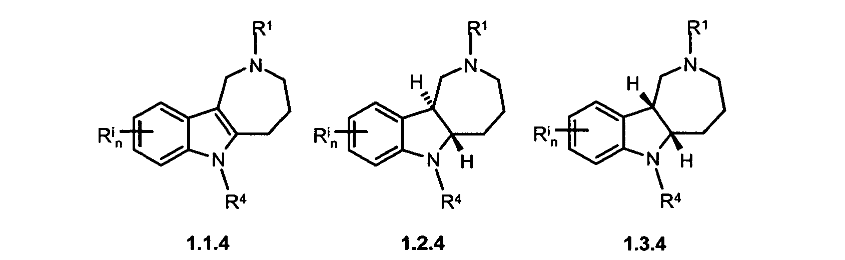 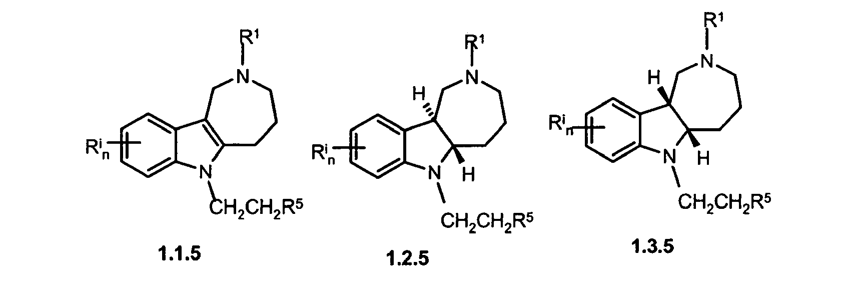 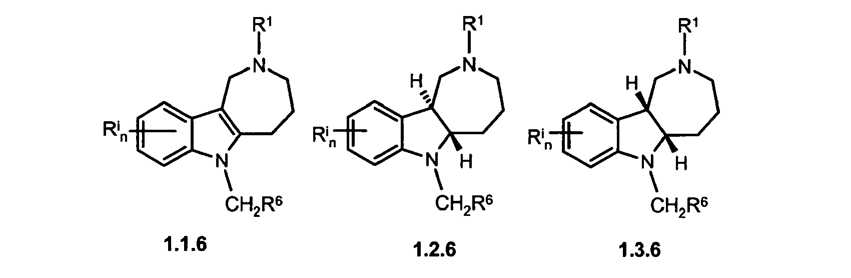 wherein:
R¹ and Rⁱₙ have the above meanings; R⁴ is an optionally substituted alkyl, an optionally substituted aryl, an optionally substituted heterocyclyl, an optionally substituted alkyloxycarbonyl or an optionally substituted alkoxycarbonylalkyl; R⁵ represents alkyloxycarbonyl, CN, aryl or heterocyclyl; R⁶ represents alkyloxycarbonyl, carbamoyl, CN, aryl or heterocyclyl.

More preferred hydrogenated azepino[4,3-b]indoles are azepino[4,3-b]indoles of general formulas **1.1.7, 1.2.7, 1.3.7, 1.1.8, 1.2.8, 1.3.8, 1.1.9, 1.2.9** and **1.3.9.** 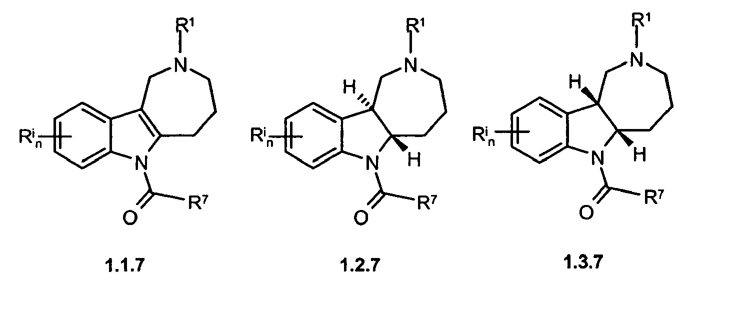 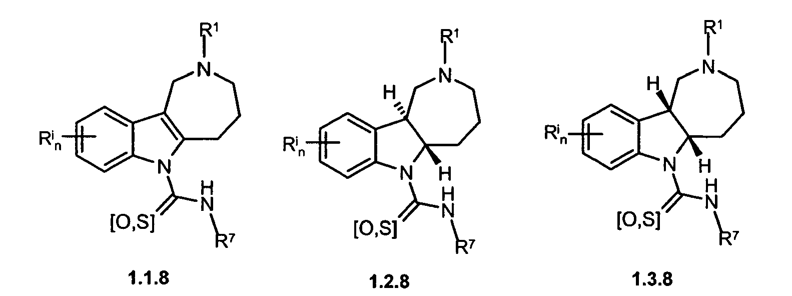 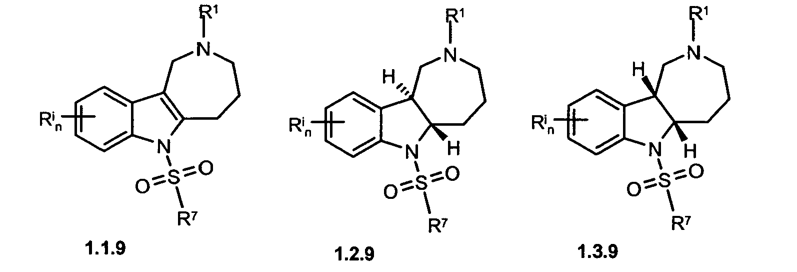 wherein:
R¹ and Rⁱₙ have the above meanings; R⁷ represents alkyl, aryl or heterocyclyl.

More preferred hydrogenated azepino[4,3-b]indoles are azepino[4,3-b]indoles of general formulas **1.1.10, 1.2.10, 1.3.10, 1.1.11, 1.2.11, 1.3.11, 1.1.12, 1.2.12** and **1.3.12.** 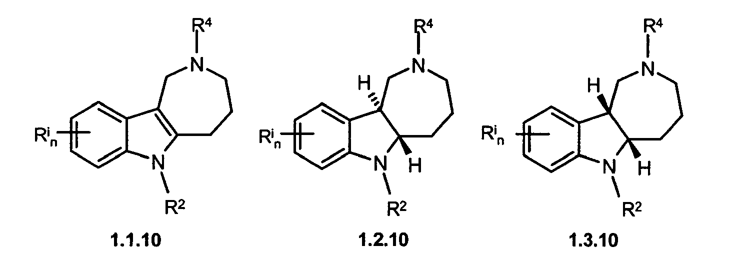 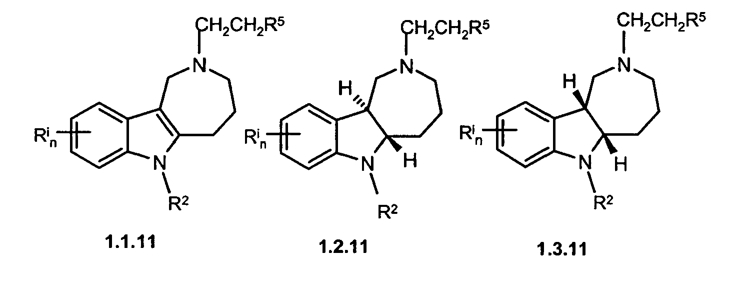 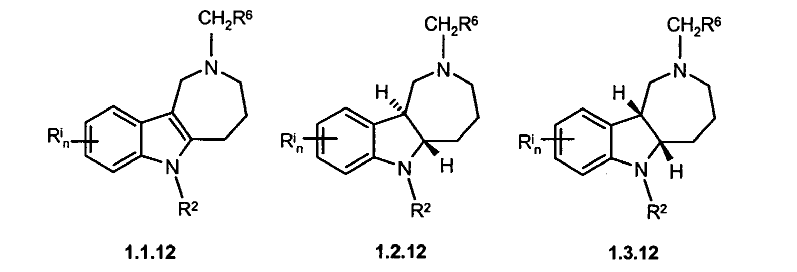 wherein:
R², R⁴, R⁵, R⁶ and Rⁱₙ have the above meanings.

More preferred hydrogenated azepino[4,3-b]indoles are azepino[4,3-b]indoles of general formulas **1.1.13, 1.2.13, 1.3.13, 1.1.14, 1.2.14, 1.3.14, 1.1.15, 1.2.15** and **1.3.15.** 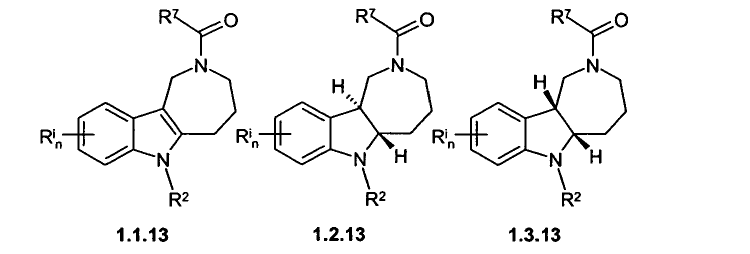 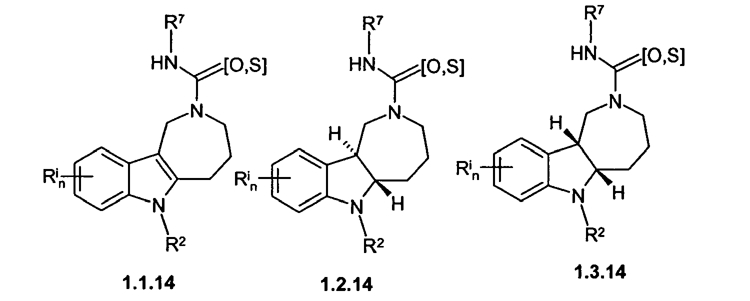 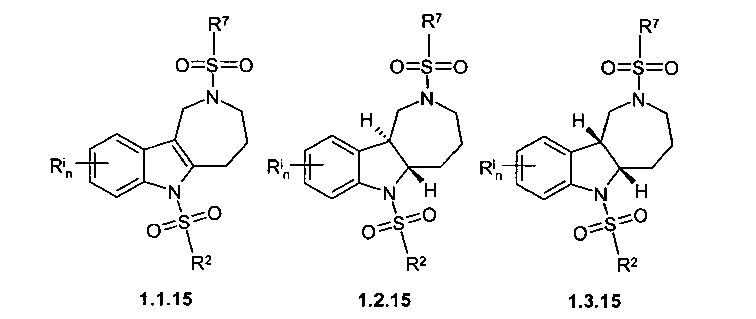 wherein:
R², R⁷ and Rⁱₙ have the above meanings.

Subject matter of the present invention is also a method for preparation of hydrogenated azepino[4,3-b]indoles of general formula **1,** racemates, optical isomers, geometrical isomers, pharmaceutically acceptable salts and/or hydrates thereof.

According to the invention a method for preparation of substituted 1,2,3,4,5,6-hexahydroazepino[4,3-b]indoles of general formulas **1.1.1, 1.1.2** consists in the reduction of the appropriate 3,4,5,6-tetrahydro-2H-azepino[4,3-b]indol-1-ones of general formulas **3, 4,** derived from oximes of general formula **2** with LiAlH₄ in organic solvent, for example, tetrahydrofuran. 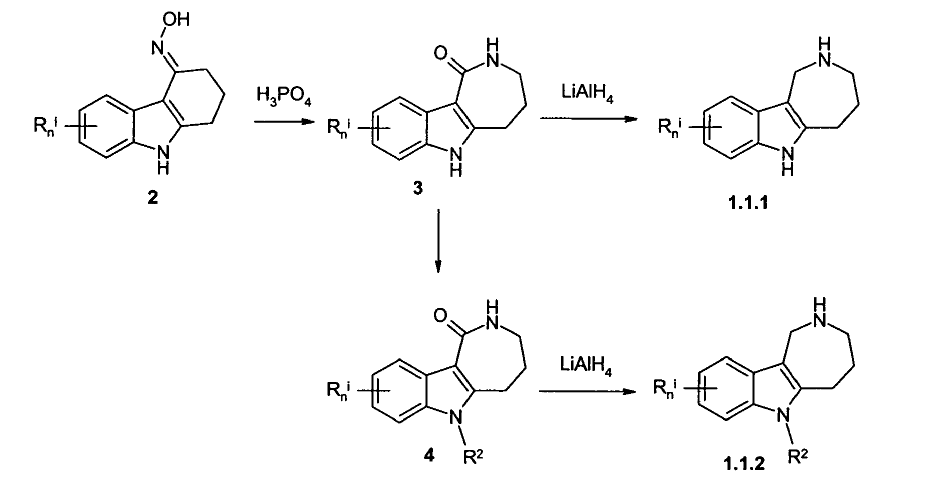 wherein:
R² and Rⁱₙ have the above meanings.

According to the invention a method for preparation of 2-BOC-substituted azepino[4,3-b]indoles of general formulas **1.1.2.1** or **1.1.3.1** consists in interaction of the suitable azepino[4,3-b]indoles of general formulas **1.1.1** or **1.1.2** with BOC-anhydride. 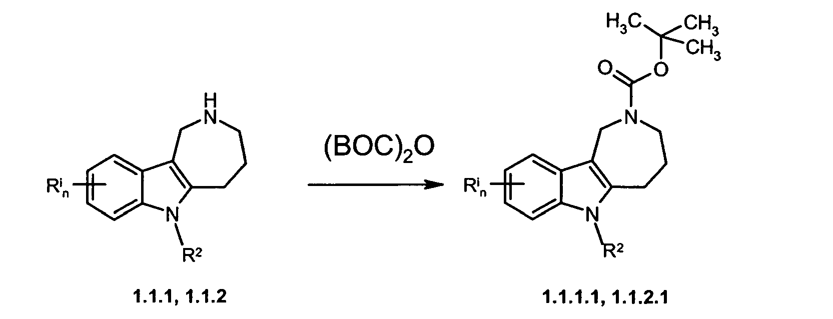 wherein:
R² and Rⁱₙ have the above meanings.

According to the invention a method for preparation of substituted 1,2,3,4,5,6-hexahydroazepino[4,3-b]indoles of general formula **1.1.3,** among them: **1.1.3.1, 1.1.3.2, 1.1.3.3, 1.1.3.4, 1.1.3.5, 1.1.3.6** consists in interaction of compounds of general formula **1.1.1** in organic solvent with the following electrophylic reagents, respectively: alkyl-, aryl- or heterocyclyl halides of general formula **5** in presence of base; electrophylic alkenes of general formula **6** in presence of base catalyst; aldehydes of general formula **7** and NaBH(AcO)₃; anhydrides or carbonic acid halides of general formula **8** in presence of base, iso(thio)cyanates of general formula **9** or sulfonyl chlorides of general formula **10** in presence of base. 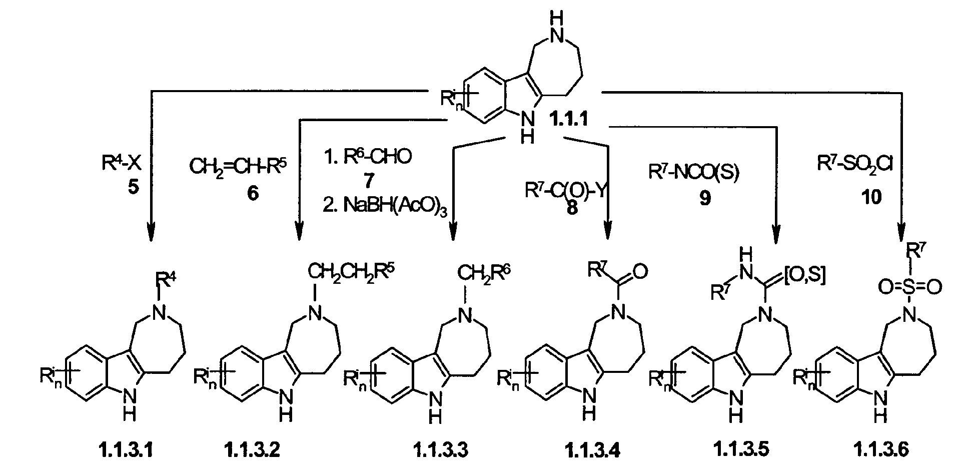 wherein:
R⁴, R⁵, R⁶, R⁷ and Rⁱₙ have the above meanings; X represents a halogen atom; Y is selected from halogen, 3H-imidazol-1-ium hydroxide, R⁷-C(O)O.

According to the invention a method for preparation of *trans*-1,2,3,4,5,5a,6,10b-octahydroazepino[4,3-b]indoles of general formula **1.2,** among them **1.2.1, 1.2.2, 1.2.3,** and/or *cis*-1,2,3,4,5,5a,6,10b-octahydroazepino[4,3-b]indoles of general formula **1.3** among them **1.3.1, 1.3.2, 1.3.3,** consists in reduction of C₅ₐ-C_{10b} double bond in the corresponding 1,2,3,4,5,6-hexahydroazepino[4,3-b]indoles of general formula **1.1,** among them **1.1.1, 1.1.2, 1.1.3,** by borane (BH₃) or its derivatives in organic solvent. 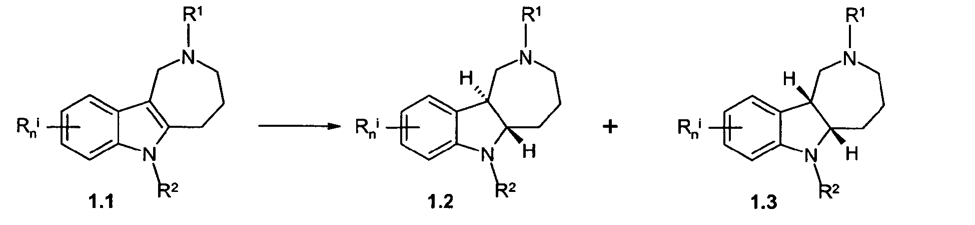 wherein:
R¹, R² and Rⁱₙ have the above meanings.

According to the invention a method for preparation of compounds of general formulas **1.1.4, 1.1.5, 1.1.6, 1.1.7, 1.1.8, 1.1.9, 1.2.4, 1.2.5, 1.2.6, 1.2.7, 1.2.8, 1.2.9, 1.3.4, 1.3.5, 1.3.6, 1.3.7, 1.3.8** and **1.3.9,** with the exception of the compounds, wherein R¹=H, consists in interaction of the appropriate compounds **1.1.3, 1.2.3** or **1.3.3,** with the exception of the compounds wherein R¹=H, in organic solvent with electrophylic reagents, such as: alkyl-, aryl- or heterocyclyl halides of general formula **5** in presence of base; electrophylic alkenes of general formula **6** in presence of base as catalyst; aldehydes of general formula **7** and NaBH(AcO)₃; anhydrides or carbonic acid halides of general formula **8** in presence of base; iso(thio)cyanates of general formula **9** or sulfonyl chlorides of general formula **10** in presence of base. 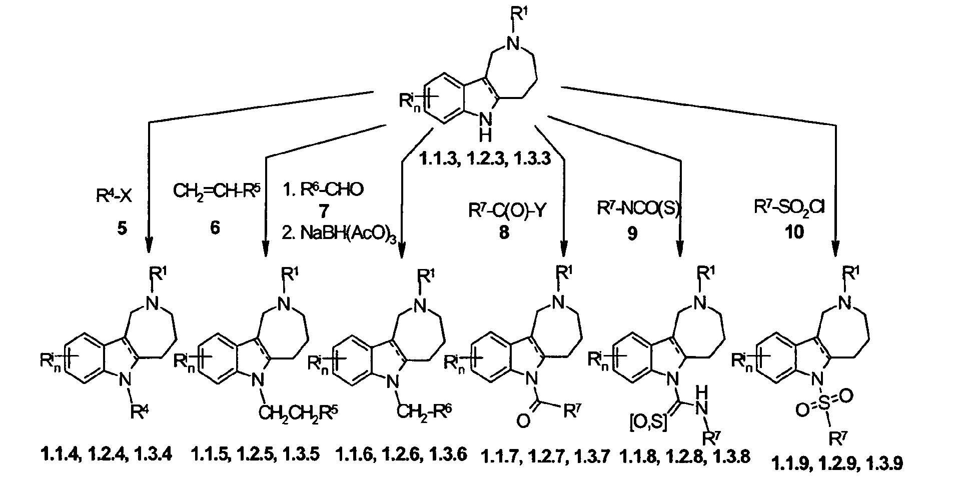 wherein:
(---), R¹, R⁴, R⁵, R⁶, R⁷, Rₙⁱ, X and Y have the above meanings, with the exception of the compounds, wherein R¹ = H.

According to the invention a method for preparation of the compounds of general formulas **1.1.1, 1.1.2, 1.2.1, 1.2.2, 1.3.1, 1.3.2,** among them **1.1.4.2, 1.1.5.2, 1.1.6.2, 1.1.7.2, 1.1.8.2, 1.1.9.2, 1.2.3.2, 1.2.4.2, 1.2.5.2, 1.2.6.2, 1.2.7.2, 1.2.8.2, 1.3.3.2, 1.3.4.2, 1.3.5.2, 1.3.6.2, 1.3.7.2, 1.3.8.2** by hydrolysis of the protective group in the corresponding compounds **1.1.4.1, 1.1.5.1, 1.1.6.1, 1.1.7.1, 1.1.8.1, 1.1.9.1, 1.2.3.1, 1.2.4.1, 1.2.5.1, 1.2.6.1, 1.2.7.1, 1.2.8.1, 1.3.3.1, 1.3.4.1, 1.3.5.1, 1.3.6.1, 1.3.7.1, 1.3.8.1.** 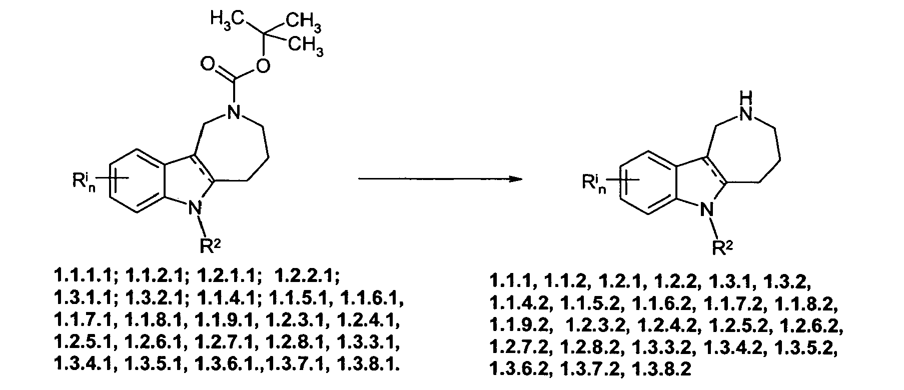 wherein:
(---), R² and Rⁱₙ have the above meanings.

According to the invention a method for preparation of the compounds of general formulas **1.1.10-1.1.15, 1.2.10-1.2.15** or **1.3.10-1.3.15** consists in interaction of the appropriate compounds **1.1.1, 1.1.2, 1.2.2** or **1.3.2** in organic solvent with electrophylic reagents, such as: alkyl-, aryl- or heterocyclyl halides of general formula **5** in presence of base; electrophylic alkenes of general formula **6** in presence of base catalyst; aldehydes of general formula **7** and NaBH(AcO)₃; anhydrides or carbonic acid halides of general formula **8** in presence of base; iso(thio)cyanates of general formula **9** or sulfonyl chlorides of general formula **10** in presence of base. 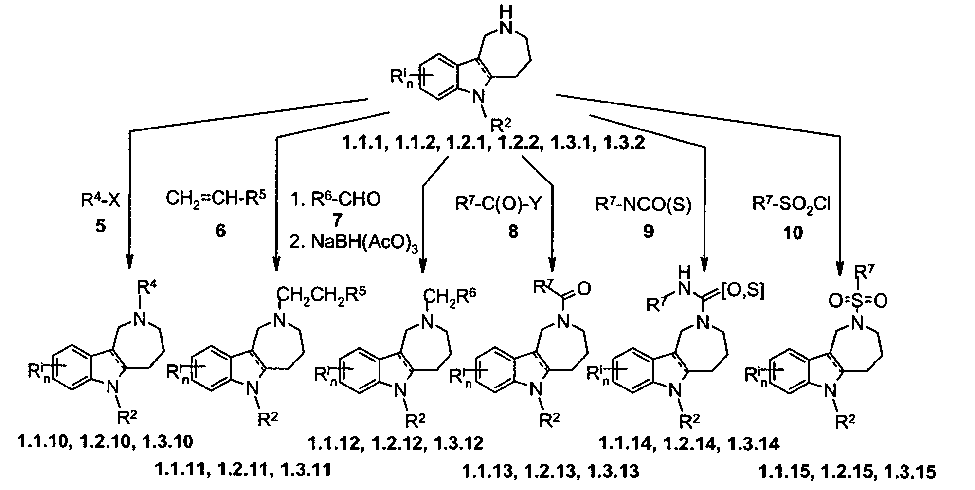 wherein:
(---), R², R⁴, R⁵, R⁶, R⁷, Rₙⁱ, X and Y have the above meanings.

The compounds of general formula **1** are prepared using methods known in the art for the preparation of analogous compounds or the novel methods described below.

According to the invention the novel substituted 1,2,3,4,5,6-hexahydroazepino[4,3-b]indoles of general formulas **1.1.1, 1.1.2** are prepared by reduction of the appropriate 3,4,5,6-tetrahydro-2H-azepino[4,3-b]indol-1-ones of general formulas **3, 4,** derived from oximes of general formula **2,** with LiAlH₄ in organic solvent, for example, tetrahydrofuran by analogy with the method given in [Rodriguez, J.G., del Valle, C., Esteban-Calderon, C., Martinez-Ripoll M. J. Chem. Crystallogr., 1995, 25(5), 2449-2457].

The reduction of C₅ₐ-C_{10b} double bond in 1,2,3,4,5,6-hexahydroazepino[4,3-b]indoles of general formula **1.1** may be performed by analogy with the reduction of C₃ₐ-C₈ₐ double bond in 1,2,3,8-tetrahydropyrrolo[4,3-b]indoles by suitable reducing agent, and leads selectively (depending on their type) to substituted *cis*-1,2,3,4,5,5a,6,10b-octahydroazepino[4,3-b]indoles of general formula 1.3 [N.K. Kotchetkov, N.F. Kutcherova, I.G. Jukova - J.Gen.Chem., 1961, v.31, Nº 3, p. 924-930 and N.F. Kutcherova, N.M. Sipilina, N.N. Novikova, I.D. Silenko, S.G. Rosenberg, V.A. Zagorevski - Khim.Geterotsikl.Soed., 1980, Nº 10, p. 1383-1386] or substituted *trans*-1,2,3,4,5,5a,6,10b-octahydroazepino[4,3-b]indoles of general formula **1.2** [V.A. Zagorevski, S.G. Rosenberg, N.M. Sipilina, L.Y. Bikova, A.P. Rodionov - Zn.Wses.Khim.Obch., 1982, v.27, N 1, p. 102-104; and J.G. Berger - Synthesis, 1974, Nº 7, p. 508-510]. In particular, *cis*-isomers of general formula **1.3** may be prepared by reduction of hexahydroderivatives of general formula **1.1** by hydrogen on Pt or by Zn (and analogous metals) in strong acid medium (for example, in hydrochloric acid and so on); or by borohydrides of alkali metals in anhydrous carboxylic acids (for example, in trifluoroacetic acid and so on) at 20-100°C; whereas trans-isomers of general formula **1.2** may be prepared by treating complexes of borane with hexahydroderivatives of general formula **1.1** (1:1) [prepared either from the corresponding bases and borane solution (in situ or after previous isolation) or from the corresponding hydrochlorides and sodium borohydride in ester solution] with mineral acids (for example, hydrochloric acid and so on) at heating.

Various amino group substituents R¹ and R² in hydrogenated azepino[4,3-b]indoles of general formula **1.1, 1.2, 1.3,** along with the methods described above, may be introduced by other well-known methods:
- alkyl derivatives may be prepared by alkylation of the appropriate hydrogenated azepino[4,3-b]indoles of general formula **1.1, 1.2, 1.3** with electrophylic agent R⁴-X of formula 5 in presence of strong base in a polar aprotic solvent such as DMF (dimethylformamide), DMSO (dimethyl sulfoxide), HMPA (hexamethylphosphorotriamide), for example, by analogy with [N.F. Kutcherova, N.M. Sharkova, V.A. Zagorevski - SU, 261386, 1965].
- compounds, containing substituted ethyl group wherein R⁵ represents CN or optionally substituted 2-, 3- or 4-pyridyl may be prepared by alkylation of the appropriate hydrogenated azepino[4,3-b]indoles of general formula **1.1, 1.2, 1.3** with electrophylic alkenes of general formula **6** in presence of catalyst - a base, as it is described in: [N.N.Kamzolova, N.F. Kutcherova, V.A. Zagorevski, J.Gen.Chem., 1964, 34, (7), 2383-2387; A.N. Kost, M.A. Urovskaya, T.V. Melnikova, O.I. Potanina - US, 367094, 1970]. The reaction is carried out wih an excess of reagent **6** (Y = CN) at room temperature in presence of catalyst, for example, quaternary ammonium or sodium alkoxide, sodium hydride, sodium ethoxide, and so on, in a polar aprotic solvent such as DMF, DMSO or HMPA;
- alkyl derivatives may be prepared also by reductive alkylation of the appropriate hydrogenated azepino[4,3-b]indoles of general formula **1.1, 1.2, 1.3** with aldehydes of general formula **7;**
- acyl derivatives, (thio)carbamoyl derivatives and sulfamoyl derivatives may be prepared by interaction of the appropriate hydrogenated azepino[4,3-b]indoles of general formula **1.1, 1.2, 1.3** with acylating agents - anhydrides or carboxylic acid halides of general formula **8,** alkyl-, cycloalkyl- or aryl-iso(thio)cyanates of general formula **9** or sulfonyl chlorides of general formula **10,** for example, by analogy with [N.K. Kotchetkov, N.F. Kutcherova, I.G. Gykova, J.Gen.Chem., 1961, 31, (3), 924-930].

Hydrogenated azepino[4,3-b]indoles of general formula **1** of the present invention may form hydrates or pharmaceutically acceptable salts. Salts may be prepared with the use of mineral acids and organic acids, for example, hydrochloric acid, hydrobromic acid, hydriodic acid, sulfuric acid, phosphoric acid, formic acid, acetic acid, propionic acid, trifluoroacetic acid, maleic acid, tartaric acid, methanesulphonic acid, benzenesulfonic acid, p-toluenesulfonic acid.

Hydrates are usually prepared upon recrystallization of compounds of general formula **1** or their salts from water or water containing solvents.

Subject matter of the present invention is also a combinatorial library of compounds exhibiting neuroprotective, cognitive stimulating and antihistaminic activity for determining hit compounds and leader compounds, composed of compounds of the general formula **1.**

Subject matter of the present invention is also a focused library of compounds exhibiting neuroprotective, cognitive stimulating and antihistaminic activity for determining and/or optimization of leader compounds containing at least one compound of general formula **1.**

According to the invention hydrogenated azepino[4,3-b]indoles of general formula **1,** racemates, optical isomers, geometrical isomers, pharmaceutically acceptable salts and/or hydrates thereof may be used as an active ingredient for treatment and prophylaxis of various diseases.

Subject matter of the present invention is also a pharmaceutical composition exhibiting neuroprotective, cognitive stimulating and antihistaminic activity for treatment of diseases patogenesis of which is associated with an excessive intracellular Ca⁺² ions concentration at animals and humans, and/or diseases determined by the disorder of histaminergetic mediator system, in the form of tablets, sheaths or injections placed in pharmaceutically acceptable packing, comprising as an active ingredient at least one hydrogenated azepino[4,3-b]indole of general formula **1,** either racemate, or optical isomer, or pharmaceutically acceptable salt and/or hydrate thereof.

A further object of the present invention is also a method for preparation of pharmaceutical composition by mixing an active ingredient with an exicipient and/or solvent, **characterized in that** as active ingredient at least one hydrogenated azepino[4,3-b]indole of general formula **1,** either racemate, or optical isomer, or geometrical isomers or acceptable salt and/or hydrate thereof in pharmacologically effective amount is used.

A further object of the present invention is the use of a pharmaceutical composition for preparation of medicaments for prophylaxis and treatment of various diseases of warm-blooded animals and humans patogenesis of which is associated with an excessive intracellular Ca⁺² ions concentration at animals and humans, and/or diseases determined by the disorder of histaminergetic mediator system.

A further object of the present invention is the use of a pharmaceutical composition for preparation of medicaments for treatment of neurological disorders (in particular, hypoxia-ischemia, hypoglycemia, convulsive conditions, brain traumas, and so on) and also neurodegenerative diseases (among them Alzheimer's disease, Huntington's chorea, lathyrism, amyotrophic lateral sclerosis and so on).

A further object of the present invention is also the use of a pharmaceutical composition for preparation of medicaments for treatment of allergic and autoimmune diseases including pollinosis, hives, a bronchial asthma, atopic dermatitis, neurodermatitis, angioneurotic hypostasis, Quincke's disease, eczema, ambustial toxemia, and also the allergic reactions caused by medicines, foodstuff, cosmetics, dust, insect stings and so on.

A further object of the present invention is also the use of a pharmaceutical composition for preparation of medicaments for enhancement of memory processes and cognitive stimulation at humans and warm-blooded animals.

If necessary, to use pharmaceutical compositions of the present invention in clinical practice, they may be mixed to prepare different forms, in which case they may comprise traditional pharmaceutical carries; for example, peroral forms (such as, tablets, gelatinous capsules, pills, solutions or suspensions); injectable forms (such as, injectable solutions or suspensions, or a dry injectable powder that only requires injectable water to be added before use); and local forms (such as ointments or solutions).

The carriers used in pharmaceutical compositions of the present invention are those that are used in pharmaceutics to produce common forms, in particular, binding and wetting agents, disintegrators, solvents, diluents, stabilizers, suspending agents, colorless agents, and taste modifying agents are used in peroral forms; antiseptics, solubilizers, and stabilizers are used in injectable forms; and bases, diluents, lubricating agents, and antiseptics are used in local forms. Medicaments may be administered perorally or parenterally (for example, intravenously, subcutaneously, intraperitoneally or locally). If any medicament is not stable in stomach, it is possible to use it for manufacturing the tablets coated by film made of material soluble in stomach or intestinal canal.

Besides, the clinical dose of hydrogenated azepino[4,3-b]indole of general formula **1,** either racemate, or optical isomer, or geometrical isomer, or acceptable salt and/or hydrate thereof may be corrected depending on: therapeutic efficiency and bio-accessibility of active ingredients in their organism, the speed of their exchange and removal from organism, and also the age, gender, and severity of the patient's symptoms. Thus, the daily intake for adults normally being 10∼500 mg, preferably 50∼300 mg. Accordingly, the above effective doses are to be taken into consideration while preparing a medicament of the present invention from the pharmaceutical composition in the form of dose units, each dose unit of the preparation containing 10∼500 mg of the compound of general formula **1,** preferably 50∼300 mg. Following the instructions of a physitian or pharmacist, the preparations may be taken several times over specified periods of time (preferably, from one to six times).

A further object of the present invention are also physiologically active compounds of general formula **1** exhibiting neuroprotective, cognitive stimulating, and antihistaminic activity, and also ability to regulate cytosolic Ca⁺² ions concentration in neurones, intended for an experimental research of physiological processes *in vivo* and *in vitro* as "pharmacological tools".

### Best Embodiment of the invention

Following below are specific examples that illustrate this invention, but not limit it thereto.

**Example 1.** General method for preparation of 3,4,5,6-tetrahydro-2H-azepino[4,3-b]indol-1-ones of general formula **4.**

These compounds have been prepared from the appropriate oximes of general formula **2** according to the method described for the preparation of the following analogues: 6-H- **4(1), 6-**methyl- and 6-tosyl-3,4,5,6-tetrahydro-2H-azepino[4,3-b]indol-1-ones [Rodriguez, J.G., del Valle, C., Esteban-Calderon, C., Martinez-Ripoll M. J. Chem. Crystallogr., 1995, 25(5), 2449-2457], among them: 3,4,5,6-tetrahydro-2H-azepino[4,3-b]indol-1-one **4(1),** LCMS: m/z 201 [M+H]; 9-methyl-3,4,5,6-tetrahydro-2H-azepino[4,3-b]indol-1-one **4(2),** LCMS: m/z 215 [M+H]; 9-fluoro-3,4,5,6-tetrahydro-2H-azepino[4,3-b]indol-1-one **4(3),** LCMS: m/z 219 [M+H]; 9-bromo-3,4,5,6-tetrahydro-2H-azepino[4,3-b]indol-1-one **4(4),** LCMS: m/z 280 [M+H]; 7-bromo-3,4,5,6-tetrahydro-2H-azepino[4,3-b]indol-1-one **4(5),** LCMS: m/z 280 [M+H]; 7,9-dimethyl-3,4,5,6-tetrahydro-2H-azepino[4,3-b]indol-1-one **4(6),** LCMS: m/z 229 [M+H]; 9-(3-fluorophenyl)-3,4,5,6-tetrahydro-2H-azepino[4,3-b]indol-1-one **4(7),** LCMS: m/z 295 [M+H]; 9-(pyridin-3-yl)-3,4,5,6-tetrahydro-2H-azepino[4,3-b]indol-1-one **4(8),** LCMS: m/z 278 [M+H]; 9-(pyridin-4-yl)-3,4,5,6-tetrahydro-2H-azepino[4,3-b]indol-1-one **4(9),** LCMS: m/z 278 [M+H]; 9-(pyrimidin-5-yl)-3,4,5,6-tetrahydro-2H-azepino[4,3-b]indol-1-one **4(10),** LCMS: m/z 279 [M+H]; 7-(pyridin-3-yl)-3,4,5,6-tetrahydro-2H-azepino[4,3-b]indol-1-one **4(11),** LCMS: m/z 278 [M+H] and others.

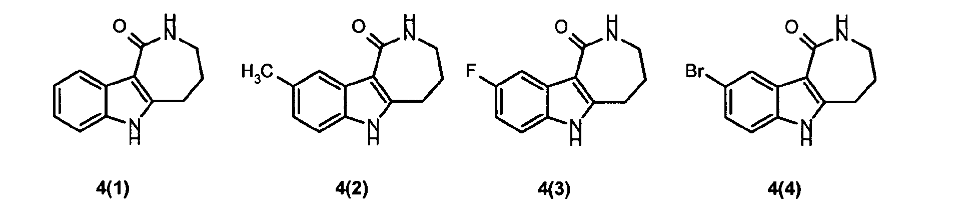

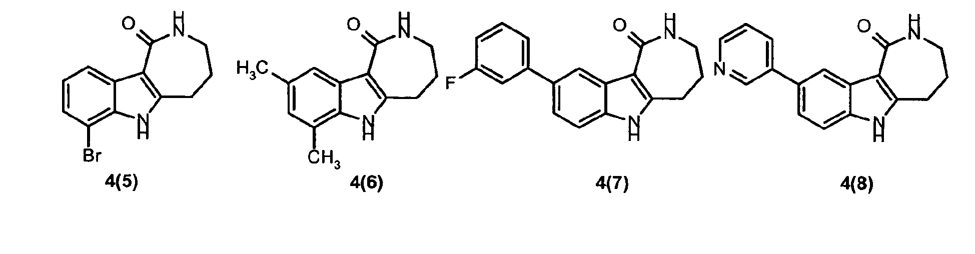

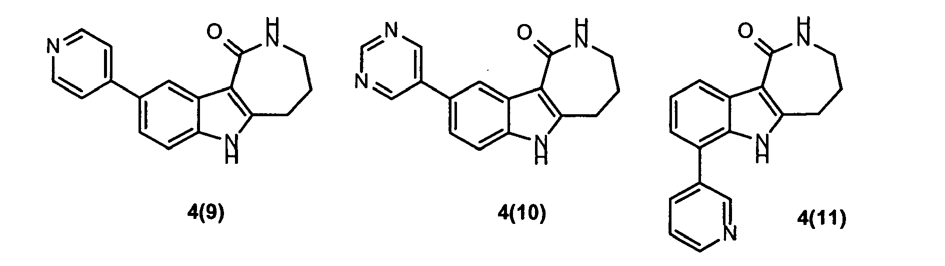

**Example 2.** General method for preparation of 3,4,5,6-tetrahydro-2H-azepino[4,3-b]indol-1-ones of general formula **4.**

110 Mg (2.7 mmol) of 60% NaH dispersion in mineral oil is added to a solution of 2 mmol of 3,4,5,6-tetrahydro-2H-azepino[4,3-b]indol-1-one **3** in 5 ml of dry DMF at stirring. After ceasing the effervescence of hydrogen the mixture is stirred for additional 30 min under argon atmosphere at room temperature. Then 2.6 mmol of alkyl halide is added and stirring is continued for 5 hr at 30-50°C. Upon completion of the reaction (LCMS monitoring) the reaction mixture is decomposed by adding 50 ml of water, the product is extracted with dichloromethane, extract is dried over Na₂SO₄. The solvent is evaporated *in vacuo*, the residue is recrystallised from a suitable solvent or purified by column chromatography eluting with DCM-THF-EtOH 7:3:0.5 mixture. It gives 3,4,5,6-tetrahydro-2H-azepino[4,3-b]indol-1-ones **4,** yield 64-85%, among them: 6-benzyl-9-methyl-3,4,5,6-tetrahydro-2H-azepino[4,3-b]indol-1-one **4(12),** LCMS: m/z 305 [M+H]; 6-(4-fluorobenzyl)-9-methyl-3,4,5,6-tetrahydro-2H-azepino[4,3-b]indol-1-one **4(13),** LCMS: m/z 323 [M+H]; 6-benzyl-9-fluoro-3,4,5,6-tetrahydro-2H-azepino[4,3-b]indol-1-one **4(14),** LC-MS: m/z 309 [M+H]; 6-(pyridin-3-ylmethyl)-3,4,5,6-tetrahydro-2H-azepino[4,3-b]indol-1-one **4(15),** LCMS: m/z 292 [M+H] and others.

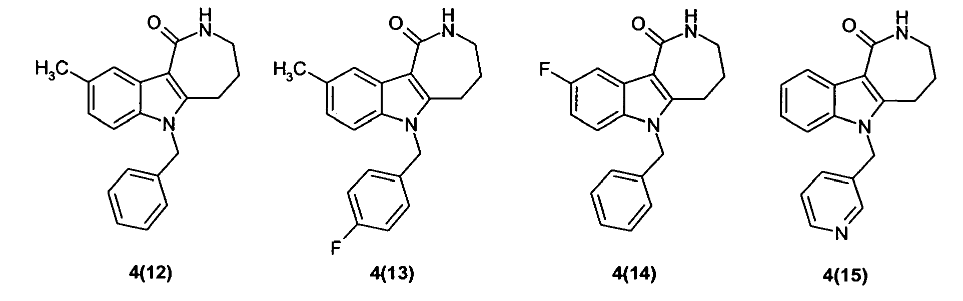

**Example 3.** General methods for preparation of 1,2,3,4,5,6-hexahydroazepino[4,3-b]indoles of general formula **1.1.1, 1.1.2.**
**A.** These compounds may be prepared by reduction of the proper 3,4,5,6-tetrahydro-2H-azepino[4,3-b]indol-1-ones of general formula **4** with LiAlH₄ according to the method described for preparation of 1,2,3,4,5,6-hexahydroazepino[4,3-b]indole **1.1.1(1)** [Bascop, S.-I.; Laronze, J.-Y.; Sapi, J. Monatsh. Chemie 1999, 130, 1159-1166], among them: 1,2,3,4,5,6-hexahydroazepino[4,3-b]indole **1.1.1(1),** LCMS: m/z 187 [M+H]; 9-methyl-1,2,3,4,5,6-hexahydroazepino[4,3-b]indole **1.1.1(2),** LCMS: m/z 201 [M+H]; 9-fluoro-1,2,3,4,5,6-hexahydroazepino[4,3-b]indole **1.1.1(3),** LCMS: m/z 205 [M+H]; 7,9-dimethyl-1,2,3,4,5,6-hexahydroazepino[4,3-b]indole **1.1.1(4),** LCMS: m/z 215 [M+H]; 9-(3-fluorophenyl)-1,2,3,4,5,6-hexahydroazepino[4,3-b]indole **1.1.1(5),** LCMS: m/z 281 [M+H]; 9-(pyridin-3-yl)-1,2,3,4,5,6-hexahydroazepino[4,3-b]indole **1.1.1(6),** LCMS: m/z 264 [M+H]; 9-(pyridin-4-yl)-1,2,3,4,5,6-hexahydroazepino[4,3-b]indole **1.1.1(7),** LCMS: m/z 264 [M+H]; 9-(pyrimidin-5-yl)-1,2,3,4,5,6-hexahydroazepino[4,3-b]indole **1.1.1(8),** LCMS: m/z 265 [M+H]; 7-(pyridin-3-yl)-1,2,3,4,5,6-hexahydroazepino[4,3-b]indole **1.1.1(9),** LCMS: m/z 264 [M+H]; 6-benzyl-9-methyl-1,2,3,4,5,6-hexahydroazepino[4,3-b]indole **1.1.2(1),** LCMS: m/z 291 [M+H]; 6-(4-fluorobenzyl)-9-methyl-1,2,3,4,5,6-hexahydroazepino[4,3-b]indole **1.1.2(2),** LCMS: m/z 308 [M+H]; 6-benzyl-9-fluoro-1,2,3,4,5,6-hexahydroazepino[4,3-b]indole **1.1.2(3),** LCMS: m/z 295 [M+H]; 6-(pyridin-3-ylmethyl)-1,2,3,4,5,6-hexahydroazepino[4,3-b]indole **1.1.2(4),** LCMS: m/z 248 [M+H] and others. 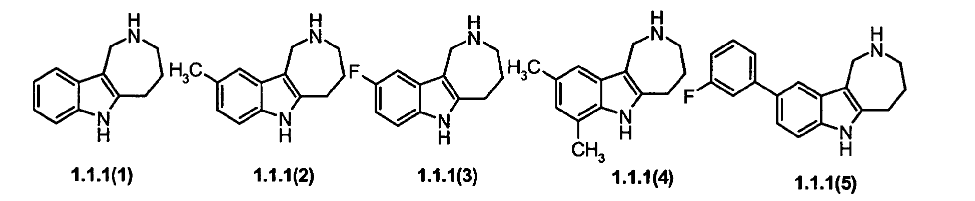 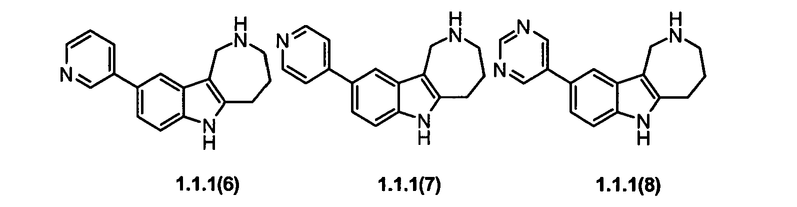 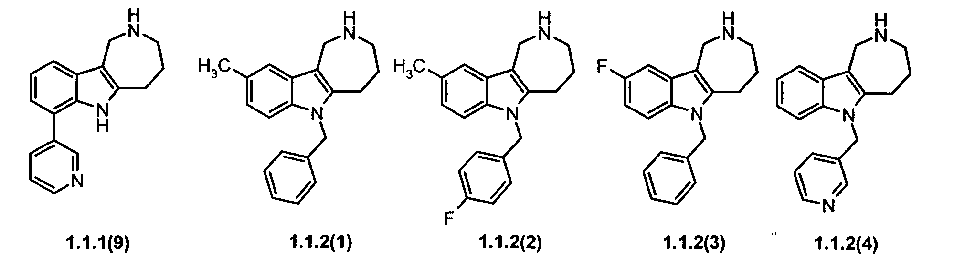
**B.** Method for preparation of *trans-* **1.2.1(1)** and *cis*-1,2,3,4,5,5a,6,10b-octahydroazepino[4,3-b]indoles **1.3.1(1).** 5 Ml of 1M BH₃-THF in THF is added to a solution of 400 mg (2 mmol) of 1,2,3,4,5,6-hexahydroazepino[4,3-b]indole **1.1.1(2)** in 10 ml of dry THF at 0°C. The mixture is refluxed for 3 hr. The solvent and excess of borane are evaporated *in vacuo*. 15 Ml of 17,5% HCl water solution is added to the residue and the resultant mixture is boiled for 15 min until clear solution is obtained. After cooling and alkalization with saturated NaOH water solution, the product is extracted with ester. The solvent is evaporated, the residue is purified by column chromatography on silica gel impregnated with triethylamine (eluent: hexane-CHCl₃-Et₃N 4:4:2). It gives: *cis*-1,2,3,4,5,5a,6,10b-octahydroazepino[4,3-b]indole **1.3.1(1):** R_{f} 0.55-0.65; LCMS: m/z 203 [M+H]; ¹H NMR (400 MHz, CDCl₃): 6.82-6.80 (m, 2H), 6.47-6.45 (m, 2H), 4.14-4.09 (m, 1H), 3.63-3.57 (m, 1H), 3.20-3.16 (m, 1H), 3.00-2.93 (m, 2H), 2.75-2.69 (m, 1H), 2.23 (s, 3H), 1.87-1.75 (m, 3H), 1.57-1.48 (m, 1H); ¹³C NMR (CDCl₃): δ= 20.66, 28.26, 33.01, 48.96, 51.13, 51.98, 62.41, 108.47, 124.67, 127.26, 127.91, 130.49, 148.35 and *trans-*1,2,3,4,5,5a,6,10b-octahydroazepino[4,3-b]indole **1.2.1(1):** R_{f} 0.40-0.55; LCMS: m/z 203 [M+H]; ¹H NMR (400 MHz, CDCl₃): 6.82-6.80 (d, 1H, J=7.1 Hz), 6.77 (s, 1H), 6.51 (d, 1H, J=7.1 Hz), 3.81-3.75 (m, 1H), 3.66-3.61 (m, 1H), 3.22-3.15 (m, 1H), 2.95-2.81 (m, 3H), 2.23 (s, 3H), 2.16-2.10 (m, 1H), 1.87-1.70 (m, 2H), 1.65-1.57 (m, 1H); ¹³C NMR (CDCl₃): δ= 20.72, 29.44, 33.57, 46.02, 49.50, 49.75, 66.41, 108.89, 123.39, 127.51, 127.94, 131.95, 148.43. 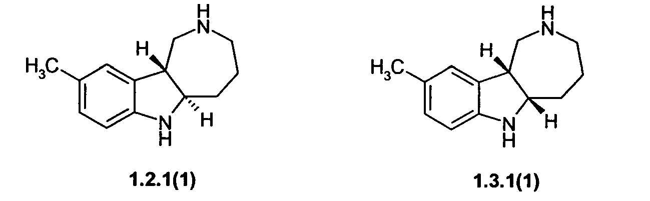
   Determination of *trans-* **1.2.1(1)** and *cis-* **1.3.1(1)** isomers is carried out on the basis of NOESY results for both isomers. Below the structural models of *trans-* **1.2.1(1)** and cis-isomers **1.3.1(1),** obtained using MOPAC method are shown. 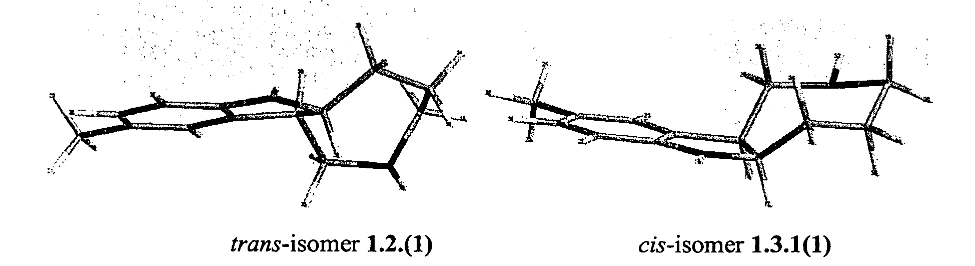
**C.** A mixture of 2 mmol of 1,2,3,4,5,6-hexahydroazepino[4,3-b]indole **1.1.1** or **1.1.3** and 500 mg (8 mmol) of NaBH₃CN in 6 ml of acetic acid is stirred at room temperature for 12 hr (LCMS monitoring). Upon completion of the reaction acetic acid is evaporated *in vacuo*, the residue is refluxed for 10 min with 17,5% HCl water solution. The clear solution is cooled, alkalized with saturated water NaOH, the product is extracted with suitable organic solvent. The extract is dried over Na₂SO₄ and evaporated *in vacuo*. The residue is purified by colomn chromatography on silica gel impregnated with triethylamine (eluent: hexane-CHCl₃-Et₃N 4:4:2). It gives *cis*-1,2,3,4,5,5a,6,10b-octahydroazepino[4,3-b]indoles of general formula **1.3,** among them: *cis*-9-methyl-1,2,3,4,5,5a,6,10b-octahydroazepino[4,3-b]indole **1.3.1(1),** LCMS: m/z 203 [M+H], ¹H NMR (400 MHz, CDCl₃): 6.82-6.80 (m, 2H), 6.47-6.45 (m, 2H), 4.14-4.09 (m, 1H), 3.63-3.57 (m, 1H), 3.20-3.16 (m, 1H), 3.00-2.93 (m, 2H), 2.75-2.69 (m, 1H), 2.23 (s, 3H), 1.87-1.75 (m, 3H), 1.57-1.48 (m, 1H), ¹³C NMR (CDCl₃): δ= 20.66, 28.26, 33.01, 48.96, 51.13, 51.98, 62.41, 108.47, 124.67, 127.26, 127.91, 130.49, 148.35; *cis*-9-fluoro-1,2,3,4,5,5a,6,10b-octahydroazepino[4,3-b]indole **1.3.1(2),** LCMS: m/z 207 [M+H]; *cis*-9-(3-fluorophenyl)-1,2,3,4,5,5a,6,10b-octahydroazepino[4,3-b]indole **1.3.1(3),** LCMS: m/z 283 [M+H]; *cis*-2-tert-butoxycarbonyl-9-methyl-1,2,3,4,5,5a,6,10b-octahydroazepino[4,3-b]indole **1.3.3(1),** LCMS: m/z 303 [M+H] and others. 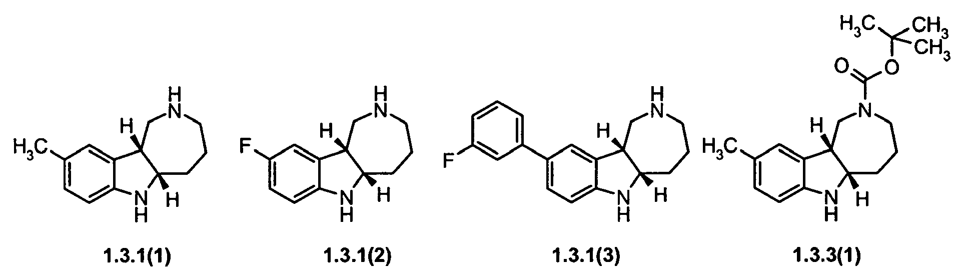
**D.** 2 M1 of polyphosphoric acid is added to a solution of 1,4 mmol of azepino[4,3-b]indoles **1.1.13, 1.2.13** or **1.3.3(1)** in 20 ml of tetrahydrofuran and the resultant mixture is stirred for 48 hr at 50°C (LCMS monitoring). Upon completion of the reaction the mixture is neutralized with 40% NaOH water solution, organic layer is separated and dried over K₂CO₃. The solvent is evaporated *in vacuo*, and the residue is purified by colomn chromatography on silica gel impregnated with triethylamine (eluent - chloroform - triethylamine 8:2 mixture). It gives 1,2,3,4,5,6-hexahydro-1H-azepino[4,3-b]indoles, among them: ethyl (9-methyl-1,2,3,4,5,6-hexahydro-1H-azepino[4,3-b]indol-6-yl)acetate **1.1.2(5),** LCMS: m/z 287 [M+H], ¹H NMR (400 MHz, DMSO-d₆): 7.21-7.17 (m, 2H), 6.86 (d, 1H, J=6.8 Hz), 5.01 (s, 2H), 4.18-4.12 (m, 2H), 3.93 (s, 2H), 3.07 (m, 2H), 2.79 (m, 2H), 2.36 (s, 3H), 1.82-1.75 (m, 2H), 1.20 (m, 3H); ethyl *trans*-(9-methyl-1,2,3,4,5,5a,6,10b-octahydroazepino[4,3-b]indol-6-yl)acetate **1.2.2(1),** LCMS: m/z 289 [M+H]; ethyl *cis*-(9-methyl-1,2,3,4,5,5a,6,10b-octahydroazepino[4,3-b]indol-6-yl)acetate **1.3.2(1),** LCMS: m/z 289 [M+H]; ethyl *cis*-3-(9-methyl-1,2,3,4,5,5a,6,10b-octahydroazepino[4,3-b]indol-6-yl)propionate **1.3.2(2),** LCMS: m/z 303 [M+H]; *cis*-6-benzyl-9-methyl-1,2,3,4,5,5a,6,10b-octahydroazepino[4,3-b]indole **1.3.2(3),** LCMS: m/z 293 [M+H]; *cis*-9-methyl-6-[2-(pyridin-2-yl)ethyl]-,2,3,4,5,5a,6,10b-octahydroazepino[4,3-b]indole **1.3.2(4),** LCMS: m/z 308 [M+H]; *cis*-9-methyl-6-[2-(pyridin-3-yl)ethyl]-1,2,3,4,5,5a,6,10b-octahydroazepino[4,3-b]indole **1.3.2(5),** LCMS: m/z 308 [M+H]; *cis*-9-methyl-6-[2-(4-methylpyridin-3-yl)ethyl]-1,2,3,4,5,5a,6,10b-octahydroazepino[4,3-b]indole **1.3.2(6),** LCMS: m/z 322 [M+H]; *cis*-9-methyl-6-[2-(pyridin-4-yl)ethyl]-1,2,3,4,5,5a,6,10b-octahydroazepino[4,3-b]indole **1.3.2(7),** LCMS: m/z 308 [M+H] and others.

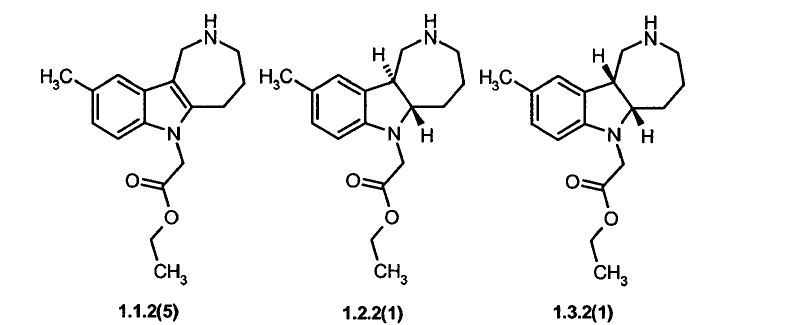

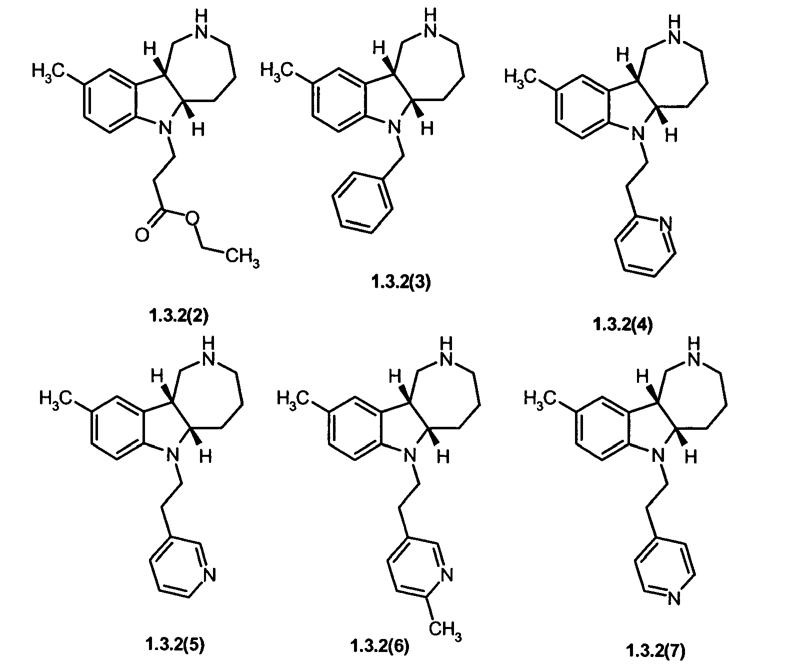

**Example 4.** General methods for preparation of 1,2,3,4,5,6-hexahydroazepino[4,3-b]indoles of general formula **1.1.3.**
**A.** A mixture of 1 mmol of azepino[4,3-b]indole **1.1.1,** 2 ml of dimethyl sulfoxide, 1,2 mmol of the desired freshly distilled vinyl derivate **6** and 15 mg (0,1 mmol) of MTBD is stirred under argon atmosphere at 20°C for 2-4 hr. Upon completion of the reaction (LCMS monitoring) the reaction mixture is dissolved in 50 ml of dichloromethane, the solution is washed twice with diluted K₂CO₃ water solution, dried over Na₂SO₄, evaporated, and the residue is purified by column chromatography on silica gel impregnated with triethylamine. It gives compounds **1.1.3.2,** among them: ethyl 3-(9-methyl-1,2,3,4,5,6-hexahydroazepino[4,3-b]indol-2-yl)propionate **1.1.3.2** (1), LCMS: m/z 301 [M+H], ¹H NMR (400 MHz, DMSO-d₆): 10.65 (br. s, 1H), 7.17 (s, 1H), 7.12 (d, 1H, J=7.0 Hz), 6.79 (d, 1H, J=7.0 Hz), 4.04 (m, 2H), 3.85 (br. s, 2H), 3.04 (m, 2H), 2.83 (m, 2H), 2.68 (m, 2H), 2.45 (m, 2H), 2.35 (s, 3H), 1.70 (br. s, 2H), 1.16 (m, 3H); 9-methyl-2-phenethyl-1,2,3,4,5,6-hexahydroazepino[4,3-b]indole **1.1.3.3(2),** LCMS: m/z 305 [M+H] and others. 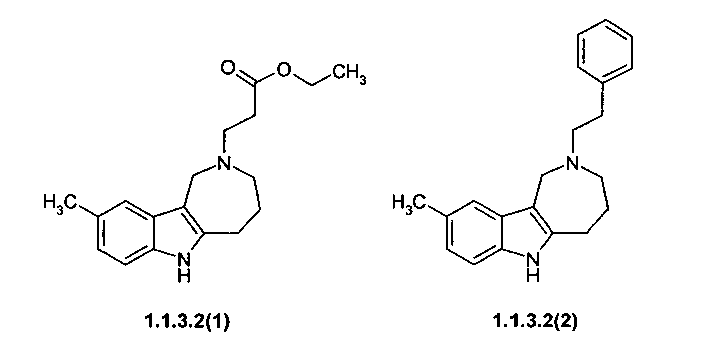
**B.** 3.6 Ml of formaldehyde solution in water is added to a solution of 0,04 mol of azepino[4,3-b]indole **1.1.1** in 600 ml of ethanol or dichloroethane, and the resultant mixtire is stirred for 30 min at room temperature. The flask is filled with argon, 600 mg of PtO₂ is inserted, the flask is blown through with hydrogen, and stirring is continued in atmosphere of hydrogen at room temperature for 48 hr (LCMS monitoring); or 0,05 mol of NaBH(AcO)₃ is added and the reaction mixture is stirred at room temperature for 2 hr till the reaction is completed (LCMS monitoring). After that the reaction mixture is filtered off, filtrate is evaporated *in vacuo*, the solid residue is washed with water and dried *in vacuo*. If needed, the product is purified by column chromatography on silica gel impregnated with triethylamine eluting with 10% solution of triethylamine in dichloromethane. It gives the desired 2-methyl substituted **1.1.3.3,** yield 61-75%, among them: 2,9-dimethyl-1,2,3,4,5,6-hexahydroazepino[4,3-b]indole **1.1.3.3(1),** LCMS: m/z 320 [M+H], ¹H NMR (400 MHz, DMSO-d₆): 8.58 (br.s, 1H), 7.67 (m, 1H), 7.27 (m, 3H), 7.09 (m, 1H), 6.90 (m, 1H), 4.50 (m, 2H), 3.87 (br.s, 2H), 3.08 (m, 2H), 2.96 (m, 2H), 2.76 (m, 2H), 2.41 (s, 3H), 2.37 (s, 3H), 1.70 (m, 2H); 2-methyl-9-fluoro-1,2,3,4,5,6-hexahydroazepino[4,3-b]indole **1.1.3.3(2),** LCMS: m/z 219 [M+H] and others. 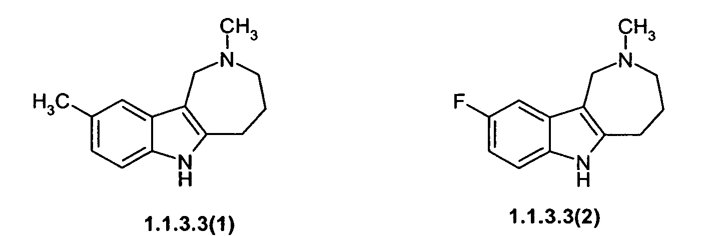
C. 0.4 Mmol of the proper aldehyde of general formula **7** and 170 mg (0.8 mmol) of NaBH(AcO)₃ are added to a solution of 0.32 mmol of azepino[4,3-b]indole 1.1.1 in 3 ml of dichloroethane. The mixture is stirred at room temperature for 2-6 hr (LCMS monitoring). Upon completion of the reaction 10 ml of water and 1 ml of 10% K₂CO₃ water solution are added to the mixture. Organic phase is separated, dried over K₂CO₃ and evaporated. The product is separated from the residue by preparative chromatography on silica gel impregnated with triethylamine, eluting with hexane-ethylacetate-triethylamine (7:2:1) mixture. It gives compounds **1.1.3.3,** yield 60-70%, among them: 2-(pyridin-3-ylmethyl)-1,2,3,4,5,6-hexahydroazepino[4,3-b]indole 1.1.3.3(3), LCMS: m/z 278 [M+H]; 9-methyl-2-(thiophen-2-ylmethyl)-1,2,3,4,5,6-hexahydroazepino[4,3-b]indole **1.1.3.3(4),** LCMS: m/z 297 [M+H]; 2-(pyridine-3-ylmethyl)-9-fluoro-1,2,3,4,5,6-hexahydroazepino[4,3-b]indole **1.1.3.3(5),** LCMS: m/z 296 [M+H]; 2-benzyl-9-bromo-1,2,3,4,5,6-hexahydroazepino[4,3-b]indole **1.1.3.3(6),** LCMS: m/z 356 [M+H]; 2-benzyl-7-bromo-1,2,3,4,5,6-hexahydroazepino[4,3-b]indole **1.1.3.3(7),** LCMS: m/z 356 [M+H]; 7,9-dimethyl-2-(3-methoxybenzyl)-1,2,3,4,5,6-hexahydroazepino[4,3-b]indole **1.1.3.3(8),** LCMS: m/z 335 [M+H]; 2-benzyl-9-(3-fluorophenyl)-1,2,3,4,5,6-hexahydroazepino[4,3-b]indole **1.1.3.3(9),** LCMS: m/z 371 [M+H]; 2-(3-methoxybenzyl)-9-(pyridin-3-yl)-1,2,3,4,5,6-hexahydroazepino[4,3-b]indole **1.1.3.3(10),** LCMS: m/z 384 [M+H]; 2-(4-chlorobenzyl)-9-(pyridin-4-yl)-1,2,3,4,5,6-hexahydroazepino[4,3-b]indole **1.1.3.3(11),** LCMS: m/z 388 [M+H]; 2-benzyl-9-(pyrimidin-5-yl)-1,2,3,4,5,6-hexahydroazepino[4,3-b]indole **1.1.3.3(12),** LCMS: m/z 355 [M+H]; 2-benzyl-7-(pyridin-3-yl)-1,2,3,4,5,6-hexahydroazepino[4,3-b]indole **1.1.3.3 (13),** LCMS: m/z 354 [M+H] and others. 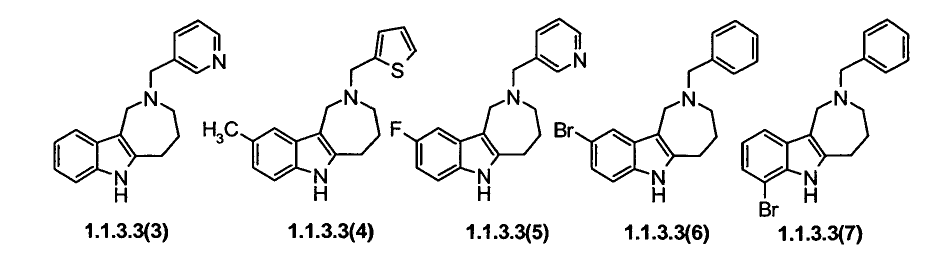 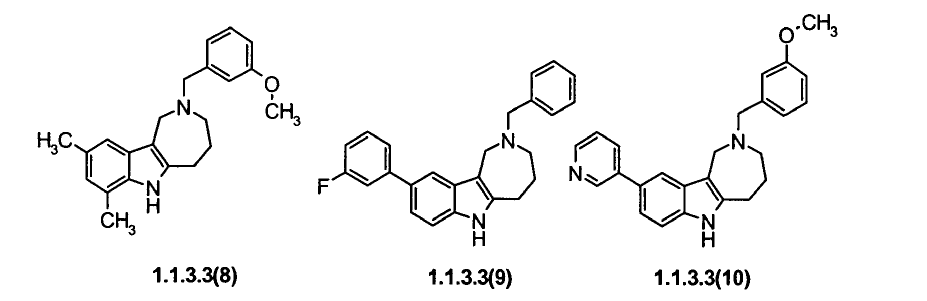 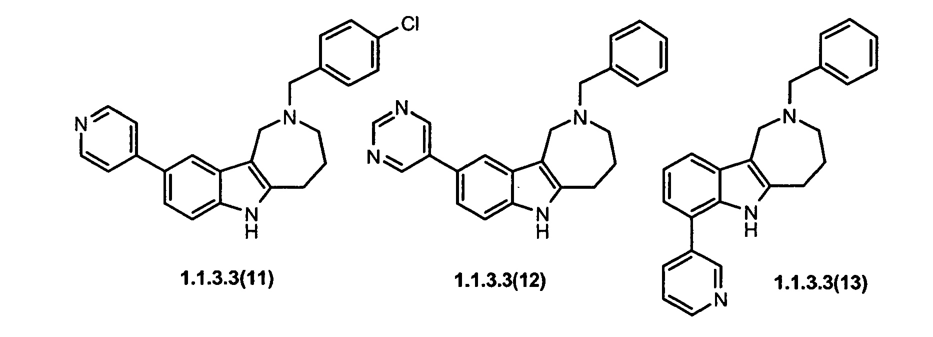
**D**. 0.2 Mmol of triethylamine and 0.2 mmol of the proper acylating reagent of general formula **8**, for example, carbonic acid chloride are added successively to a solution of 0.16 mmol of azepino[4,3-b]indole **1.1.1** in 3 ml of tetrahydrofuran at stirring. Stirring is continued for 2-6 hr at room temperature. Upon completion of the reaction (LCMS monitoring) precipitated triethylamine hydrochloride is filtered off (solid on filter is washed additionally with 10 ml of tetrahydrofuran). Tetrahydrofuran solution of compounds **1.1.3.4** is evaporated *in vacuo*, the residue is purified by column chromatography on silica gel impregnated with triethylamine eluting with hexane-chloroform-triethylamine (5:4:1) mixture. It gives compounds **1.1.3.4,** yield 65-75%, among them: 2-benzoyl-9-methyl-1,2,3,4,5,6-hexahydroazepino[4,3-b]indole **1.1.3.4 (1),** LCMS: m/z 305 [M+H]; 2-acetyl-9-fluoro-1,2,3,4,5,6-hexahydroazepino[4,3-b]indole **1.1.3.4 (2),** LCMS: m/z 247 [M+H]; 2-butyloxycarbonyl-9-methyl-1,2,3,4,5,6-hexahydroazepino[4,3-b]indole **1.1.3.4 (3),** LCMS: m/z 301 [M+H]; 2-ethyloxycarbonyl-9-methyl-1,2,3,4,5,6-hexahydroazepino[4,3-b]indole **1.1.3.4 (4),** LCMS: m/z 273 [M+H]; 2-butyloxycarbonyl-9-fluoro-1,2,3,4,5,6-hexahydroazepino[4,3-b]indole **1.1.3.4 (5),** LCMS: m/z 305 [M+H]; 2-butyloxycarbonyl-9-(pyridin-3-yl)-1,2,3,4,5,6-hexahydroazepino[4,3-b]indole **1.1.3. (6),** LCMS: m/z 305 [M+H] and others. 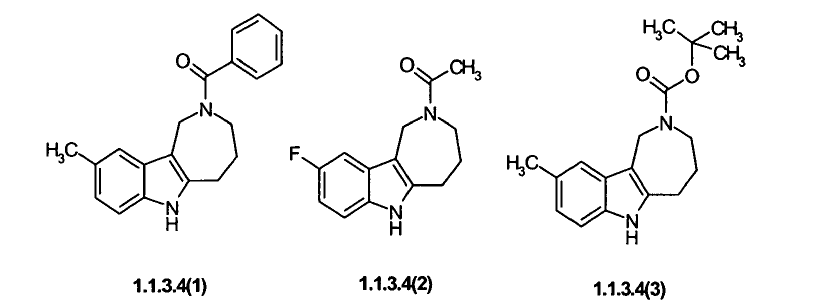 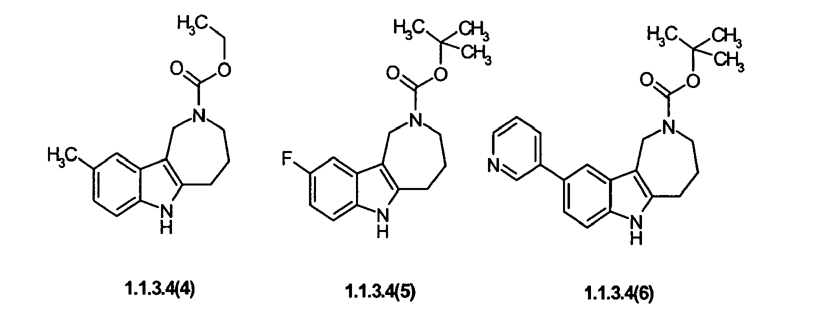
**E.** 0.2 Mmol of isocyanate or isothiocyanate of general formula **9** is added to a solution of 0.16 mmol of azepino[4,3-b]indole 1.1.1 in 3 ml of THF at stirring. Mixture is stirred for 4 hr at room temperature. Upon completion of the reaction (LCMS monitoring) the resultant solution is evaporated in vacuum, the residue is purified by column chromatography on silica gel impregnated with triethylamine, eluent - hexane-chloroform-triethylamine (5:4:1) mixture. It gives compounds **1.1.3.5,** yield 55-80%, among them: 2-phenylcarbamoyl-9-methyl-1,2,3,4,5,6-hexahydroazepino[4,3-b]indole **1.1.3.5** (1), LCMS: m/z 320 [M+H], ¹H NMR (400 MHz, DMSO-d₆): 10.72 (s, 1H), 8.32 (s, 1H), 7.43-7.39 (m, 3H), 7.19-7.11 (m, 3H), 6.90-6.79 (m, 2H), 4.71 (s, 2H), 3.77 (m, 2H), 2.93 (m, 2H), 2.37 (s, 3H), 1.89 (m, 2H); 9-methyl-2-(4-fluorophenylthio-carbamoyl)-1,2,3,4,5,6-hexahydroazepino[4,3-b]indole **1.1.3.5 (2),** LCMS: m/z 354 [M+H] and others. 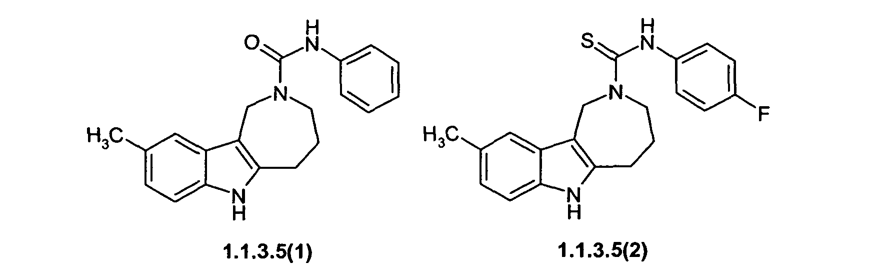
**F.** 0.2 Mmol of triethylamine and 0.2 mmol of the corresponding sulfonyl chloride of general formula **10** are added successively to a solution of 0.16 mmol of azepino[4,3-b]indole **1.1.1** in 3 ml of tetrahydrofuran. Stirring is continued for 2-6 hr at 50°C. Upon completion of the reaction (LCMS monitoring) precipitated triethylamine hydrochloride is filtered off (solid on filter is washed additionally with 10 ml of tetrahydrofuran). Tetrahydrofuran solution of compounds **1.1.3.6** is evaporated *in vacuo,* the residue is purified by column chromatography on silica gel impregnated with triethylamine eluting with hexane-chloroform-triethylamine (5:4:1) mixture. It gives compounds **1.1.3.6,** yield 62-85%, among them: 2-phenylsulfonyl-9-methyl-1,2,3,4,5,6-hexahydroazepino[4,3-b]indole **1.1.3.6 (1),** LCMS: m/z 341 [M+H], ¹H NMR (400 MHz, DMSO-d₆): 10.74 (br.s, 1H), 7.73 (m, 2H), 7.61 (m, 1H), 7.49 (m, 2H), 7.24 (s, 1H), 7.14 (m, 1H), 6.84 (m, 1H), 4.46 (s, 2H), 3.50 (m, 2H), 2.78 (m, 2H), 2.39 (s, 3H), 1.83 (m, 2H); 2-phenylsulfonyl-9-fluoro-1,2,3,4,5,6-hexahydroazepino[4,3-b]indole **1.1.3.6 (2),** LCMS: m/z 345 [M+H] and others. 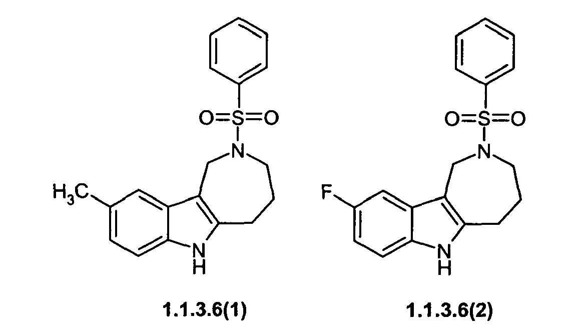
**G**. 5 Mmol of 1,2,3,4,5,6-hexahydroazepino[4,3-b]indole **1.1.3** dissolved in 25 ml of THF is added to 10 ml of 1 M solution of BH₃ in THF at 0-5°C at stirring. The reaction mixture is refluxed for 1 hr under argon atmosphere. Upon completion of the reaction (LCMS monitoring) the solvent is evaporated *in vacuo*, 50 ml of 10% HCl water solution is added to the residue and the resultant mixture is refluxed for 30 min. Upon completion of hydrolysis (LCMS control) the reaction mixture is evaporated to a half of volume and alkalized with 10% KOH water solution. Product is extracted with dichloroethane, extract is dried over K₂CO₃, solvent is evaporated *in vacuo,* the residue is purified by column chromatography on silica gel impregnated with Et₃N. It gives *trans*-1,2,3,4,5,5a,6,10b-octahydroazepino[4,3-b]indoles 1.2.3, yield 60-80%, among them: *trans*-2,9-dimethyl-1,2,3,4,5,5a,6,10b-octahydroazepino[4,3-b]indole 1.2.3.2(1), LCMS: m/z 217 [M+H], ¹H NMR (400 MHz, DMSO-d6): 6.77 (s, 1H), 6.75 (d, 1H, J=7.69 Hz), 6.40 (d, 1H, J=7.69 Hz), 5.42 (br.s, 1H), 3.70 (m, 1H), 3.24 (m, 1H), 3.05 (m, 1H), 2.68 (m, 1H), 2.52 (m, 2H), 2.38 (s, 3H), 2.19 (s, 3H), 2.05 (m, 1H), 1.80-1.55 (m, 3H); *trans*-2-benzyl-9-methyl-1,2,3,4,5,5a,6,10b-octahydroazepino[4,3-b]indole 1.2.3.2(2), LCMS: m/z 293 [M+H]; *trans*-2-benzyl-9-(3-fluorophenyl)-1,2,3,4,5,5a,6,10b-octahydroazepino[4,3-b]indole **1.2.3.2(3),** LCMS: m/z 373 [M+H]; *trans*-2-benzyl-9-(pyrimidin-5-yl)-1,2,3,4,5,5a,6,10b-octahydroazepino[4,3-b]indole **1.2.3.2(4),** LCMS: m/z 357 [M+H]; *trans*-2-benzoyl-9-methyl-1,2,3,4,5,5a,6,10b-octahydroazepino[4,3-b]indole **1.2.3.5(1),** LCMS: m/z 307 [M+H]; *trans*-2-benzenesulfonyl-9-methyl-1,2,3,4,5,5a,6,10b-octahydroazepino[4,3-b]indole **1.2.3.7(1),** LCMS: m/z 343 [M+H] and others.

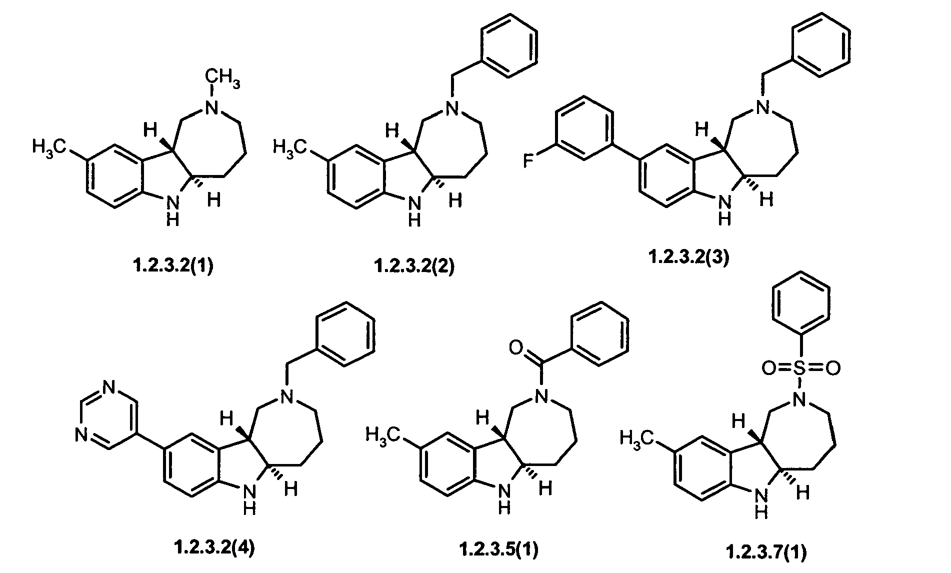

**Example 5.** General methods for preparation of 1,2,3,4,5,6-hexahydroazepino[4,3-b]indoles of general formula **1.1, 1.2, 1.3.**
**A.** A mixture of 2 mmol of azepino[4,3-b]indole **1.1.3,** 2,5 mmol of alkyl halide of general formula **5,** for example, iodide or bromide, and 750 mg of Cs₂CO₃ in 10 ml of dimethylformamide (DMF) or N-methylpyrrolidone (NMP) is stirred vigorously for 6 hr at 50°C. Upon completion of the reaction (LCMS control) the products are isolated in the usual manner. It gives compounds **1.1.4,** yield 50-80%, among them: ethyl (2-(*tert-*butyloxycarbonyl)-9-methyl-1,2,3,4,5,6-hexahydro-1H-azepino[4,3-b]indol-6-yl)acetate **1.1.4(1),** LCMS: m/z 387 [M+H]; ethyl (2-benzoyl-9-methyl-1,2,3,4,5,6-hexahydro-1H-azepino[4,3-b]indol-6-yl)acetate **1.1.4(2),** LCMS: m/z 391 [M+H]; ethyl (9-methyl-2-phenylcarbamoyl-1,2,3,4,5,6-hexahydro-1H-azepino[4,3-b]indol-6-yl)acetate **1.1.4(3),** LCMS: m/z 405 [M+H]; ethyl (9-methyl-2-phenylsulfonyl-1,2,3,4,5,6-hexahydro-1H-azepino[4,3-b]indol-6-yl)acetate **1.1.4(4),** LCMS: m/z 427 [M+H]; 2-benzoyl-9-methyl-6-(4-trifluoromethylbenzyl)-1,2,3,4,5,6-hexahydroazepino[4,3-b]indole **1.1.4(5),** LCMS: m/z 463 [M+H]; [9-methyl-2-benzoyl-1,2,3,4,5,6-hexahydro-1H-azepino[4,3-b]indol-6-yl)acetonitrile **1.1.4 (6),** LCMS: m/z 344 [M+H]; (9-methyl-2-phenylsulfonyl-1,2,3,4,5,6-hexahydro-1H-azepino[4,3-b]indol-6-yl)acetamide **1.1.4(7),** LCMS: m/z 398 [M+H]; ethyl *cis*-(2-tert-butoxycarbonyl-9-methyl-1,2,3,4,5,5a,6,10b-octahydro-1H-azepino[4,3-b]indol-6-yl)acetate **1.3.4(1),** LCMS: m/z 389 [M+H]; *cis*-6-benzyl-2-(tert-butoxycarbonyl)-9-methyl-1,2,3,4,5,5a,6,10b-octahydroazepino[4,3-b]indole **1.3.4(2),** LCMS: m/z 393 [M+H] and others. 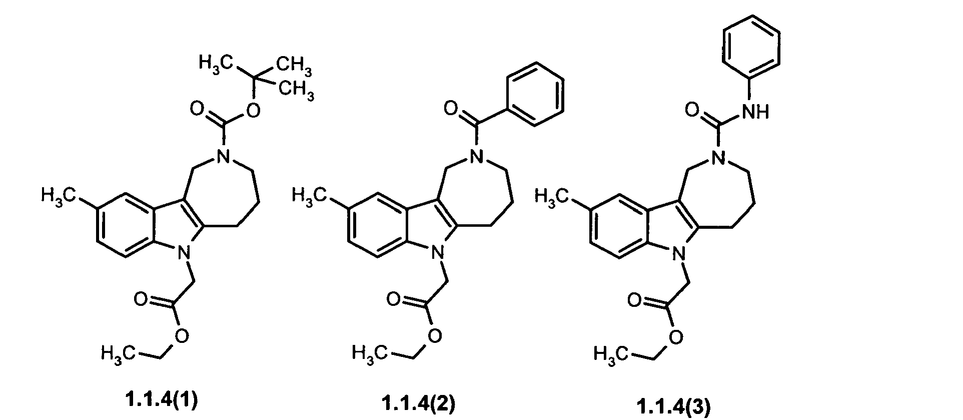 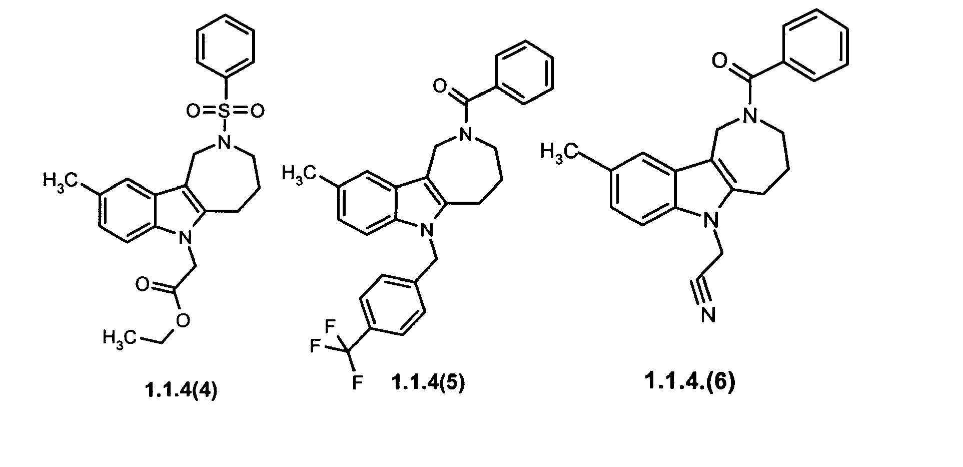 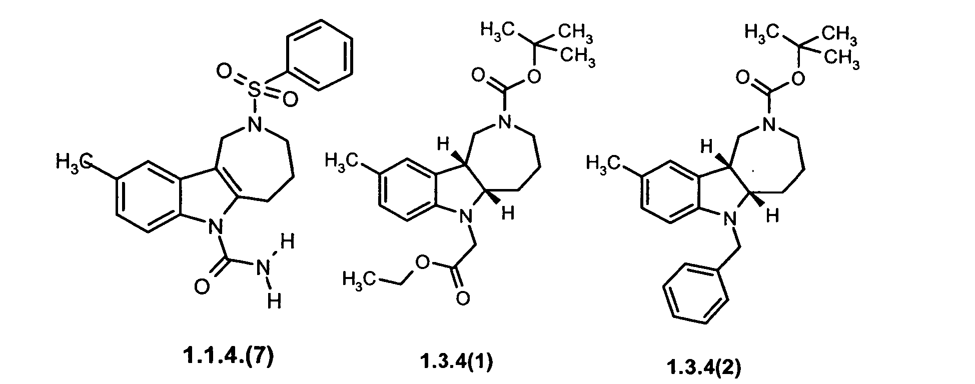
**B**. These compounds may be prepared from the proper azepino[4,3-b]indoles 1.1.3 and aryl- or heteroaryl halides according to the method described for preparation of 7-fluoro-4-(4-fluorophenyl)-1,2,3,4-tetrahydropyrrolo[4,3-b]indole [Welch, W.M., Harbert, C.A., Weissman, A. J. Med. Chem. 1980, 23, 704-707], among them: 2,9-dimethyl-6-(4-trifluoromethylphenyl)-1,2,3,4,5,6-hexahydroazepino[4,3-b]indole **1.1.4(8),** LCMS: m/z 359 [M+H]; 6-(4-carboxy-3-nitrophenyl)-2-methyl-9-fluoro-1,2,3,4,5,6-hexahydroazepino[4,3-b]indole **1.1.4(9),** LCMS: m/z 384 [M+H] and others. 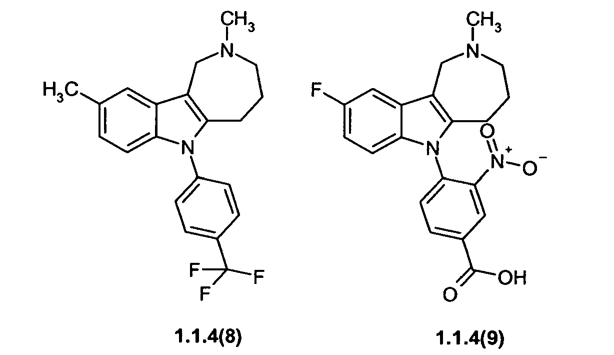
**C**. 5 Mmol of 1,2,3,4,5,6-hexahydroazepino[4,3-b]indole **1.1.4** dissolved in 25 ml of THF at stirring is added to 10 ml of 1 M solution of BH₃ in THF at 0-5°C. The reaction mixture is refluxed for 1 hr under argon atmosphere. Upon completion of the reaction (LCMS monitoring) the solvent is evaporated *in vacuo,* 50 ml of 10% HCl water solution is added to the residue and the resultant mixture is refluxed for 30 min. Upon completion of hydrolysis (LCMS control) the reaction mixture is evaporated to a half of volume and alkalized with 10% KOH water solution. Product is extracted with dichloromethane, extract is dried over K₂CO₃, solvent is evaporated in vacuo, residue is purified by column chromatography on silica gel impregnated with Et₃N. It gives *trans*-1,2,3,4,5,5a,6,10b-octahydroazepino[4,3-b]indoles **1.2.4,** yield 60-80%, among them: *trans*-2,9-dimethyl-6-benzyl-1,2,3,4,5,5a,6,10b-octahydroazepino[4,3-b]indole **1.2.4(1),** LCMS: m/z 307 [M+H] and others. 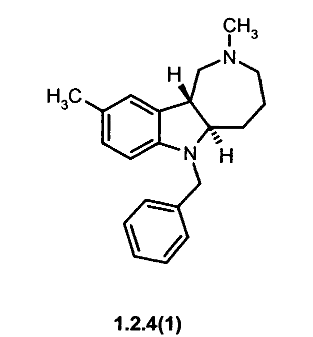
**D**. A mixture of 1 mmol of azepino[4,3-b]indole **1.1.3,** 1200 mkl of dimethylsulfoxide, 300 mkl of water, 3 mmol of the proper freshly distilled vinyl derivate **6** and **3** mmol of MeONa; or 1,2,3,4,5,5a,6,10b-octahydroazepino[4,3-b]indole **1.3.3** under argon atmosphere is stirred at 80-100°C for 2-48 hr. Upon completion of the reaction (LCMS monitoring) the reaction mixture is dissolved in 50 ml of dichloromethane, the solution is washed twice with water solution of K₂CO₃, dried over Na₂SO₄, evaporated, the residue is purified by column chromatogrphy on silica gel impregnated with triethylamine. It gives compounds **1.1,** among them: 2,9-dimethyl-6-[2-(pyridin-2-yl)ethyl]-1,2,3,4,5,6-hexahydroazepino[4,3-b]indole **1.1.5(1),** LCMS: m/z 320 [M+H], ¹H NMR (400 MHz, DMSO-d₆): 8.58 (br.s, 1H), 7.67 (m, 1H), 7.27 (m, 3H), 7.09 (m, 1H), 6.90 (m, 1H), 4.50 (m, 2H), 3.87 (br.s, 2H), 3.08 (m, 2H), 2.96 (m, 2H), 2.76 (m, 2H), 2.41 (s, 3H), 2.37 (s, 3H), 1.70 (m, 2H); 2,9- 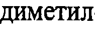 -6-[2-(6-methylpyridin-3-yl)ethyl]-1,2,3,4,5,6-hexahydroazepino[4,3-b]indole **1.1.5(2),** LCMS: m/z 334 [M+H], ¹H NMR (400 MHz, DMSO-d₆): 8.16 (br.s, 1H), 7.39 (m, 1H), 7.27 (m, 2H), 7.14 (m, 1H), 6.90 (m, 1H), 4.34 (m, 2H), 3.73 (br.s, 2H), 2.91 (m, 2H), 2.86 (m, 2H), 2.69 (m, 2H), 2.44 (s, 3H), 2.41 (s, 3H), 2.29 (s, 3H), 1.62 (m, 2H); 2,9-dimethyl-6-[2-(pyridin-4-yl)ethyl]-1,2,3,4,5,6-hexahydroazepino[4,3-b]indole **1.1.5(3),** LCMS: m/z 320 [M+H], ¹H NMR (400 MHz, DMSO-d6): 8.44 (m, 2H), 7.26 (m, 2H), 7.13 (m, 2H), 6.89 (m, 1H), 4.40 (m, 2H), 3.75 (br.s, 2H), 2.95 (m, 2H), 2.87 (m, 2H), 2.68 (m, 2H), 2.41 (s, 3H), 2.30 (s, 3H), 1.59 (m, 2H); ethyl [2,9-dimethyl-1,2,3,4,5,6-hexahydroazepino[4,3-b]indol-6-yl]propionate **1.1.5(4),** LCMS: m/z 315 [M+H], ¹H NMR (400 MHz, DMSO-d₆): 7.24 (d, 1H, J=7.69 Hz), 7.19 (s, 1H), 6.87 (d, 1H, J=7.69 Hz), 4.36 (m, 2H), 4.10-4.00 (m, 2H), 3.70 (s, 2H), 2.89-2.85 (m, 4H), 2.64 (m, 2H), 2.37 (s, 3H), 2.29 (s, 3H), 1.79 (m, 2H), 1.12 (m, 3H); 2,9-dimethyl-6-[2-(pyridin-3-yl)-ethyl]-1,2,3,4,5,6-hexahydroazepino[4,3-b]indole **1.1.5(5),** LCMS: m/z 320 [M+H]; 2-methyl-6-[2-(pyridin-3-yl)ethyl]-9-fluoro-1,2,3,4,5,6-hexahydroazepino[4,3-b]indole **1.1.5(6),** LCMS: m/z 324 [M+H]; 9-bromo-2-methyl-6-[2-(pyridin-3-yl)ethyl]-1,2,3,4,5,6-hexahydroazepino[4,3-b]indole **1.1.5(7),** LCMS: m/z 385 [M+H]; 2-methyl-9-(pyridin-4-yl)-6-[2-(pyridin-3-yl)ethyl]-1,2,3,4,5,6-hexahydroazepino[4,3-b]indole **1.1.5(8),** LCMS: m/z 383 [M+H]; [2,9-dimethyl-1,2,3,4,5,6-hexahydroazepino[4,3-b]indol-6(1H)-yl]propionitrile **1.1.5(9),** LCMS: m/z 268 [M+H] and others. 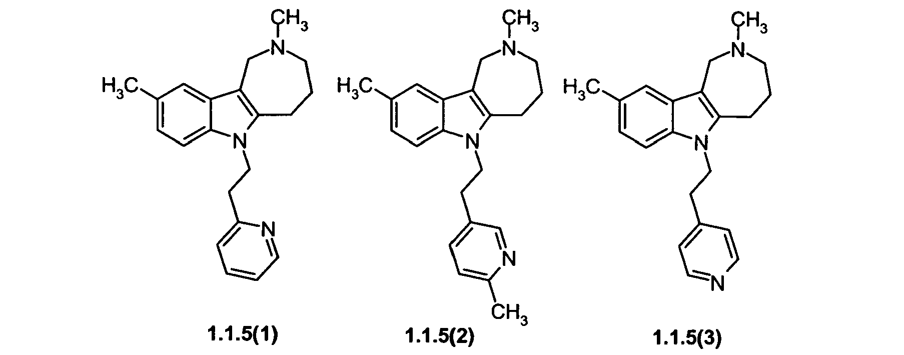 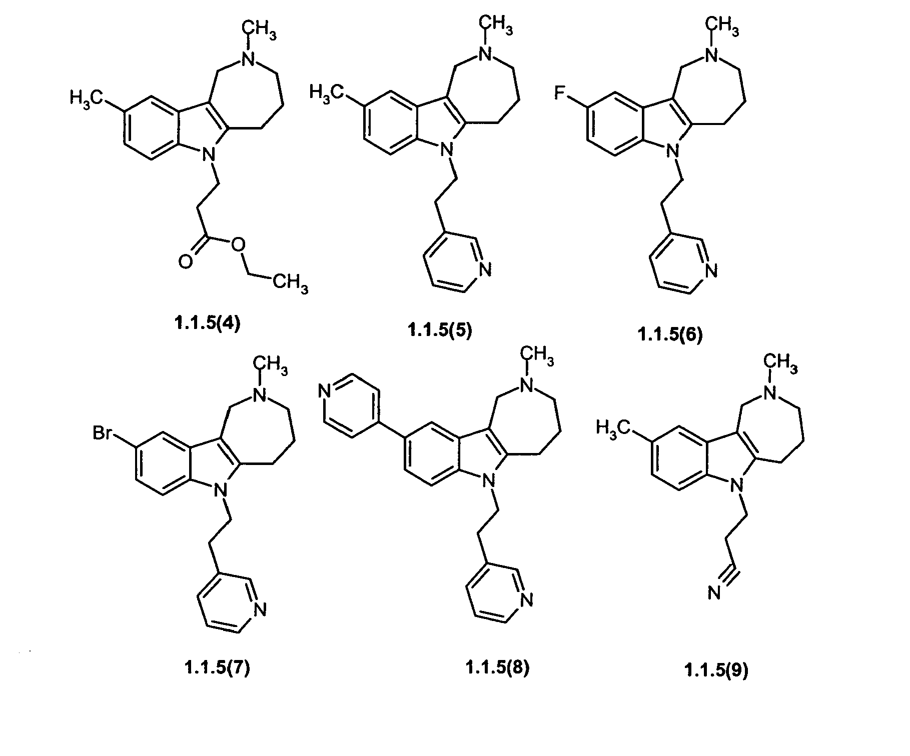 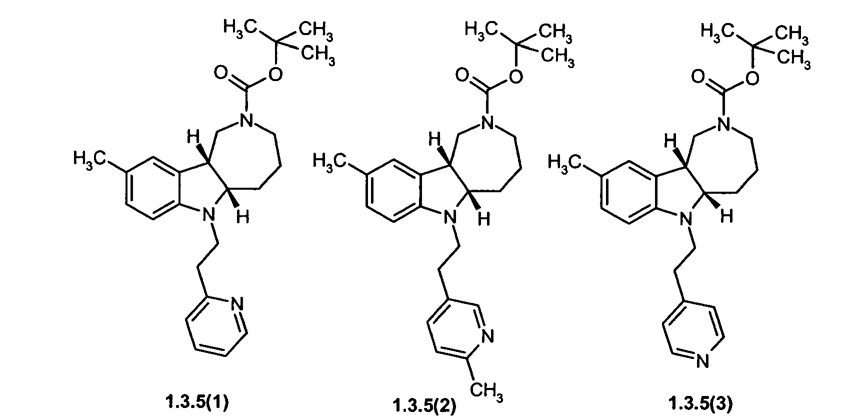 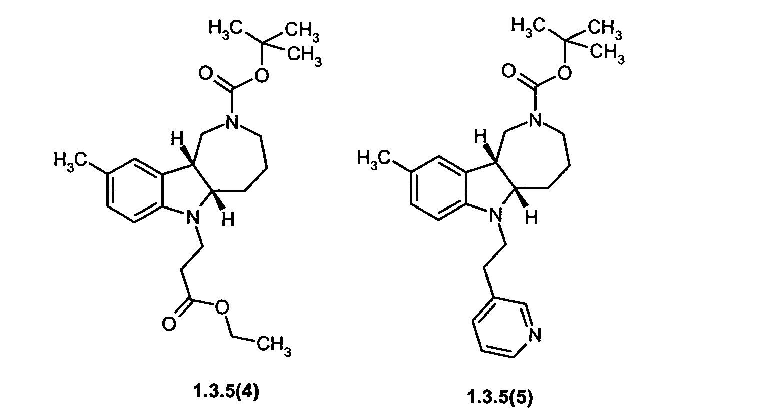
**E.** 5 Mmol of 1,2,3,4,5,6-hexahydroazepino[4,3-b]indole **1.1.5** dissolved in 25 ml of THF at stirring is added to 10 ml of 1 M solution of BH₃ in THF at 0-5°C. The reaction mixture is refluxed for 1 hr under argon atmosphere. Upon completion of the reaction (LCMS monitoring) the solvent is evaporated *in vacuo,* 50 ml of 10% HCl water solution is added to the residue and the resultant mixture is refluxed for 30 min. Upon completion of hydrolysis (LCMS control) the reaction mixture is evaporated to a half of volume and alkalized with 10% KOH water solution. Product is extracted with dichloromethane, extract is dried over K₂CO₃, solvent is evaporated *in vacuo,* the residue is purified by column chromatography on silica gel impregnated with Et₃N. It gives *trans*-1,2,3,4,5,5a,6,10b-octahydroazepino[4,3-b]indoles 1.2.5, yield 60-80%, among them: *trans*-2,9-dimethyl-6-[2-(pyridin-2-yl)ethyl]-1,2,3,4,5,5a,6,10b-octahydroazepino[4,3-b]indole **1.2.5(1),** LCMS: m/z 322 [M+H]; trans-2,9-dimethyl-6-[2-(6-methylpyridin-3-yl)ethyl]-1,2,3,4,5,5a,6,10b-octahydroazepino[4,3-b]indole **1.2.5(2),** LCMS: m/z 336 [M+H], ¹H NMR (400 MHz, DMSO-d6): 8.32 (s, 1H), 7,57 (d, 1H, J=8.06 Hz), 7.15 (d, 1H, J=8.06 Hz), 6.81 (d, 1H, J=7.70, 6.77 (s, 1H), 6.39 (d, 1H, J=7.70 Hz), 3.44 (m, 2H), 3.20 (m, 3H), 3.03 (m, 1H), 2.72 (m, 1H), 2.64 (m, 2H), 2.44 (m, 1H), 2.42 (s, 3H), 2.33 (s, 3H), 2.17 (s, 3H), 2.15 (m, 1H), 1.73 (m, 2H), 1.40 (m, 1H); *trans*-2,9-dimethyl-6-[2-(pyridin-4-yl)ethyl]-1,2,3,4,5,5a,6,10b-octahydroazepino[4,3-b]indole **1.2.5(3),** LCMS: m/z 322 [M+H]; *trans*-2,9-dimethyl-6-[2-(pyridin-3-yl)ethyl]-1,2,3,4,5,5a,6,10b-octahydroazepino[4,3-b]indole **1.2.5(4),** LCMS: m/z 322 [M+H]; *trans*-2-methyl-6-[2-(pyridin-3-yl)ethyl]-9-fluoro-1,2,3,4,5,5a,6,10b-octahydroazepino[4,3-b]indole 1.2.5(5), LCMS: m/z 326 [M+H]; *trans-2-*methyl-6-[2-(pyridin-3-yl)ethyl]-9-(pyridin-4-yl)-1,2,3,4,5,5a,6,10b-octahydroazepino[4,3-b]indole **1.2.5(6),** LCMS: m/z 385 [M+H] and others. 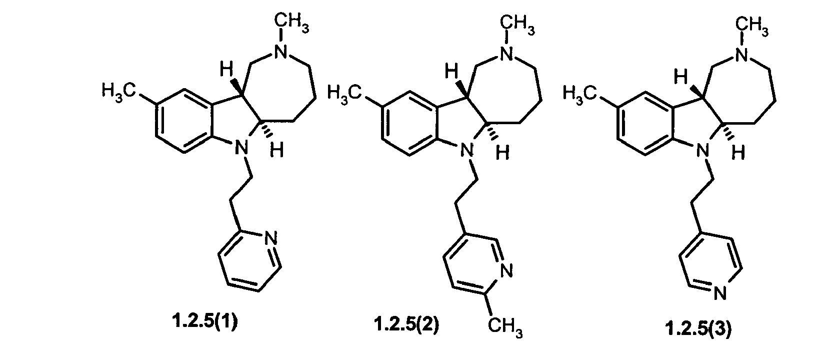 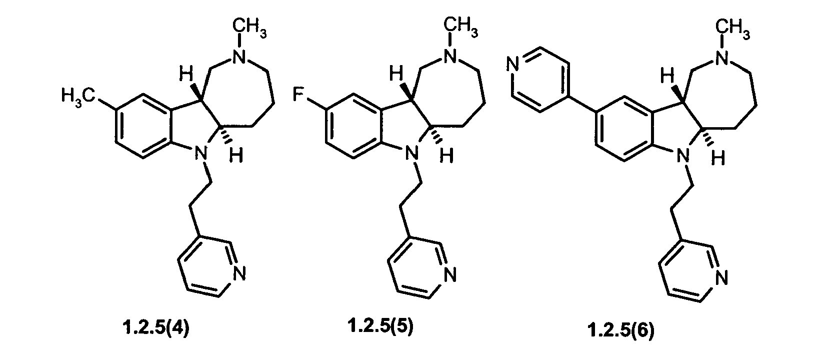
**F**. A mixture of 0.32 mmol of *trans*-1,2,3,4,5,5a,6,10b-octahydroazepino[4,3-b]indole **1.2.3** or *cis*-1,2,3,4,5,5a,6,10b-octahydroazepino[4,3-b]indole **1.3.3,** 0.4 mmol of the appropriate aldehyde of general formula 7, 170 mg (0.8 mmol) of NaBH(AcO)₃ and 3 ml of dichloroethane is stirred at room temperature for 2-6 hr (LCMS control). Upon completion of the reaction 10 ml of water and 1 ml of 10% K₂CO₃water solution are added. Organic layer is separated, dried over K₂CO₃ and evaporated. The product is isolated from the residue by preparative chromatography on silica gel impregnated with triethylamine eluting with hexane-ethyl acetate triethylamine (7:2:1) mixture. It gives hydrogenated azepino[4,3-b]indoles of general formulas **1.2.6** or **1.3.6,** yield 60-70%, among them: *trans*-6-benzyl-2,9-dimethyl-1,2,3,4,5,5a,6,10b-octahydroazepino[4,3-b]indole **1.2.6(1),** LCMS: m/z 307 [M+H]; *trans*-4-(2,9-dimethyl-1,2,3,4,5,5a,10b-octahydroazepino[4,3-b]indol-6-ylmethyl)benzoic acid 1.2.6(2), LCMS: m/z 351 [M+H]; *trans*-2,9-dimethyl-6-phenethyl-1,2,3,4,5,5a,6,10b-octahydroazepino[4,3-b]indole **1.2.6(3),** LCMS: m/z 321 [M+H]; trans-2,9-dimethyl-6-(pyridin-2-ylmethyl)-1,2,3,4,5,5a,6,10b-octahydroazepino[4,3-b]indole **1.2.6(4),** LCMS: m/z 308 [M+H]; *trans*-2,9-dimethyl-6-(pyridin-3-ylmethyl)-1,2,3,4,5,5a,6,10b-octahydroazepino[4,3-b]indole **1.2.6(5),** LCMS: m/z 308 [M+H]; trans-2,9-dimethyl-6-(pyridin-4-ylmethyl)-1,2,3,4,5,5a,6,10b-octahydroazepino[4,3-b]indole **1.2.6(6),** LCMS: m/z 308 [M+H]; *cis*-6-benzyl-2-(tert-butoxycarbonyl)-9-methyl-1,2,3,4,5,5a,6,10b-octahydroazepino[4,3-b]indole **1.3.6(1),** LCMS: m/z 393 [M+H] and others. 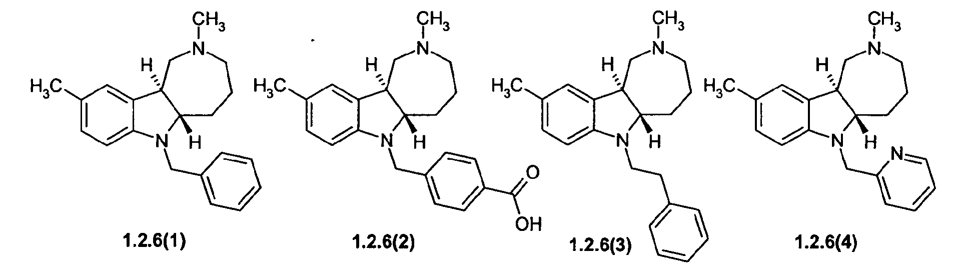 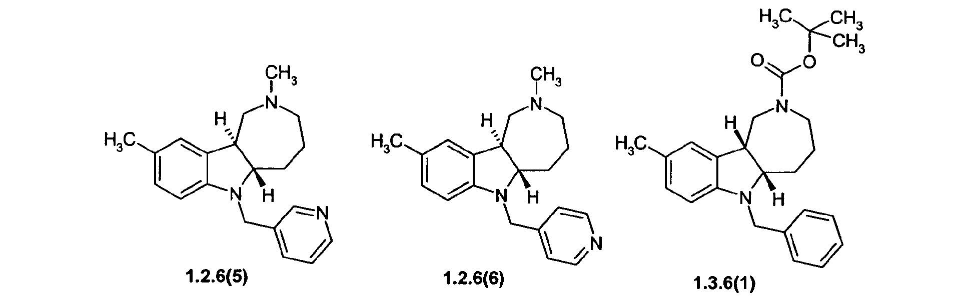
**G.** 0.2 Mmol of triethylamine and 0.2 mmol of the proper acylating agent of general formula **8,** for example, carbonic acid chloride, are added successively to a solution of 0.16 mmol of azepino[4,3-b]indole **1.1.3** in 3 ml of tetrahydrofuran at stirring. Stirring is continued for 2-6 hr at room temperature. Upon completion of the reaction (LCMS monitoring) precipitated triethylamine hydrochloride is filtered off (on filter the solid is washed additionally with 10 ml of tetrahydrofuran). Tetrahydrofuran solution of compounds **1.1.7** is evaporated *in vacuo*, the residue is purified by column chromatography on silica gel impregnated with triethylamine eluting with hexane-chloroform-triethylamine (5:4:1) mixture. It gives compounds **1.1.7,** yield 65-75%, among them: 2,9-dimethyl-6-(4-chlorobenzoyl)-1,2,3,4,5,6-hexahydroazepino[4,3-b]indole **1.1.7(1),** LCMS: m/z 353 [M+H]; 6-benzoyl-2-methyl-9-fluoro-1,2,3,4,5,6-hexahydroazepino[4,3-b]indole **1.1.7(2),** LCMS: m/z 323 [M+H] and others. 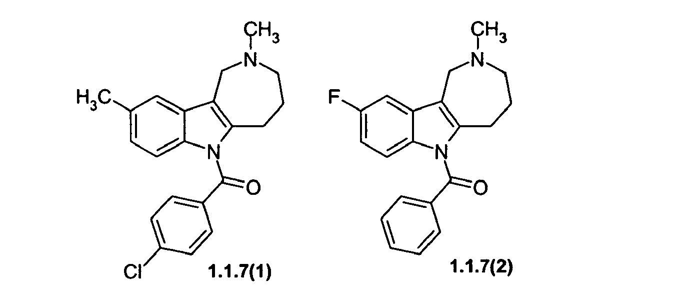
**H.** 5 Mmol of 1,2,3,4,5,6-hexahydroazepino[4,3-b]indole 1.1.7 dissolved in 25 ml of THF at stirring is added to 10 ml of 1 M solution of BH₃ in THF at 0-5°C. The reaction mixture is refluxed for 1 hr under argon atmosphere. Upon completion of the reaction (LCMS monitoring) the solvent is evaporated *in vacuo*, 50 ml of 10% HCl water solution is added to the residue and the resultant mixture is refluxed for 30 min. Upon completion of hydrolysis (LCMS control) the reaction mixture is evaporated to a half of volume and alkalized with 10% KOH water solution. Product is extracted with dichloromethane, extract is dried over K₂CO₃, solvent is evaporated *in vacuo*, and the residue is purified by column chromatography on silica gel impregnated with Et₃N. It gives *trans*-1,2,3,4,5,5a,6,10b-octahydroazepino[4,3-b]indoles **1.2.7,** yield 60-80%, among them: *trans*-2,9-dimethyl-6-(4-chlorobenzoyl)-1,2,3,4,5,5a,6,10b-octahydroazepino[4,3-b]indole 1.2.7(1), LCMS: m/z 355 [M+H]; *trans*-6-benzoyl-2-methyl-9-fluoro-1,2,3,4,5,5a,6,10b-octahydroazepino[4,3-b]indole **1.2.7(2),** LCMS: m/z 325 [M+H]; *trans*-6-benzoyl-2,9-dimethyl-1,2,3,4,5,5a,6,10b-octahydroazepino[4,3-b]indole **1.2.7(3),** LCMS: m/z 321 [M+H]; *trans*-6-acetyl-2,9-dimethyl-1,2,3,4,5,5a,6,10b-octahydroazepino[4,3-b]indole **1.2.7(4),** LCMS: m/z 259 [M+H]; *cis*-6-benzoyl-2-(tert-butoxycarbonyl)-9-methyl-1,2,3,4,5,5a,6,10b-octahydroazepino[4,3-b]indole **1.3.7(1),** LCMS: m/z 407 [M+H] and others. 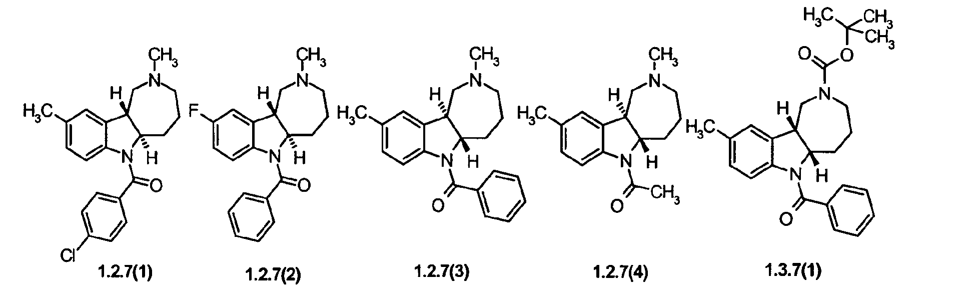
**I.** 0.2 Mmol of isocyanate or isothiocyanate of general formula 9 is added to a solution of 0.16 mmol of azepino[4,3-b]indole **1.1.3** in 3 ml THF. Mixture is stirred for 4 hr at room temperature. Upon completion of the reaction (LCMS monitoring) the resultant solution is evaporated *in vacuo,* the residue is purified by column chromatography on silica gel impregnated with triethylamine eluting with hexane-chloroform-triethylamine (5:4:1) mixture. It gives compounds **1.1.8,** yield 55-80%, among them: 2,9-dimethyl-6-(3-fluorophenylcarbamoyl)-1,2,3,4,5,6-hexahydroazepino[4,3-b]indole **1.1.8(1),** LCMS: m/z 352 [M+H]; 6-(2,4-dichlorophenylthiocarbamoyl)-2-methyl-9-fluoro-1,2,3,4,5,6-hexahydroazepino[4,3-b]indole **1.1.8(2),** LCMS: m/z 422 [M+H] and others. 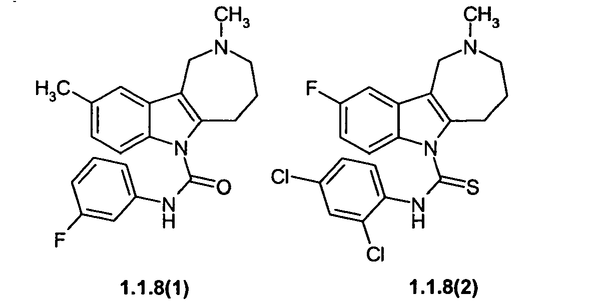
**J.** 0.2 Mmol of triethylamine and 0.2 mmol of the proper sulfonyl chloride of general formula **10** are added successively to a solution of 0.16 mmol of azepino[4,3-b]indole **1.1.3** in 3 ml of tetrahydrofuran at stirring. Stirring is continued for 2-6 hr at 50°C. Upon completion of the reaction (LCMS monitoring) precipitated triethylamine hydrochloride is filtered off (solid on filter is washed additionally with 10 ml of tetrahydrofuran). Tetrahydrofuran solution of compounds **1.1.9** is evaporated *in vacuo,* the residue is purified by column chromatography on silica gel impregnated with triethylamine eluting with hexane-chloroform-triethylamine (5:4:1) mixture. It gives compounds **1.1.9,** yield 72-85%, among them: 2,9-dimethyl-6-methylsulfonyl-1,2,3,4,5,6-hexahydroazepino[4,3-b]indole **1.1.9(1),** LCMS: m/z 293 [M+H]; 2-methyl-6-phenylsulfonyl-9-fluoro-1,2,3,4,5,6-hexahydroazepino[4,3-b]indole **1.1.9(2),** LCMS: m/z 359 [M+H] and others. 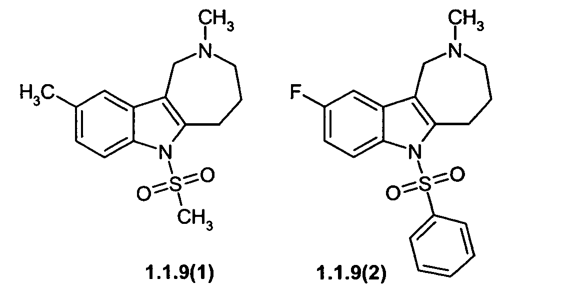
**K.** A mixture of 1 mmol of azepino[4,3-b]indole 1.1.2, 2 ml of dimethylsulfoxide, 1,2 mmol of the proper freshly distilled vinyl derivate 6 and 15 mg (0,1 mmol) of MTBD under argon is stirred at 20°C for 2-4 hr. Upon completion of the reaction (LCMS monitoring) the reaction mixture is dissolved in 50 ml of dichloromethane, the solution is washed twice with diluted K₂CO₃ water solution, dried over Na₂SO₄, evaporated, the residue is purified by column chromatogrphy on silica gel impregnated with triethylamine. It gives compounds **1.1.11,** among them: ethyl 3-(6-benzyl-9-methyl-1,2,3,4,5,6-hexahydroazepino[4,3-b]indol-2-yl)propionate **1.1.11(1),** LCMS: m/z 391 [M+H], ¹H NMR (400 MHz, DMSO-d₆): 7.35-7.18 (m, 5H), 6.95-6.92 (m, 2H), 6.83 (d, 1H, J=7.69 Hz), 5.38 (s, 2H), 4.04 (m, 2H), 3.88 (s, 2H), 3.00 (m, 2H), 2.82 (m, 2H), 2.67 (m, 2H), 2.45 (m, 2H), 2.36 (s, 3H), 1.64 (m, 2H), 1.15 (m, 3H); 6-benzyl-9-methyl-2-[2-(pyridin-3-yl)ethyl]-1,2,3,4,5,6-hexahydroazepino[4,3-b]indole **1.1.11(2),** LCMS: m/z 396 [M+H] and others. 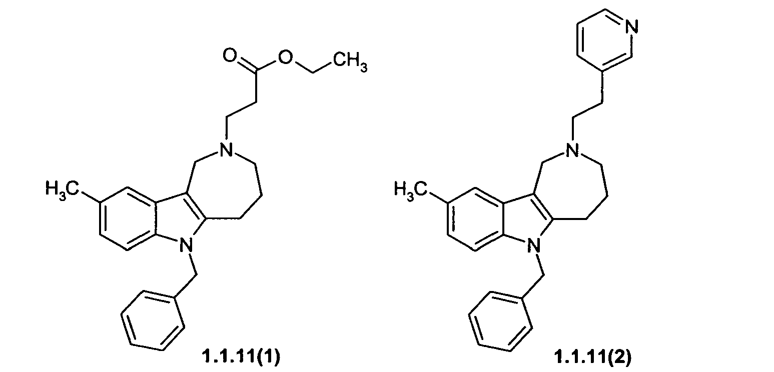
**L**. 3.6 Ml of formaldehyde water solution is added to a solution of 0,04 mol of azepino[4,3-b]indole **1.1.2, 1.2.2** or **1.3.2** in 600 ml of ethanol or dichloroethane, and the resultant mixtire is stirred for 30 min at room temperature. The flask is filled with argon, 600 mg of PtO₂ is inserted, the flask is blown through with hydrogen, and stirring is continued in atmosphere of hydrogen at room temperature for 48 hr (LCMS monitoring), or 0,05 mol of NaBH(AcO)₃ is added and the reaction mixture is stirred at room temperature for 2 hr till the reaction is completed (LCMS monitoring). After that the reaction mixture is filtered off, filtrate is evaporated *in vacuo*, the solid residue is washed with water and dried *in vacuo*. In need, the product is purified chromatographically on silica gel impregnated with triethylamine eluting with 10% solution of triethylamine in dichloromethane. It gives the desired 2-methyl substituted compounds **1.1,** yield 61-75%, among them: ethyl (2,9-dimethyl-1,2,3,4,5,6-hexahydroazepino[4,3-b]indol-6-yl)acetate **1.1.12(1),** LCMS: m/z 301 [M+H], ¹H NMR (400 MHz, DMSO-d₆): 7.21-7.18 (m, 2H), 6.86 (d, 1H, J=6.8 Hz), 5.02 (s, 2H), 4.15-4.09 (m, 2H), 3.76 (s, 2H), 2.89 (m, 2H), 2.76 (m, 2H), 2.36 (s, 3H), 2.31 (s, 3H), 1.78 (br. s, 2H), 1.19 (m, 3H); ethyl *trans*-(2,9-dimethyl-1,2,3,4,5,5a,6,10b-octahydroazepino[4,3-b]indol-6-yl)acetate **1.2.12(1),** LCMS: m/z 301 [M+H]; ethyl *cis*-(9-methyl-1,2,3,4,5,5a,6,10b-octahydroazepino[4,3-b]indol-6-yl)acetate **1.3.12(1),** LCMS: m/z 301 [M+H]; ethyl *cis*-3-(9-methyl-1,2,3,4,5,5a,6,10b-octahydroazepino[4,3-b]indol-6-yl)propionate **1.3.12(2),** LCMS: m/z 317 [M+H]; *cis*-6-benzyl-9-methyl-1,2,3,4,5,5a,6,10b-octahydroazepino[4,3-b]indole **1.3.12(3),** LCMS: m/z 307 [M+H]; *cis*-9-methyl-6-[2-(pyridin-2-yl)ethyl]-1,2,3,4,5,5a,6,10b-octahydroazepino[4,3-b]indole **1.3.12(4),** LCMS: m/z 322 [M+H]; *cis*-9-methyl-6-[2-(pyridin-3-yl)ethyl]-1,2,3,4,5,5a,6,10b-octahydroazepino[4,3-b]indole **1.3.12(5),** LCMS: m/z 322 [M+H]; *cis*-9-methyl-6-[2-(4-methylpyridin-3-yl)ethyl]-1,2,3,4,5,5a,6,10b-octahydroazepino[4,3-b]indole **1.3.12(6),** LCMS: m/z 336 [M+H]; *cis*-9-methyl-6-[2-(pyridin-4-yl)ethyl]-1,2,3,4,5,5a,6,10b-octahydroazepino[4,3-b]indole **1.3.12(7),** LCMS: m/z 322 [M+H] and others. 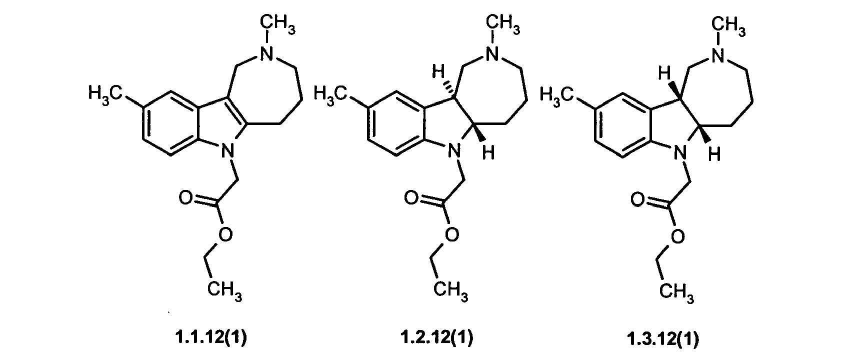 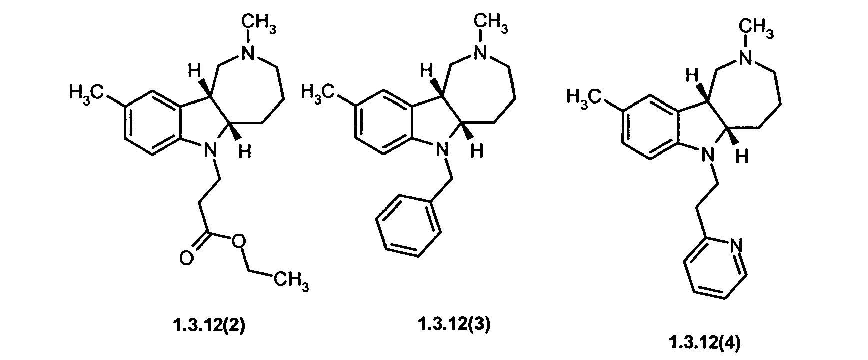 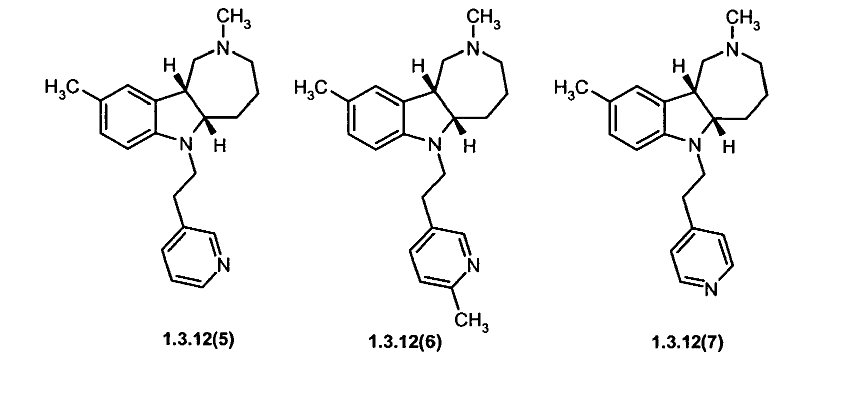
**M.** 0.4 Mmol of the proper aldehyde of general formula **7** and 170 mg (0.8 mmol) of NaBH(AcO)₃ are added to a solution of 0.32 mmol of azepino[4,3-b]indole 1.1.2 in 3 ml of dichloroethane. The mixture is stirred at room temperature for 2-6 hr (LCMS monitoring). Upon completion of the reaction 10 ml of water and 1 ml of 10% K₂CO₃ water solution are added to the mixture. Organic phase is separated, dried over K₂CO₃ and evaporated. The product is isolated from the residue by preparative chromatography on silica gel impregnated with triethylamine eluting with hexane-ethylacetate-triethylamine (7:2:1) mixture. It gives compounds **1.1.12,** yield 60-70%, among them: 6-benzyl-9-methyl-2-(pyridin-3-ylmethyl)-1,2,3,4,5,6-hexahydroazepino[4,3-b]indole **1.1.12(2),** LCMS: m/z 382 [M+H]; 2,6-dibenzyl-9-methyl-1,2,3,4,5,6-hexahydroazepino[4,3-b]indole **1.1.12(3),** LCMS: m/z 381 [M+H]; 2,6-dibenzyl-9-fluoro-1,2,3,4,5,6-hexahydroazepino[4,3-b]indole **1.1.12(4),** LCMS: m/z 385 [M+H]; 2-(4-fluorobenzyl)-6-(pyridin-3-ylmethyl)-1,2,3,4,5,6-hexahydroazepino[4,3-b]indole **1.1.12(5),** LCMS: m/z 386 [M+H]; ethyl [9-methyl-2-(pyridin-3-ylmethyl)-1,2,3,4,5,6-hexahydroazepino[4,3-b]indol-6-yl]carboxylate **1.1.12(6),** LCMS: m/z 378 [M+H], ¹H NMR (400 MHz, DMSO-d₆): 8.48 (m, 1H), 8.42 (s, 1H), 7.67-7.65 (m, 1H), 7.37-7.30 (m, 1H), 7.21 (d, 1H, J=6.4 Hz), 6.95 (s, 1H), 6.85 (d, 1H, J=6.4 Hz), 5.05 (s, 2H), 4.15-4.09 (m, 2H), 3.83 (s, 2H), 3.63 (s, 2H), 3.02 (m, 2H), 2.82 (m, 2H), 2.32 (s, 3H), 1.77 (br. s, 2H), 1.20 (m, 3H) and others. 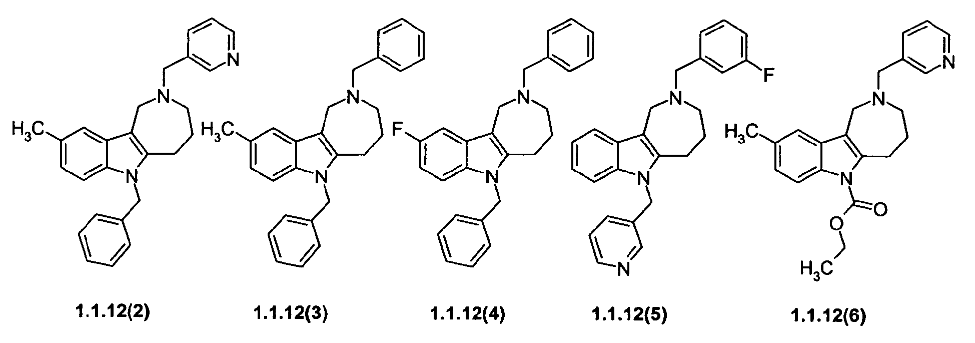
**N.** 0.2 Mmol of triethylamine and 0.2 mmol of the proper acylating agent of general formula **8**, for example, carbonic acid chloride, are added successively to a solution of 0.16 mmol of azepino[4,3-b]indole **1.1.2** in 3 ml of tetrahydrofuran at stirring. Stirring is continued for 2-6 hr at room temperature. Upon completion of the reaction (LCMS monitoring) precipitated triethylamine hydrochloride is filtered off (on filter solid is washed additionally with 10 ml of tetrahydrofuran). Tetrahydrofuran solution of compounds **1.1.13** is evaporated *in vacuo*, the residue is purified by column chromatography on silica gel impregnated with triethylamine eluting with hexane-chloroform-triethylamine (5:4:1) mixture. It gives compounds **1.1.13,** yield 65-75%, 6-(3-methoxybenzyl)-2-(3-chlorobenzoyl)-1,2,3,4,5,6-hexahydroazepino[4,3-b]indole **1.1.13(1),** LCMS: m/z 445 [M+H]; 2-acetyl-6-benzyl-9-methyl-1,2,3,4,5,6-hexahydroazepino[4,3-b]indole **1.1.13(2),** LCMS: m/z 333 [M+H] and others. 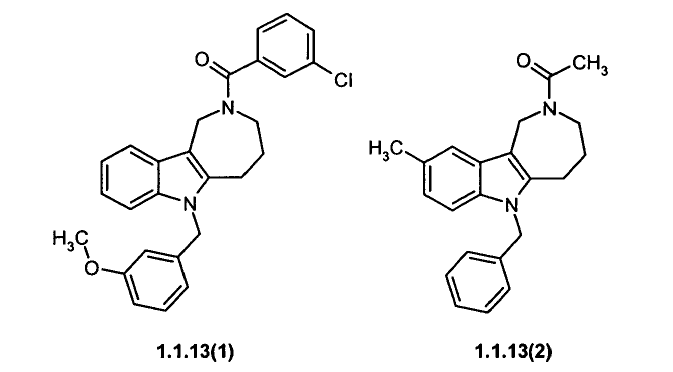
**O.** 0.2 Mmol of isocyanate or isothiocyanate of general formula **9** is added to a solution of 0.16 mmol of azepino[4,3-b]indole **1.1.2** in 3 ml THF. Mixture is stirred for 4 hr at room temperature. Upon completion of the reaction (LCMS monitoring) the resultant solution is evaporated *in vacuo,* the residue is purified by column chromatography on silica gel impregnated with triethylamine eluting with hexane-chloroform-triethylamine (5:4:1) mixture. It gives compounds **1.1.14,** yield 55-80%, among them: 6-benzyl-9-methyl-2-phenylcarbamoyl-1,2,3,4,5,6-hexahydroazepino[4,3-b]indole **1.1.14(1),** LCMS: m/z 410 [M+H]; 6-benzyl-9-methyl-2-(4-fluorophenylthiocarbamoyl)-1,2,3,4,5,6-hexahydroazepino[4,3-b]indole **1.1.14(2),** LCMS: m/z 444 [M+H] and others. 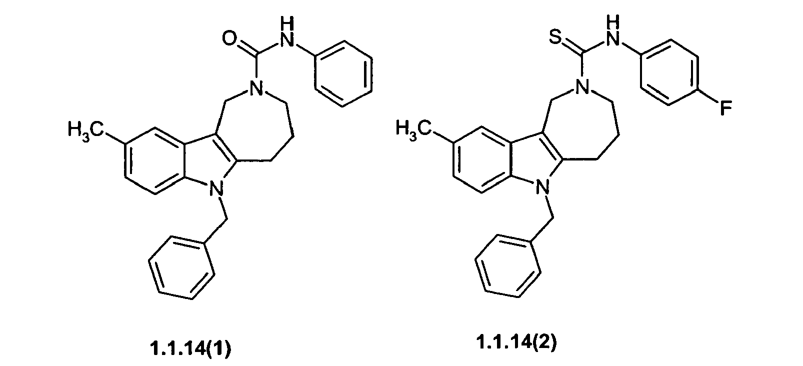
**P.** 0.2 Mmol of triethylamine and 0.2 mmol of the proper sulfonyl chloride of general formula **10** are added successively to a solution of 0.16 mmol of azepino[4,3-b]indole **1.1.2** in 3 ml of tetrahydrofuran at stirring. Stirring is continued for 2-6 hr at 50°C. Upon completion of the reaction (LCMS monitoring) precipitated triethylamine hydrochloride is filtered off (the solid on filter is washed additionally with 10 ml of tetrahydrofuran). Tetrahydrofuran solution of compounds **1.1.15** is evaporated *in vacuo,* the residue is purified by column chromatography on silica gel impregnated with triethylamine eluting with hexane-chloroform-triethylamine (5:4:1) mixture. It gives compounds **1.1.15,** yield 72-85%, among them: 6-benzyl-9-methyl-2-phenylsulfonyl-1,2,3,4,5,6-hexahydroazepino[4,3-b]indole **1.1.15(1),** LCMS: m/z 431 [M+H]; - 6-benzyl-2-phenylsulfonyl-9-fluoro-1,2,3,4,5,6-hexahydroazepino[4,3-b]indole **1.1.15(2),** LCMS: m/z 435 [M+H] and others.

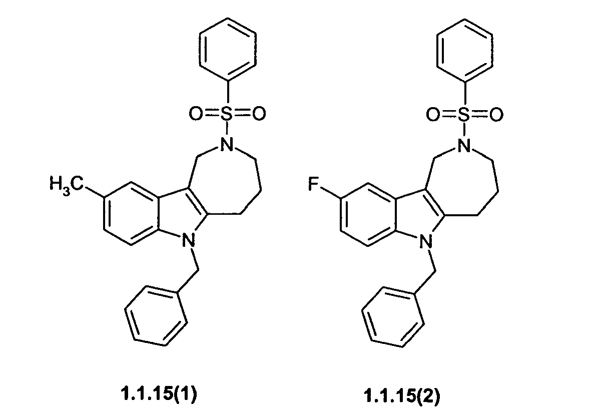

**Example 6.** General methods for preparation of compounds of general formula 1 in the form of salts. To a solution of 1 equivalent of compound 1 in dioxane a solution of ∼4-8 equivalents of the desired acid in dry dioxane or tetrahydrofuran is added. The precipitated white solid is separated, washed with dioxane or tetrahydrofuran and dried *in vacuo*. It gives compounds **1** in the form of salts, among them: 2,9-dimethyl-6-[2-(pyridin-2-yl)ethyl]-1,2,3,4,5,6-hexahydroazepino[4,3-b]indole dihydrochloride **1.1.5(1)HCl,** comprising, according to NMR data, 1 molecule of dioxane that can not be removed at keeping it *in vacuo*, LCMS: m/z 320 [M+H], ¹H NMR (400 MHz, DMSO-d₆): 11.04 (br.s, 1H), 8.79 (m, 1H), 8.40 (m, 1H), 7.88 (m, 2H), 7.50 (s, 1H) 7.34 (m, 1H), 6.89 (m, 1H), 4.68 (m, 3H), 4.45 (m, 1H), 3.63 (m, 1H), 3.46 (m, 3H), 3.11 (m, 2H), 2.79 (s, 3H), 2.38 (s, 3H), 2.11 (m, 2H); 2,9-dimethyl-6-[2-(6-methylpyridin-3-yl)ethyl]-1,2,3,4,5,6-hexahydroazepino[4,3-b]indole dihydrochloride **1.1.5(2)HCl,** LCMS: m/z 334 [M+H], ¹H NMR (400 MHz, DMSO-d₆): 11.08 (br.s, 1H), 8.64 (s, 1H), 8.22 (m, 1H), 7.78 (m, 1H), 7.43 (s, 1H), 7.35 (m, 1H), 6.94 (m, 1H), 4.64 (m, 1H), 4.48 (m, 3H), 3.63 (m, 2H), 3.41 (m, 1H), 3.15 (m, 1H), 3.00 (m, 2H), 2.78 (m, 3H), 2.74 (s, 3H), 2.41 (s, 3H), 2.03 (m, 2H); 2,9-dimethyl-6-[2-(pyridin-4-yl)ethyl]-1,2,3,4,5,6-hexahydroazepino[4,3-b]indole dihydrochloride **1.1.5(3)HCl,** LCMS: m/z 320 [M+H], ¹H NMR (400 MHz, DMSO-d6): 11.16 (br.s, 1H), 8.80 (m, 2H), 7.88 (m, 2H), 7.88 (m, 2H), 7.41 (s, 1H), 7.29 (m, 1H), 6.90 (m, 1H), 4.62 (m, 3H), 4.43 (m, 1H), 3.62 (m, 1H), 3.42 (m, 1H), 3.30 (m, 2H), 2.98 (m, 2H), 2.78 (s, 3H), 2.40 (s, 3H), 2.03 (m, 2H) and others.

Example 7. Tests of biological activity of compounds of general formula 1. In order to determine a physiological activity of the proposed compounds a focused library was compiled to include compounds of general formula 1, some of which are shown in Table 2 below. The compounds were tested as potential histamine H1 receptor antagonists, and their ability to regulate cytosolic calcium ions concentration in cells by blocking calcium channels regulated by intracellular calcium repositories was determined. Cells SK-N-SH (ATCC, USA) were grown on DMEM medium (Invitrogen, USA), containing 10% fetal calf serum (FBS) and antibiotic penicillin-streptomycin in CO₂ incubator (5% CO₂) untill cell density was 1*10⁵ cells/cm². The cells were removed from flask surface by reagent TrypLE Express (Invitrogen, USA), collected by centrifugation and resuspended in medium Hybridoma Serum Free Medium (HSFM, Sigma, USA) in concentration of 4*10⁶ cells/ml. For measurement of cytosolic calcium concentration the cells were loaded with calcium sensitive fluorescent dye Fura 2 AM (Invitrogen, USA) by means of hatching of cells with a dye in suspension for 30 minutes at room temperature.The cells were collected by centrifugation, resuspended in HSFM, incubated in suspention for 15 minutes, collected by centrifugation, washed twice in HSFM and resuspended in HSFM in concentration of 4*10⁶ cells/ml.

Registration of calcium streams in cells was carried out using spectrofluorometer Shimadzu-RF5301PC. The cells were diluted with buffer (NaCl 0.145 M, KCl 0.0054 M, NaH₂PO₄ 0.001 M, MgSO₄ 0.0008 M, CaCl₂ 0.0018 M, HEPES 0.03 M, D-glucose 0.0112 M pH 7.4) till concentration 1*10⁵ cells/ml in measuring cuvette provided with magnetic stirrer, and fluorescence was registered using a regime of two-wave excitement (340 and 380 nm, respectively) at wavelength of emission 510 nm (F1 and F2, respectively). In 20 seconds after beginning of registration 10 mM of histamine water solution was added (final concentration is 10 µM). In another 30 seconds, after achievement of calcium cytosolic concentration peak a solution of tested compound in DMSO was added and registration was continued for 3 minutes. Serial dilutions of the compounds in dimethylsulfoxide (DMSO) were prepared for determination of their biological activity, and the effect of the concentration of compound on the calcium streams induced by histamine was determined.

Transformation of fluorescence signal into calcium concentration was carried out by means of equation imbedded in program Super Ion Probe (Shimadzu), for which maximum of free calcium concentration by addition of digitonin (Sigma, USA) up to concentration of 0.1 mg/ml and zero calcium concentration by addition of ethylenediamine-tetraacetate (EDTA) up to concentration of 10 mM were determined. Kinetic curves of cytosolic calcium decrease after addition of tested compound compared to concentration after addition of histamine were calculated using single-phase exponential model by means of Prism 4 (GraphPad Software, Inc.) program: $\left[Ca\right] = {\left[Ca\right]}_{max} * exp \left(-K*T\right) + {\left[Ca\right]}_{min ,}$
wherein T means time after addition of tested compound, [Ca]ₘₐₓ and [Ca]ₘᵢₙ correspond to maximum (peak meaning after addition of histamine) and minimum (equilibrium level to which the curve converges after addition of tested compound) concentrations of cytosolic calcium, and K means a kinetic constant of the decrease of cytosolic calcium concentration which is calculated by minimization of the least squares of deviations.

The dependence of calculated constants of calcium decreasing (*K*) on the concentration of tested compound (C) was drawn up, and using this dependence by means of program Prism 4 the value of EC₅₀ (concentration of tested compound corresponding to half-maximum increase of a kinetic constant of cytozoic calcium concentration decrease) was defined using four parametrical equation.$K = {K}_{Bkg} + \frac{{K}_{max} ⁢ {C}^{N}}{{{EC}_{50}}^{N} + {C}^{N}} ,$
wherein *K_{Bkg}* and *Kₘₐₓ* are constants of the decrease of cytosolic calcium concentration in the presence of infinite great concentration of tested compound and without it, respectively, and N is Hill coefficient. In Table 2 below the values corresponding to the reciprocal logarithm of EC₅₀ (-LogEC₅₀=pEC₅₀) for some of tested compounds of general formula **1** used as measures of these compounds activity are presented.

**Table 2. Activity of tested compounds of general formula 1**

| **Nº comp.** | **Formula** | **pIC₅₀ Phase 1** | **pIC₅₀ Phase2** |
|---|---|---|---|
| **1.1.5(1)** | 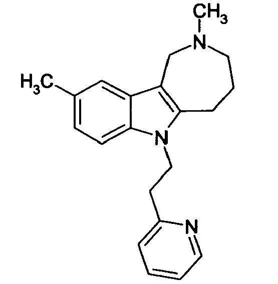 | 5,5 | 5,5 |
| **1.1.5(2)** | 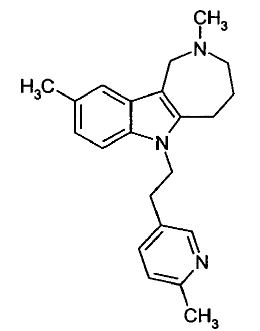 | 6,5 | 5,8 |
| **1.1.5(3)** | 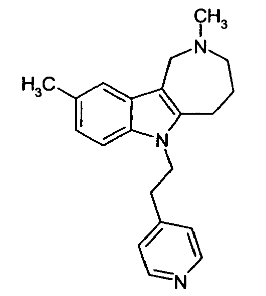 | 6,7 | 5,9 |
| **1.1.5(5)** | 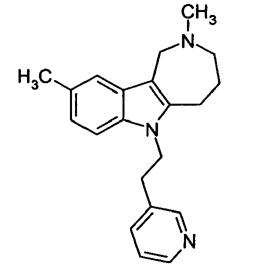 | 6,3 | 5,5 |
| **1.1.3.3(1)** | 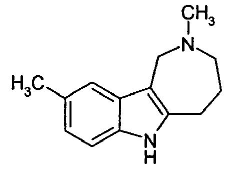 | <4 | <4 |
| **1.1.3.4(1)** | 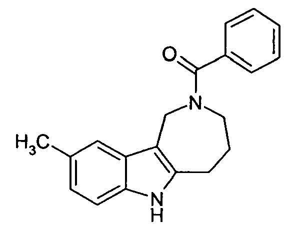 | <4 | <4 |
| **1.1.3.5(1)** | 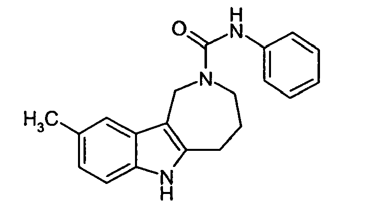 | <4 | <4 |
| **1.1.3.6(1)** | 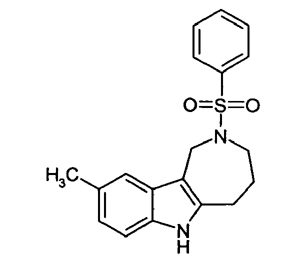 | <4 | <4 |
| **1.2.5(1)** | 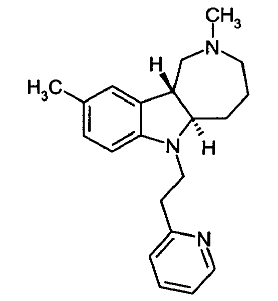 | 6 | 5,6 |
| **1.2.5(2)** | 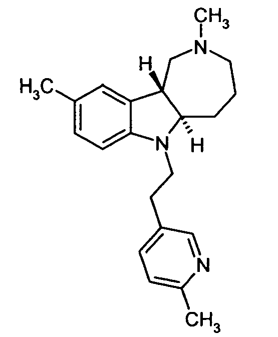 | 6,6 | 6 |
| **1.2.5(3)** | 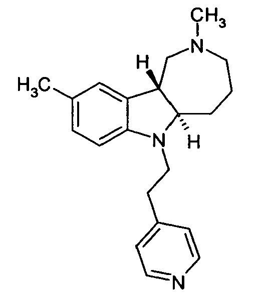 | 6,8 | 6 |
| **1.2.5(4)** | 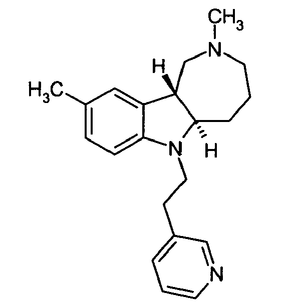 | 6,8 | 5,6 |
| **1.2.5(5)** | 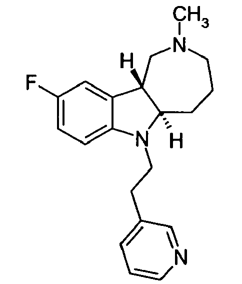 | 6.9 | 6.2 |
| **1.2.5(6)** | 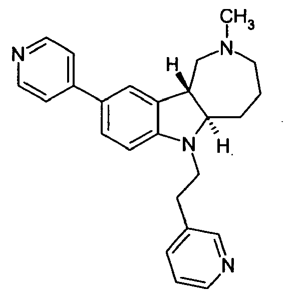 | 6.6 | 6.0 |
| **1.2.6(1)** | 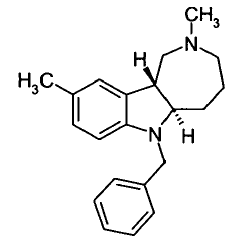 | 6,9 | 6,2 |
| **1.2.6(2)** | 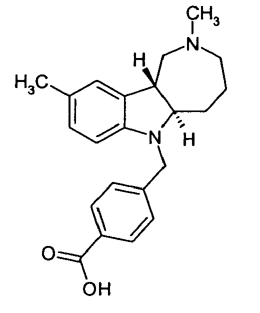 | <4 | <4 |
| **1.2.6(3)** | 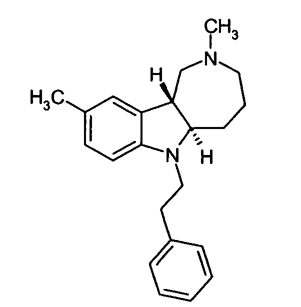 | 6,8 | 5,7 |
| **1.2.6(4)** | 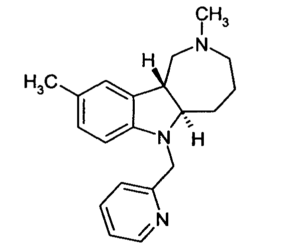 | 6,2 | 5,8 |
| **1.2.6(5)** | 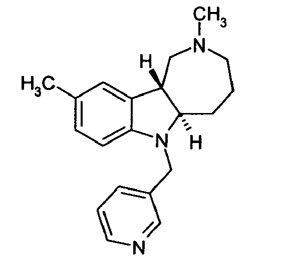 | 7 | 5,8 |
| **1.2.6(6)** | 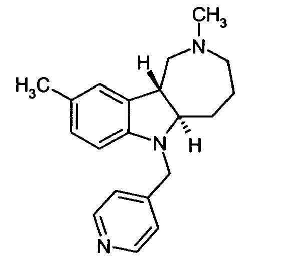 | 6,7 | 5,3 |
| **1.2.7(3)** | 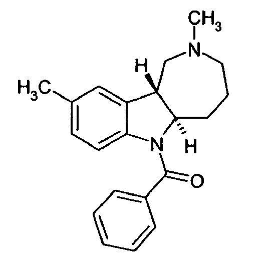 | <4 | <4 |
| **1.2.7(4)** | 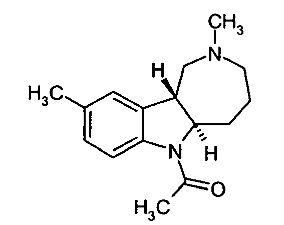 | <4 | <4 |
| **1.3.12(3)** | 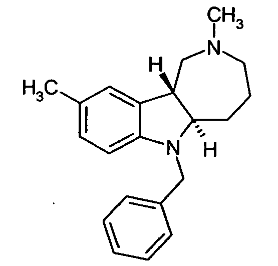 | 6,5 | 5,4 |
| **1.3.12(5)** | 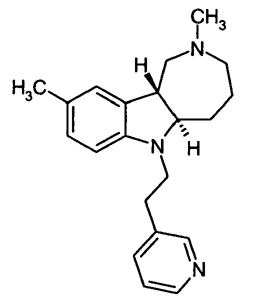 | 6,7 | 5,6 |

As can be seen from data given in Table 2 compounds of general formula 1 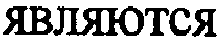 are effective histamine receptor blocators (**Phase 1 -** compounds prevent calcium ions entry in cells due to antagonistic action on H1-receptors), and also accelerate intraplasmatic calcium removal (**Phase 2**), that testifies their neuroprotective, cognitive-stimulating and antihistaminic activity.

**Example 9.** Testing of compounds of general formula 1 on spatial memory in test of novel object recognition. Test of novel object recognition is based on the fact that rats and mice investigate spontaneously a novel object or novel object localization (NL) for longer period of time than the known object or known object localization (KL). The test was tried on rats [A. Ennaceur and J. Delacour, A new one-trial test for neurobiological studies of memory in rats. 1: behavioral data. Behav. Brain Res. 1988, 31, 47-59; J.C. Dodart, C. Mathis and A. Ungerer, Scopolamine-induced deficits in a two-trial object recognition task in mice. NeuroReport 1997, 8, 1173-1178; C. Messier, Object recognition in mice: improvement of memory by glucose. Neurobiol. Learn. Mem. 1997, 67, 172-175; C. Pittenger, Y.Y. Huang, R.F. Paletzki, R. Bourtchouladze, H. Scanlin, S. Vronskaya et al., Reversible inhibition of CREB/ATF transcription factors in region CA1 of the dorsal hippocampus disrupts hippocampus-dependent spatial memory. Neuron 2002, 34, 447-462; A.E. Ryabinin, M.N. Miller and S. Durrant, Effects of acute alcohol administration on object recognition learning in C57BL/6J mice. Pharmacol. Biochem. Behav. 2002, 71, 307-312; F. Sargolini, P. Roullet, A. Oliverio and A. Mele, Effects of intra-accumbens focal administrations of glutamate antagonists on object recognition memory in mice. Behav. Brain Res. 2003, 138, 153-163].

Test of novel object recognition consists of two tests. The first one is novel object localization test use for examination of spatial memory, and the second one - test of novel object recognition for exploring non spatial memory [D. Gaffan, Amnesia for complex naturalistic scenes and for objects following fornix transaction in the Rhesus monkey. Eur. J. Neurosci. 1992, 4, 381-388; B. Kolb, K. Buhrmann, R. McDonald and R. Sutherland, Dissociation of the medial prefrontal, posterior parietal, and posterior temporal cortex for spatial navigation and recognition memory in the rat. Cereb. Cortex 1994, 6, 664-680; T. Steckler, W.H.I.M. Drinkenburgh, A. Sahgal and J.P. Aggleton, Recognition memory in rats. I. Concepts and classification. Prog. Neurobiol. 1998, 54, 289-311. T. Steckler, W.H.I.M. Drinkenburgh, A. Sahgal and J.P. Aggleton, Recognition memory in rats II. Neuroanatomical substrates. Prog. Neurobiol. 1998, 54, 313-332].

### Methods of investigation.

Subjects were male mice of 3-5 month age of C57BL/6 line, weighing 20 - 24 g. Animals were housed 5 per cage at light mode 12x12 hr with a light part from 8 till 20 o'clock with water and food available. Experimental setup was made of non-transparent white organic glass measuring 48x38x30 cm. As objects of recognition brown glass bottles measuring 2,7 cm diameter and 5,5 cm high were used. The cage and objects of recognition were wiped with 85% alcohol 2 - 3 min prior placing an animal in the cage. Animals were always placed in the cage centre. 40 Mg of compound of general formula **1** were dissolved in 0,2 ml of DMSO with the following addition of distilled water up to desired concentration. Solutions were prepared just before a trial. All experiments were carried out on two groups of 10 mice each. Animals were injected in dose 1 and 5 mg/kg in volume 0,05 ml on 10 g of animal weight one hour before the trial. The control animals were injected with an equal volume of distilled water.

### Procedure.

*Acquaintance with the behavioural cage*. At the first test day mice were placed into a research chamber and allowed to acclimate for 20 - 30 min. After that each animal was placed for 10 minutes into an empty, previously treated with alcohol behavioural chamber for acclimation. Then the animal was placed into the cage and taken back to vivarium.

*Training*. Next day the same mice were placed into a behavioural chamber and allowed to explore for 20 - 30 min; after acclimation animals were injected intragastrically by the solution of tested compound. In an hour after injection an animal was placed into a behavioural chamber, in the bottom of which two identical objects (glass bottles) for recognition had been placed on diagonal 14,5 cm from the corners. Training time for each animal was 15 min. In 15 min the animal was placed back into the cage and taken back to vivarium.

*Testing*. Testing was carried out in 48 hr after training. For this after acclimation the animal was placed for 1 min into the behavioural chamber for repeated acquaitance. In a minute the animal was taken away, and one object in the familiar localization and the other one in the novel localization were placed in the bottom of the chamber. Using two electronic seconds counters the time of exploration of each object separately during 10 min was registered with accuracy 0,1 sec. Animals behaviour was observed through a mirror. Direct sniffing the object at a distance of 2 cm away or touching an object was counted as a positive reaction of exploration. The left object was in familiar localisation, and right one - in novel.

*Statistical treatment of results*. Because of considerable variation of time for object exploration between animals, we estimated % of time spent exploring for each mouse from the formula tNL/(tKL + tNL) x 100. Total time spent exploring both objects was taken as 100%. Further treatment of results was carried out according to Student's method using t-test.

*Research of acute toxicity*. Acute toxicity of the proposed compounds was determined in male mice of C57BL/6 line. Six animals were injected unitary in the dose of 100 mg/kg. Observation time was 14 days.

Results for some tested azepino[4,3-b]indoles of general formula 1 are represented below:
- animals treated with 2,9-dimethyl-6-[2-(6-methylpyridin-3-yl)ethyl]-1,2,3,4,5,6-hexahydroazepino[4,3-b]indole dihydrochloride **1.1.5(2)HCl** in the dose of 1 mg/kg investigated the object in a novel localization for 58,7±9,7% of time, and in familiar localization - 41,3±9,7% (P=0,03); and animals being dosed with 5 mg/kg of compound **1.1.5(2)HCl** investigated the object ina novel localization for 58,8±11,1% of time, and in familiar localization - 41,2±11,1% (P=0,04). The data shows a stimulating effect on memory of compound **1.1.5(2)HCl** in doses of 1 mg/kg and 5 mg/kg only.
- animals treated with 2,9-dimethyl-6-[(2-pyridin-4-yl)ethyl]-1,2,3,4,5,6-hexahydroazepino[4,3-b]indole dihydrochloride **1.1.5(3)HCl** in the dose of 1 mg/kg needed 55,5±6,2% of time to investigate the object in novel localization, and in familiar localization - 44,5±6,2%(P=0,03); and being injected in the dose of 5 mg/kg animals needed 59,9±5,9% of time to investigate the object in a novel localization, and in familiar localization - 40,1±5,9% (P=0,002). The data given show a stimulating effect on memory of compound **1.1.5(3)HCl** in doses of 1 mg/kg and 5 mg/kg only.
- animals treated with *trans*-2,9-dimethyl-6-[(pyridin-2-yl)methyl]-1,2,3,4,5,5a,6,10b-octahydroazepino[4,3-b]indole dihydrochloride **1.2.6(4)HCl** in the dose of 1 mg/kg needed 49,1± 3,3% of time to investigate the object in a novel localization, and in familiar localization
- 41,3±9,7% (P=0,03). At the dose leveled up to 5 mg/kg the animals investigated the object in a novel localization for 60,1±9,0 % of time, and in familiar localization - 39,9±9,0 % (P=0,004). The data given show a stimulating effect on memory of compound **1.2.6(4)HCl,** and the effect is equally expressed in doses of 1 and 5 mg/kg.
- compounds **1.1.5(2)HCl, 1.1.5(3)HCl** 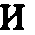 **1.2.6(4)HCl** do not cause animals' death after unitary introduction into stomach in the dose of 100 mg/kg during 14 days of watching. Therefore, these compounds are low-toxic, and it can be anticipated that their LD₅₀ value is more than 100 mg/kg.
- animals treated with *trans*-2,9-dimethyl-6-[(pyridin-2-yl)methyl]-1,2,3,4,5,5a,6,10b-octahydroazepino[4,3-b]indole dihydrochloride **1.2.6(4)HCl** in doses 100 and 50 mg/kg, in 5-10 min after injection showed signs of pronounced sedative action of the dose, that lasted more then 24 hr. This effect was not observed for doses of 1 and 5 mg/kg

Test results on mice have shown that azepino[4,3-b]indoles of general formula **1**:
- have an activating effect on animal's memory in test of familiar object recognition in a noval localization in doses of 1 mg/kg and more;
- have a pronounced sedative action in doses of 100 and 50 mg/kg that lasts for 24 hr and more;
- nontoxic and have LD₅₀ value of tested compounds 100 mg/kg and more.

**Example 10.** An example illustrating preparation of tablets containing 100 mg of an active ingredient. A mixture of 1600 mg of starch, 1600 mg of ground lactose, 400 mg of talc and 100 mg of 2,9-dimethyl-6-[2-(6-methylpyridin-3-yl)ethyl]-1,2,3,4,5,6-hexahydroazepino[4,3-b]indole dihydrochloride **1.1.5(2)HCl** is prepared and compressed into a bar. The resultant bar is comminuted into granules and sifted through a sieve to collect granules of 14-16 mesh. The granules thus obtained are shaped into tablets of suitable form weighing 560 mg weight each. Similarly, according to the invention, pharmaceutical compositions are produced in the form of tablets comprising other substituted hydrogenated azepino[4,3-b]indoles could be prepared in a similar way.

**Example 11**. Capsules comprising 200 mg of 2,9-dimethyl-6-(pyridin-2-ylmethyl)-1,2,3,4,5,5a,6,10b-octahydroazepino[4,3-b]indole dihydrochloride **1.2.6(4)HCl,** according to the invention, may be prepared by careful mixing of compound **1.2.6(4)HCl** with lactose powder in ratio 2 : 1. The resultant powdery mixture is packed into gelatin capsules of suitable size 300 mg to a capsule.

**Example 12**. Injectable compositions for intramuscular, intraperitoneal or subcutaneous injections may be prepared by mixing 500 mg of an active ingredient with proper solubility, for example, 2,9-dimethyl-6-[2-(6-methylpyridin-3-yl)ethyl]-1,2,3,4,5,6-hexahydroazepino[4,3-b]indole dihydrochloride **1.1.5(2)HCl** with 300 mg of chlorobutanol, 2 ml of propylene glycol, and 100 ml of injectable water.The resultant solution is filtered and placed into 1 ml ampoules, and the ampoules are sealed and sterilized in an autoclave.

### Industrial applicability

The invention could be used in medicine, veterinary, biochemistry.

## Claims

1. Hydrogenated azepino[4,3-b]indoles of general formula **1** or racemates, optical isomers, geometrical isomers, pharmaceutically acceptable salts and/or hydrates thereof: 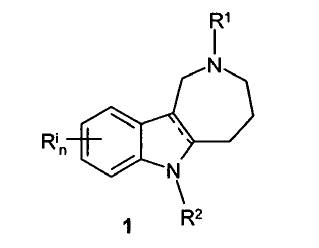 wherein:
solid line together with the dashed line (---) represents a single or a double bond;
R¹ and R² independently of each other are amino group substituents, selected from hydrogen; optionally substituted C₁-C₈ alkyl with substituents selected from optionally substituted aryl or 5-6-membered azaheterocyclyl; C₁-C₈ alkoxycarbonyl; optionally substituted phenyl; optionally substituted carbonylamino or thiocarbonylamino; substituted acyl; C₁-C₈ alkylsulfonyl; optionally substituted arylsulfonyl; upon that, the substituents in the said R₁ and R₂ independently selected from C₁-C₈ alkyl, halogen atoms, nitro group, carboxy group, alkoxy, aryl;
R¹ₙ represents one or more "substituents of cyclic structure" of the same or different structure selected from hydrogen, halogen, C₁-C₈ alkyl, C₆-C₁₀ aryl, 5-6-membered azaheterocyclyl,
with the exception of:
1,2,3,4,5,6-hexahydroazepino[4,3-b]indole **A(1)** and also 7-methyl, isopropyl, chloro; 8-ethyl, fluoro; 9-propyl, chloro, bromo; 10-ethyl, fluoro- substituted 1,2,3,4,5,6-hexahydroazepino[4,3-b]indoles; 2-methyl-**A(2)**, 2-formyl-**A(3)**, 2-acetyl-**A(4)** and 6-methyl-1,2,3,4,5,6-hexahydroazepino[4,3-b]indoles **A(5),** and also 3,4,5,6-tetrahydro-N-phenylazepino[4,3-b]indole-2(1H)-carboxamide **A(6);** 3,4,5,6-tetrahydro-N-(2-chlorophenyl)-azepino[4,3-b]indole-2(1H)-carboxamide **A(7);** 3,4,5,6-tetrahydro-N-(2-fluorophenyl)-azepino[4,3-b]indole-2(1H)-carboxamide **A(8);** 3,4,5,6-tetrahydro-N-(3-methylphenyl)-azepino[4,3-b]indole-2(1H)-carboxamide **A(9).**

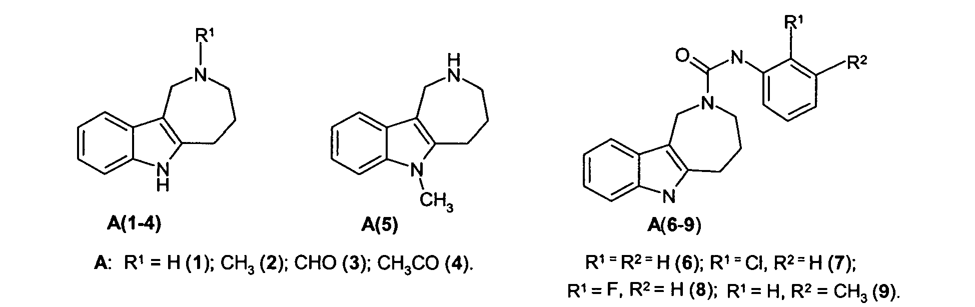

2. Compounds as claimed in claim 1 representing 1,2,3,4,5,6-hexahydroazepino[4,3-b]indoles of general formula **1.1,** *trans*-1,2,3,4,5,5a,6,10b-octahydroazepino[4,3-b]indoles of general formula **1.2** and *cis*-1,2,3,4,5,5a,6,10b-octahydroazepino[4,3-b]indoles of general formula **1.3:** 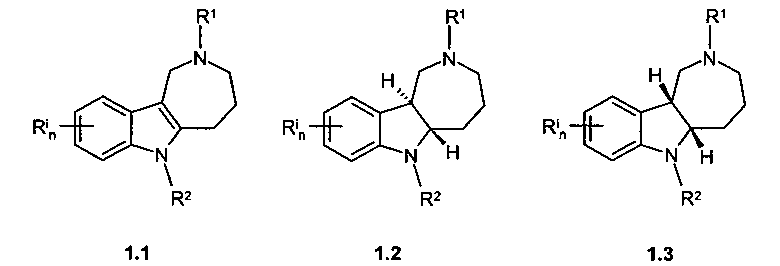 wherein: R¹, R² and Rⁱₙ have the above meanings.

3. Compounds as claimed in any of claims 1, 2 representing 1,2,3,4,5,6-hexahydroazepino[4,3-b]indoles of general formulas **1.1.1, 1.1.2, 1.1.3,** *trans*-1,2,3,4,5,5a,6,10b-octahydroazepino[4,3-b]indoles of general formulas **1.2.1, 1.2.2, 1.2.3** or *cis*-1,2,3,4,5,5a,6,10b-octahydroazepino[4,3-b]indoles of general formulas **1.3.1, 1.3.2, 1.3.3:** 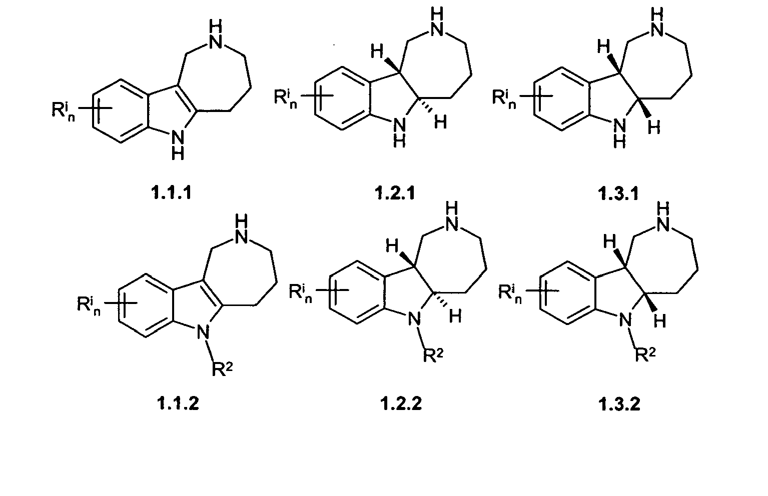 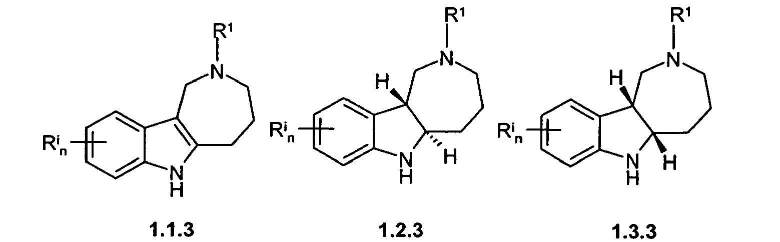 wherein: R¹, R² and Rₙⁱ have the above meanings.

4. Compounds as claimed in any of claims 1, 2 representing 2-tert-butyloxycarbonyl-1,2,3,4,5,6-hexahydroazepino[4,3-b]indoles of general formulas **1.1.1.1, 1.1.2.1,** *trans*-2-tert-butyloxycarbonyl-1,2,3,4,5,5a,6,10b-octahydroazepino[4,3-b]indoles of general formulas **1.2.1.1, 1.2.2.1** or *cis*-2-tert-butyloxycarbonyl-1,2,3,4,5,5a,6,10b-octahydroazepino[4,3-b]indoles of general formulas **1.3.1.1, 1.3.2.1:** 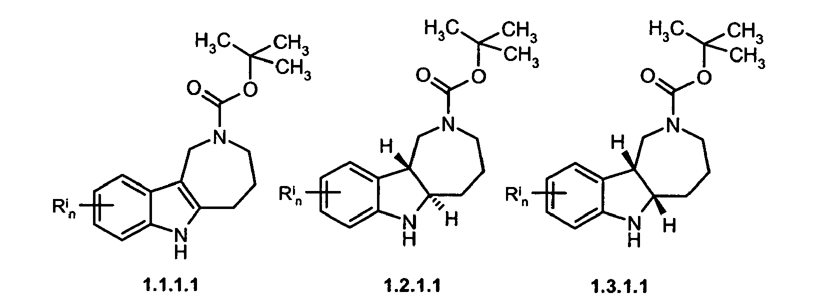 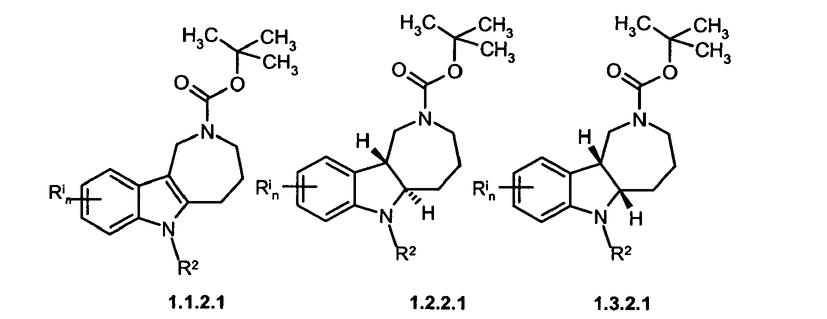 wherein: R² and Rₙⁱ have the above meanings.

5. Compounds as claimed in any of claims 1, 2 representing azepino[4,3-b]indoles of general formulas **1.1.4, 1.2.4, 1.3.4, 1.1.5, 1.2.5, 1.3.5, 1.1.6, 1.2.6, 1.3.6:** 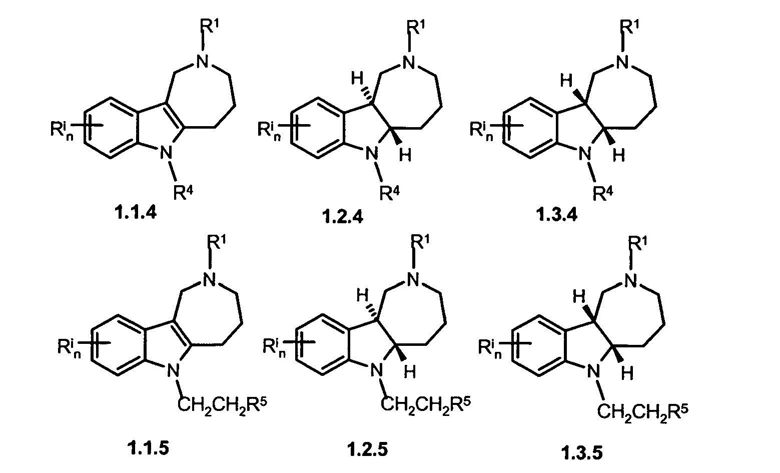 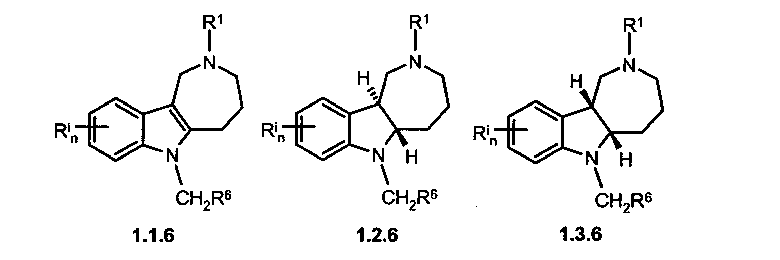 wherein: R¹ and Rₙⁱ have the above meanings; R⁴ is optionally substituted alkyl, optionally substituted aryl, optionally substituted heterocyclyl, optionally substituted alkyloxycarbonyl or optionally substituted alkoxycarbonylalkyl; R⁵ represents alkyloxycarbonyl, CN, aryl or heterocyclyl; R⁶ represents alkyloxycarbonyl, carbamoyl, CN, aryl or heterocyclyl.

6. Compounds as claimed in any of claims 1, 2 representing azepino[4,3-b]indoles of general formulas **1.1.7, 1.2.7, 1.3.7, 1.1.8, 1.2.8, 1.3.8, 1.1.9, 1.2.9** and **1.3:** 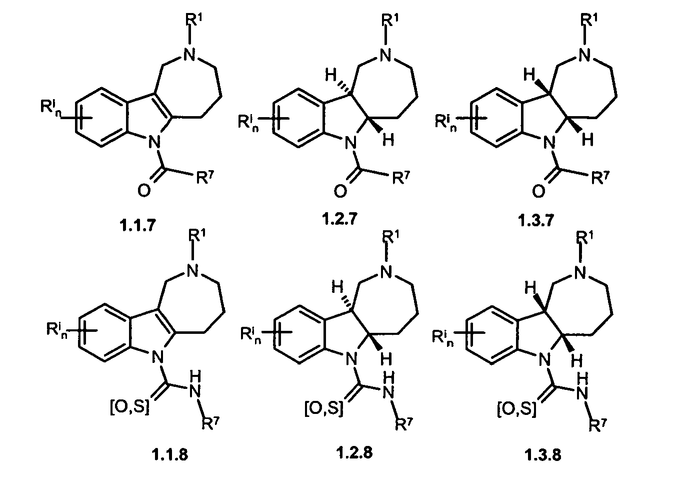 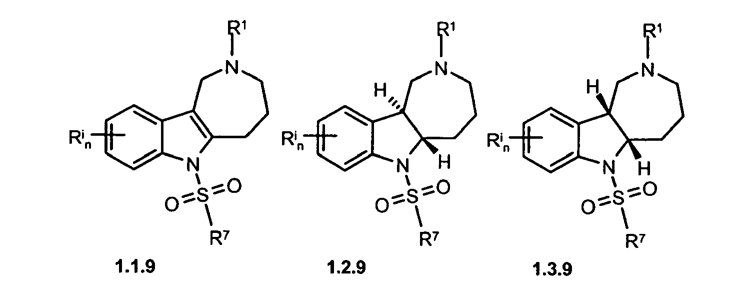 wherein: R¹ and Rⁱₙ have the above meanings; R⁷ represents alkyl, aryl or heterocyclyl.

7. Compounds as claimed in any of claims 1, 2 representing azepino[4,3-b]indoles of general formulas **1.1.10, 1.2.10, 1.3.10, 1.1.11, 1.2.11, 1.3.11, 1.1.12, 1.2.12** and **1.3.12:** 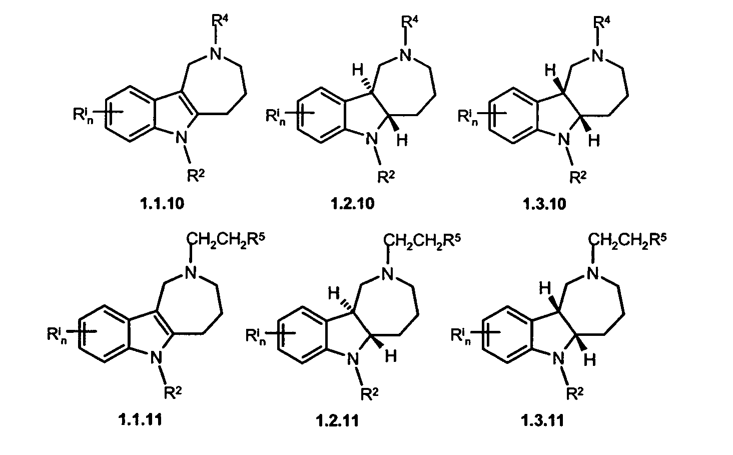 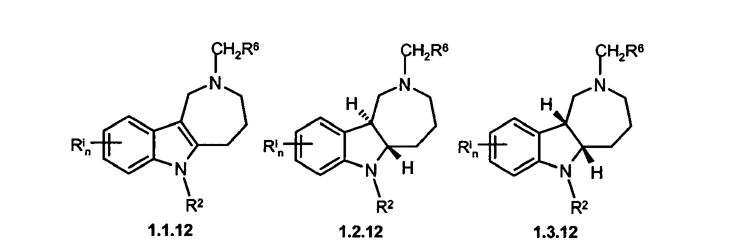 wherein: 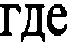 : R², R⁴, R⁵, R⁶ and Rₙⁱ have the above meanings.

8. Compounds as claimed in any of claims 1, 2 representing azepino[4,3-b]indoles of general formulas **1.1.13, 1.2.13, 1.3.13, 1.1.14, 1.2.14, 1.3.14, 1.1.15, 1.2.15** and **1.3.15:** 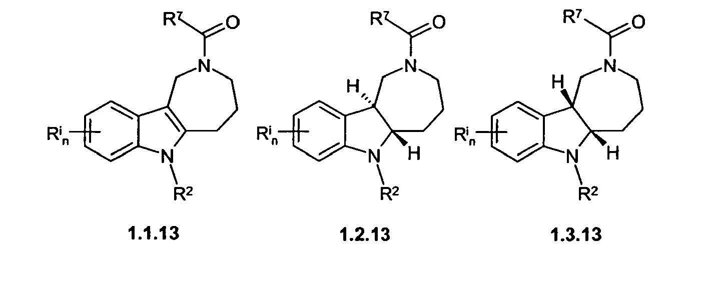 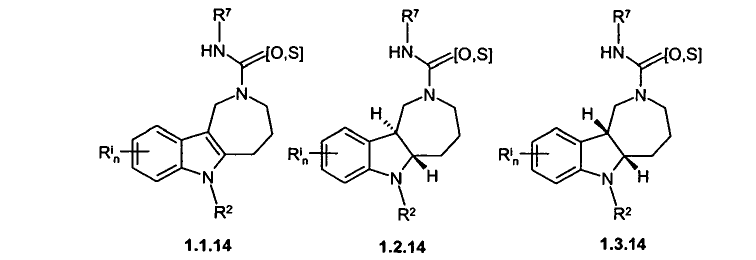 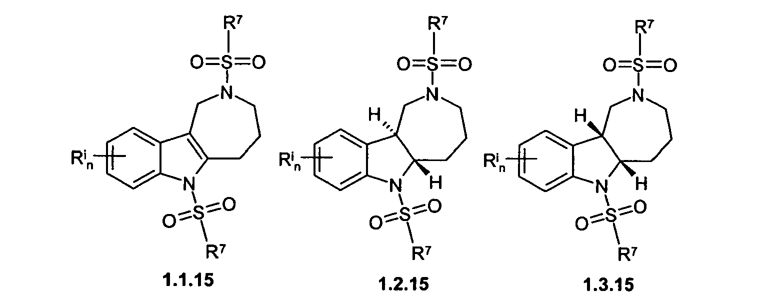 wherein: R², R⁷ and Rₙⁱ have the above meanings.

9. A method for preparation of compounds of general formulas **1.1.1, 1.1.2** as claimed in claim 3 by reduction of the corresponding compounds of general formula **4** with lithium aluminum hydride in organic solvent 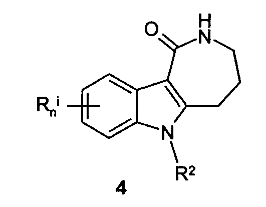 wherein: R² and Rₙⁱ have the above meanings.

10. A method for preparation of compounds as claimed in any of claims 1, 2 by reduction of compounds of general formula **1.1** with borane or its complexes in organic solvent.

11. A method for preparation of compounds as claimed in claim 4 by interaction of compounds of general formulas **1.1.1, 1.1.2, 1.2.1, 1.2.2, 1.3.1** or **1.3.2** with BOC-anhydride of the formula [tert-C₄H₉OC(O)]₂O in organic solvent.

12. A method for preparation of compounds as claimed in claim 5 by interaction of compounds of general formulas **1.1.3, 1.2.3** or **1.3.3,** with the exception of the compounds wherein R¹ = H, in organic solvent with the desired electrophilic reagents, such as: alkyl-, aryl-, or heterocyclyl halides of general formula **5** in presence of base; electrophilic alkenes of general formula 6 in presence of base catalyst; aldehydes of general formula 7 and NaBH(AcO)₃ 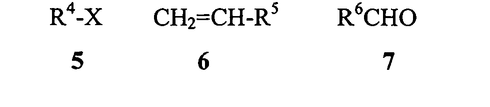 wherein: R⁴, R⁵ and R⁶ have the above meanings; X is a halogen atom.

13. A method for preparation of compounds as claimed in claim 6 by interaction of compounds of general formulas **1.1.3, 1.2.3** or **1.3.3,** with the exception of the compounds wherein R¹ = H, in organic solvent with the desired electrophilic reagents, such as: anhydrides or carbonic acid halides of general formula **8;** iso(thio)cyanates of general formula **9** or sulfonyl chlorides of general formula **10** 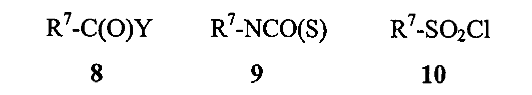 wherein: R⁷ has the above meaning; Y is a halogen atom, 3H-imidazol-1-ium hydroxide, R⁷-C(O)O.

14. A method for preparation of compounds as claimed in claim 7 by interaction of compounds of general formulas **1.1.1, 1.1.2, 1.2.1, 1.2.2, 1.3.1** or **1.3.2** in organic solvent with the desired electrophilic reagents, such as: alkyl-, aryl- or heterocyclyl halides of general formula **5** in presence of base; electrophilic alkenes of general formula **6** in presence of base catalyst; aldehydes of general formula 7 and NaBH(AcO)₃ 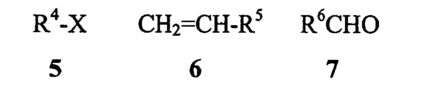 wherein: R⁴, R⁵, R⁶ and X have the above meanings.

15. A method for preparation of compounds as claimed in claim 8 by interaction of compounds of general formulas **1.1.1, 1.1.2, 1.2.1, 1.2.2, 1.3.1** or **1.3.2** in organic solvent with the desired electrophilic reagents, such as: anhydrides or carbonic acid halides of general formula **8;** iso(thio)cyanates of general formula 9 or sulfonyl chlorides of general formula **10** 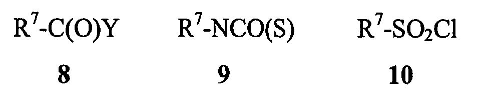 wherein: R⁷ and Y have the above meanings.

16. A method for preparation of compounds as claimed in any of claims 1, 2, wherein R¹ = H, by hydrolysis of BOC-protective group in the corresponding compounds of general formulas **1.1.1.1, 1.1.2.1, 1.2.1.1, 1.2.2.1, 1.3.1.1** or **1.3.2.1.**

17. A combinatorial library of compounds exhibiting neuroprotective, cognitive stimulating and antihistaminic activity for determining hit compounds and leader compounds composed of compounds of general formula **1** as claimed in claim 1.

18. A focused library of compounds exhibiting neuroprotective, cognitive stimulating and antihistaminic activity for determining and/or optimization of leader compounds containing at least one compound of general formula **1** as claimed in claim 1.

19. Pharmaceutical composition having neuroprotective, cognitive stimulating and antihistaminic activity for treatment of diseases patogenesis of which is connected with an excessive intracellular Ca⁺² ions concentration at animals and humans, and/or diseases associated with the disorder of histaminergetic mediator system, in the form of tablets, sheaths or injections placed in pharmaceutically acceptable packing, comprising as an active ingredient pharmaceutically effective amount of at least one hydrogenated azepino[4,3-b]indole of general formula **1,** either racemate, or optical isomer, or geometrical isomer, or pharmaceutically acceptable salt and/or hydrate thereof as claimed in claim 1.

20. A method for preparation of pharmaceutical composition as claimed in claim 19 by mixing an active ingredient with an exicipient and/or solvent, **characterized in that** as active ingredient at least one hydrogenated azepino[4,3-b]indole of general formula 1, either racemate, or optical isomer, or geometrical isomer, or pharmaceutically acceptable salt and/or hydrate thereof as claimed in claim 1 in pharmacologically effective amount is used.

21. Use of pharmaceutical composition as claimed in claim 19 for preparation of medicaments for prophylaxis and treatment of various diseases of warm-blooded animals and humans patogenesis of which is connected with an excessive intracellular Ca⁺² ions concentration and/or diseases associated with the disorder of histaminergetic mediator system.

22. Use of pharmaceutical composition as claimed in claim 21 for treatment of neurological disorders (in particular, hypoxia-ischemia, hypoglycemia, convulsive conditions, brain traumas, and so on) and also neurodegenerative diseases (among them Alzheimer's disease, Huntington's chorea, lathyrism, amyotrophic lateral sclerosis and so on).

23. Use of pharmaceutical composition as claimed in claim 21 for treatment of allergic and autoimmune diseases including pollinosis, hives, a bronchial asthma, atopic dermatitis, neurodermatitis, angioneurotic hypostasis, Quincke's disease, eczema, ambustial toxemia, and also the allergic reactions caused by medicines, foodstuff, cosmetics, dust, insect stings and so on.

24. Use of pharmaceutical composition as claimed in claim 19 for preparation of medicaments for enhancement of memory processes and cognitive stimulation at humans and warm blooded animals.

25. Compounds of general formula **1** as claimed in claim 1 possessing neuroprotective, cognitive stimulating and antihistaminic activity, and also ability to regulate cytosolic Ca⁺² ions concentration in neurones intended for experimental investigation of physiological processes *in vivo* and *in vitro* as "pharmacological tools".
